(19) **Europäisches Patentamt / European Patent Office / Office européen des brevets**

(11) **EP 3 589 206 B1**

(12) **EUROPEAN PATENT SPECIFICATION**

(45) Date of publication and mention
of the grant of the patent:
**09.04.2025 Bulletin 2025/15**

(21) Application number: **18708492.6**

(22) Date of filing: **28.02.2018**

(51) International Patent Classification (IPC):
**A61B 5/16** (2006.01)    **A61B 5/18** (2006.01)
**G06Q 10/00** (2023.01)

(52) Cooperative Patent Classification (CPC):
**G06Q 10/00; A61B 5/02405; A61B 5/0531;**
**A61B 5/11; A61B 5/1118; A61B 5/1128;**
**A61B 5/163; A61B 5/165; A61B 5/18;**
**A61B 5/7275; G16H 50/20;** A61B 5/0077;
A61B 5/02; A61B 5/7285; A61B 2503/20;    (Cont.)

(86) International application number:
**PCT/IB2018/051277**

(87) International publication number:
**WO 2018/158702 (07.09.2018 Gazette 2018/36)**

(54) **PRODUCTION MANAGEMENT APPARATUS, METHOD, AND PROGRAM**

VORRICHTUNG, VERFAHREN UND PROGRAMM ZUR PRODUKTIONSVERWALTUNG

APPAREIL, PROCÉDÉ ET PROGRAMME DE GESTION DE PRODUCTION

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **28.02.2017   JP 2017037287**
**01.09.2017   PCT/IB2017/055271**

(43) Date of publication of application:
**08.01.2020   Bulletin 2020/02**

(73) Proprietor: **Omron Corporation**
**Kyoto-shi, Kyoto 600-8530 (JP)**

(72) Inventors:
• **KOTAKE, Yasuyo**
  **Kyoto-shi**
  **Kyoto 600-8530 (JP)**
• **NAKAJIMA, Hiroshi**
  **Kyoto-shi**
  **Kyoto 600-8530 (JP)**

(74) Representative: **Hoffmann Eitle**
**Patent- und Rechtsanwälte PartmbB**
**Arabellastraße 30**
**81925 München (DE)**

(56) References cited:
WO-A1-2006/000166      WO-A1-2009/052633
JP-A- 2011 019 921      US-A1- 2009 066 521
US-A1- 2014 240 132     US-A1- 2014 336 473

**(Cont. next page)**

(52) Cooperative Patent Classification (CPC): (Cont.)
G16H 50/30

## Description

FIELD

The present invention relates to a production management apparatus, a method, and a program used in a production line involving an operation performed by a worker. Further, the invention relates to drive assisting apparatus, method, and program, as well as to healthcare support apparatus, method and program.

BACKGROUND

Early detection of equipment malfunctions in various facilities, such as production lines, is a key to preventing the operational efficiency from decreasing. A system has thus been developed for detecting a sign of an equipment malfunction by, for example, obtaining measurement data indicating the operating states of equipment from multiple sensors, and comparing the obtained measurement data with pre-generated learning data (refer to, for example, Patent Literature 1).

In a production line involving an operation performed by a worker, factors known to influence the productivity, or specifically the quality and the amount of production, include 4M (machines, methods, materials, and men) factors. Three of these factors, namely, machines, methods, and materials (3M), have been repeatedly improved and enhanced to increase the productivity. However, the factor "men" depends on the skill level, the aptitude, and the physical and mental states of a worker. Typically, a manager visually observes the physical and mental states of the worker, and intervenes as appropriate for the worker to maintain and enhance the productivity.

CITATION LIST

PATENT LITERATURE

Patent Literature 1: Japanese Patent No. 5530019

WO 2009/052633 A1 (MOTT CHRISTOPHER [CA]; MOLLICONE DANIEL [US]; VAN DONGEN HANS [US]; HU) 30 April 2009 (2009-04-30) discloses systems and methods provided for generating individualized predictions of alertness or performance for human subjects. Alertness or performance predictions may be individualized to incorporate a subject's individual traits and/or individual states. These individual traits and/or individual states (or parameters which represent these individual traits and/or individual states) may be represented by random variables in a mathematical model of human alertness. The mathematical model and/or prediction techniques may incorporate effects of the subject's sleep timing, the subject's intake of biologically active agents (e.g. caffeine) and/or the subject's circadian rhythms. The mathematical model and/or prediction techniques may incorporate feedback from the subject's measured alertness and/or performance.

WO 2006/000166 A1 (PAVELKA MILOSLAV [CZ]; KESHI TAMER [CZ]) 5 January 2006 (2006-01-05) describes a method of operator fatigue detection from operator muscular activity is performed by detecting at least one parameter affected by operator muscular activity, which parameter is assessed using fatigue assessment rules obtained using a data mining method from a corresponding parameter of at least one operator for whom the extent of fatigue is known. The device to perform the fatigue detection method comprises a programmable unit with a preprogrammed fatigue detection model - that is to say fatigue assessment rules obtained by measuring a signal of at least one of parameters generated by operator muscular activity for at least one fatigued operator and at least one alert operator, a detector or detectors to measure or measure and process the signal of the parameter or parameters used by the model, connected to the input of the programmable unit to assess operator fatigue, while the programmable unit output is connected to a fatigue signaling device.

US 2014/240132 A1 (BYCHKOV DAVID [US]) 28 August 2014 (2014-08-28) describes an approach provided for determining one or more behavioral states of a vehicle operator and causing one or more alerts and/or one or more management options based on the determined one or more behavioral states. The approach involves causing physiological information associated with a vehicle operator to be collected by one or more sensors. The approach also involves causing, at least in part, the physiological information to be communicated to a device that comprises at least one of the one or more sensors or is remote from at least one of the one or more sensors. The approach further involves processing the physiological information to determine a behavioral state associated with the vehicle operator. The approach additionally involves causing, at least in part, one or more alerts to be communicated to a device associated with the vehicle operator based, at least in part, on the determined behavioral state.

US 2014/336473 A1 (GRECO DEVON [US]) 13 November 2014 (2014-11-13) describes a biofeedback system and method enables biofeedback training to be accomplished during normal interaction by an individual with the individual's environment, for example while reading, playing video games, watching TV, participating in sports activities, or at work.

**EP 3 589 206 B1**

Physiologic data is processed and used to generate one or more control signals based on the physiologic data. The control signals may be proportional to a result of the data processing, or based on comparison of the processing results with at least one fixed or adaptive threshold. The control signal is supplied to a wearable device through which the individual receives sensory information from the individual's environment, and serves to interrupt or modify the sensory information. The wearable device may be an eyeglass device including a dynamic lens display, with the control signal being supplied to the dynamic lens display to modulate visual information received through the eyeglass device by obscuring, distorting, or otherwise affecting the clarity of the visual information. Feedback may also be provided in the form of auditory or tactile feedback.

JP 2011 019921 A (VOLVO TECHNOLOGY CORP) 3 February 2011 (2011-02-03) relates to a system and method for facilitating driver safety, and in particular to countermeasures against the effects of drowsiness and adverse conditions similar to distraction on driver performance.

US 2009/066521 A1 (ATLAS DAN [IL] ET AL) 12 March 2009 (2009-03-12) describes a method and system for detecting the physiological onset of operator fatigue. The condition of the operator is correlated with physiological signs of the early stages in the process of falling asleep according to the Hori EEG model. Passive correlation is done via monitoring of the gripping pressure of the operator's hand(s) on a handgrip containing pressure transducers. Correlation with Hori stage 2 is a preliminary indicator of the onset of operator fatigue. Active correlation is done via contingent Psychomotor Vigilance Tests administered upon the preliminary indication, with operator stimulus from a vibratory element in the handgrip and operator response being a rapid increase in gripping pressure. Correlation of the frequency of contingent PVT requests with Hori stage 3 is a critical indicator of the onset of operator fatigue. Upon detection of the onset of fatigue, an alarm is signaled to the operator and other personnel.

SUMMARY

The objection of the present disclosure is achieved by the appended independent claims and advantageous embodiments by the dependent claims. Examples and embodiments are provided in the description to facilitate the understanding of the present disclosure.

TECHNICAL PROBLEM

However, this technique of observing the physical and mental states of a worker relies on the experience or the intuition of the manager for accurate determination of the worker's physical and mental states affecting the productivity. This technique may not always determine the physical and mental states of the worker accurately. Moreover, after successful determination of changes in the worker's physical and mental states, the technique further relies on the manager for decisions about the details and the timing of the intervention. The intervention may not always be appropriate for improving and enhancing the productivity in a stable manner.

In response to the above issue, one or more aspects of the invention are directed to a production management apparatus, a method, and a program that allow an appropriate intervention to be constantly provided for a worker without relying on the experience or the intuition of a manager and improve and enhance the productivity. Further, prior art techniques have tried to improve safety in driving. However, there is a problem that known techniques do not take accurately into account the state of the driver, and how to obtain and use this in an objective and repeatable way in order to further improve safety. Still further, healthcare devices are known for supporting healthcare of a person; however, these devices do not take into account the accurate state of the person, and how this can be obtained and used in an objective and repeatable way so as to contribute to an improvement of the person's health conditions.

SOLUTION TO PROBLEM

In response to the above issue(s) as recognized by the inventors, a first aspect of the present invention provides a production management apparatus, a production management method, or a production management program for managing a production line involving an operation performed by a worker. The apparatus or the method includes an activity obtaining unit or process for obtaining information indicating an activity of the worker during the operation, a first estimation unit or process for estimating emotion and cognition of the worker during the operation based on the obtained information indicating the activity used as a primary indicator, and first learning data indicating a relationship between the activity and the emotion of the worker and a relationship between the activity and the cognition of the worker, a second estimation unit or process for estimating productivity of the worker based on the estimated emotion and cognition each used as a secondary indicator, and second learning data indicating a relationship between the productivity, and the emotion and the cognition of the worker, and an intervention determination unit or process for determining an intervention (preferably, timing and a detail thereof) to be provided for the worker based on the productivity estimated by the second estimation unit and a predetermined condition for providing an intervention.

In the apparatus, method, or program according to a second aspect of the present invention, the intervention determination unit includes a first determination unit that determines that a first intervention is to be provided for the worker at a time when the productivity estimated by the second estimation unit is determined not to meet a first condition, and a second determination unit that determines that a second intervention different from the first intervention is to be provided for the worker (or indirectly obtained by highly accurate measurement(s), see also further below) at a time when the productivity estimated by the second estimation unit is determined not to meet a second condition after the first intervention is provided.

In the apparatus, method, or program according to a third aspect of the present invention, the first determination unit determines that a visual or auditory stimulus is to be provided for the worker as the first intervention, and the second determination unit determines that a tactile stimulus is to be provided for the worker as the second intervention (in general, the second intervention is different from the first intervention, for instance in at least one amongst timing and details/characteristics of the intervention).

In the apparatus, method, or program according to a fourth aspect of the present invention, the intervention determination unit further includes a third determination unit that determines that the worker is to be instructed to stop the operation at a time when the productivity estimated by the second estimation unit is determined not to meet a third condition after the first or second intervention is provided.

Further aspects are herein described, numbered as A1, A2, etc. for convenience:

According to aspect A1, it is provided a production management apparatus for managing a production line involving an operation performed by a worker, the apparatus comprising:

an activity obtaining unit configured to obtain information indicating an activity of the worker during the operation, the information indicating an activity of the worker being preferablz information relating to at least one physiological parameter obtained by means of at least one activity sensor;

a first estimation unit configured to estimate emotion and cognition of the worker during the operation based on the obtained information indicating the activity used as a primary indicator, and first learning data indicating a relationship between the activity and the emotion of the worker and a relationship between the activity and the cognition of the workerwherein the first learning data preferably comprises data generated on the basis of information indicating emotion of at least one worker, information indicating cognition of the at least one worker, and information indicating activity of the at least one worker, wherein said information indicating emotion preferably relate to at least one physiological parameter obtained by means of at least one first sensor, said information indicating cognition preferably relate to at least one parameter indicative of cognition and obtained by means of at least one second sensor, and said information indicating activity preferably relate to at least one physiological parameter obtained by means of at least one third sensor;

a second estimation unit configured to estimate productivity of the worker based on the estimated emotion and cognition each used as a secondary indicator, and second learning data indicating a relationship between the productivity, and the emotion and the cognition of the worker; and

an intervention determination unit configured to determine an intervention to be provided for the worker based on the productivity estimated by the second estimation unit and a predetermined condition for providing an intervention.

A2. The production management apparatus according to aspect A1, wherein at least two amongst the at least one first sensor, the at least one second sensor and the at least one third sensor are different from each other.

A3. The production management apparatus according to aspect A1 or A2, wherein, when at least two amongst the at least one first sensor, the at least one second sensor and the at least one third sensor are substantially the same, then said at least two sensors being substantially the same are set according to different respective configurations.

A4. The production management apparatus according to any of aspects A1 to A3, wherein the activity sensor and the at least one third sensor are substantially the same.

A5. The production management apparatus according to any of aspects A1 to A4, wherein the second learning data comprises data generated on the basis of information indicating performance, said information indicating emotion of at least one worker, and said information indicating cognition of the at least one worker, wherein information indicating performance indicate performance in correspondence of said information indicating emotion and said information indicating cognition.

A6. The production management apparatus according to any of aspects A1 to A5, wherein the intervention determination unit is further configured to determine at least one of timing and characteristic of the intervention based at least on the productivity estimated.

A7. The production management apparatus according to any of aspects A1 or A6, wherein the intervention determination unit is configured to determine a first intervention and a second intervention to be provided to the worker at a first point in time and, respectively, second point in time, wherein the first intervention and the second intervention are different from each other. According to an optional aspect of anz of the above aspects, the intervention determination unit is configured to determine an intervention to be provided for the worker at a time when the productivity estimated by the second estimation unit is determined not to meet a condition after a previous intervention is applied.

A8. The production management apparatus according to any of aspect A1 to A7, wherein

the intervention determination unit includes
a first determination unit configured to determine that a first intervention is to be provided for the worker at a time when the productivity estimated by the second estimation unit is determined not to meet a first condition; and
a second determination unit configured to determine that a second intervention different from the first intervention is to be provided for the worker at a time when the productivity estimated by the second estimation unit is determined not to meet a second condition after the first intervention is provided.

A9. The production management apparatus according to aspect A8, wherein

the first determination unit determines that a visual or auditory stimulus is to be provided for the worker as the first intervention, and
the second determination unit determines that a tactile stimulus is to be provided for the worker as the second intervention.

A10. The production management apparatus according to aspect A8 or aspect A9, wherein

the intervention determination unit further includes
a third determination unit configured to determine that the worker is to be instructed to stop the operation at a time when the productivity estimated by the second estimation unit is determined not to meet a third condition after the first or second intervention is provided.

A11. A system comprising a production management apparatus according to any of aspects A1 to A10, and at least one article obtained by means of said manufacturing apparatus.
It is noted that preferable aspects like aspects A2 to A10 are applicable also to the below described aspects, and in general also to the further below described embodiments.
A12. A production management method to be implemented by a production management apparatus that manages a production line involving an operation performed by a worker, the method comprising:

obtaining information indicating an activity of the worker during the operation, the information indicating an activity of the worker preferably including information relating to at least one physiological parameter obtained by means of at least one activity sensor,
estimating emotion and cognition of the worker during the operation based on the obtained information indicating the activity used as a primary indicator, and first learning data indicating a relationship between the activity, and the emotion of the worker, and a relationship between the activity and the cognition of the worker, wherein the first learning data preferably comprises data generated on the basis of information indicating emotion of at least one worker, information indicating cognition of the at least one worker, and information indicating activity of the at least one worker, wherein said information indicating emotion preferably relate to at least one physiological parameter obtained by means of at least one first sensor, said information indicating cognition preferably relate to at least one parameter indicative of cognition and obtained by means of at least one second sensor, and said information indicating activity preferably relate to at least one physiological parameter obtained by means of at least one third sensor;
estimating productivity of the worker based on the estimated emotion and cognition each used as a secondary indicator, and second learning data indicating a relationship between the productivity, and the emotion and the cognition of the worker; and
determining timing to intervene for the worker and a detail of the intervention based on the productivity estimated by the second estimation unit and a predetermined condition for providing an intervention.

A13. A production management program enabling a processor to function as the units included in the production management apparatus according to any one of aspect A1 to A12.
A14. A drive assisting apparatus for providing driving assistance, the apparatus comprising:

an activity obtaining unit configured to obtain information indicating an activity of a subject during driving a vehicle, the information indicating an activity of the subject preferably includes information relating to at least one physiological parameter obtained by means of at least one activity sensor;

a first estimation unit configured to estimate emotion and cognition of the subject during driving based on the obtained information indicating the activity used as a primary indicator, and first learning data indicating a relationship between the activity and the emotion of the subject and a relationship between the activity and the cognition of the subject;

a second estimation unit configured to estimate performance of the subject based on the estimated emotion and cognition each used as a secondary indicator, and second learning data indicating a relationship between performance, and the emotion and the cognition of the subject when driving, wherein the first learning data preferably comprises data generated on the basis of information indicating emotion of at least one subject, information indicating cognition of the at least one subject, and information indicating activity of the at least one subject, wherein said information indicating emotion preferably relate to at least one physiological parameter obtained by means of at least one first sensor, said information indicating cognition preferably relate to at least one parameter indicative of cognition and obtained by means of at least one second sensor, and said information indicating activity preferably relate to at least one physiological parameter obtained by means of at least one third sensor; and

an intervention determination unit configured to determine an intervention to be provided for the subject based on the performance estimated by the second estimation unit and a predetermined condition for providing an intervention.

A15. A drive assisting method for providing driving assistance, the method comprising steps of:

obtaining information indicating an activity of a subject during driving a vehicle, the information indicating an activity of the subject preferably including information relating to at least one physiological parameter obtained by means of at least one activity sensor;

estimating emotion and cognition of the subject during driving based on the obtained information indicating the activity used as a primary indicator, and first learning data indicating a relationship between the activity and the emotion of the subject and a relationship between the activity and the cognition of the subject, wherein the first learning data preferably comprises data generated on the basis of information indicating emotion of at least one subject, information indicating cognition of the at least one subject, and information indicating activity of the at least one subject, wherein said information indicating emotion preferably relate to at least one physiological parameter obtained by means of at least one first sensor, said information indicating cognition preferably relate to at least one parameter indicative of cognition and obtained by means of at least one second sensor, and said information indicating activity preferably relate to at least one physiological parameter obtained by means of at least one third sensor;

estimating performance of the subject based on the estimated emotion and cognition each used as a secondary indicator, and second learning data indicating a relationship between performance, and the emotion and the cognition of the subject when driving; and

determining an intervention to be provided for the subject based on the performance estimated by the second estimation unit and a predetermined condition for providing an intervention.

A16. An apparatus for healthcare support of a subject, the apparatus comprising:

an activity obtaining unit configured to obtain information indicating an activity of a subject when executing an operation, the information indicating an activity of the subject preferably including information relating to at least one physiological parameter obtained by means of at least one activity sensor;

a first estimation unit configured to estimate emotion and cognition of the subject during executing the operation based on the obtained information indicating the activity used as a primary indicator, and first learning data indicating a relationship between the activity and the emotion of the subject and a relationship between the activity and the cognition of the subject;

a second estimation unit configured to estimate performance of the subject based on the estimated emotion and cognition each used as a secondary indicator, and second learning data indicating a relationship between performance, and the emotion and the cognition of the subject when driving, wherein the first learning data preferably comprises data generated on the basis of information indicating emotion of at least one subject, information indicating cognition of the at least one subject, and information indicating activity of the at least one subject, wherein said information indicating emotion preferably relate to at least one physiological parameter obtained by means of at least one first sensor, said information indicating cognition preferably relate to at least one parameter indicative of cognition and obtained by means of at least one second sensor, and said information indicating activity preferably relate to at least one physiological parameter obtained by means of at least one third sensor; and

an intervention determination unit configured to determine an intervention to be provided for the subject based on the performance estimated by the second estimation unit and a predetermined condition for providing an intervention.

A17. The apparatus for healthcare support of a subject according to aspect A16, wherein executing an operation includes at least one amongst executing an interacting operation with a machine and performing a physical exercise.

A18. An method for healthcare support of a subject, the method comprising steps of:

obtaining information indicating an activity of a subject when executing an operation, the information indicating an activity of the subject preferably comprising information relating to at least one physiological parameter obtained by means of at least one activity sensor;

estimating emotion and cognition of the subject during executing the operation based on the obtained information indicating the activity used as a primary indicator, and first learning data indicating a relationship between the activity and the emotion of the subject and a relationship between the activity and the cognition of the subject, wherein the first learning data preferably comprises data generated on the basis of information indicating emotion of at least one subject, information indicating cognition of the at least one subject, and information indicating activity of the at least one subject, wherein said information indicating emotion preferably relate to at least one physiological parameter obtained by means of at least one first sensor, said information indicating cognition preferably relate to at least one parameter indicative of cognition and obtained by means of at least one second sensor, and said information indicating activity preferably relate to at least one physiological parameter obtained by means of at least one third sensor;

estimating performance of the subject based on the estimated emotion and cognition each used as a secondary indicator, and second learning data indicating a relationship between performance, and the emotion and the cognition of the subject when driving; and

determining an intervention to be provided for the subject based on the performance estimated by the second estimation unit and a predetermined condition for providing an intervention.

A19. A computer program comprising instructions which, when executed on a computer, cause the computer to execute steps according to any of aspect A12, A15 or A18.

A20. An apparatus for handling performance in executing a task by a subject (or an apparatus for determining an intervention to apply to a subject executing a task), the apparatus comprising:

an activity obtaining unit configured to obtain information indicating an activity of the sbject during execution of the task, the information indicating an activity of the subject preferably comprising information relating to at least one physiological parameter obtained by means of at least one activity sensor;

a first estimation unit configured to estimate emotion and cognition of the subject during the operation based on the obtained information indicating the activity used as a primary indicator, and first learning data indicating a relationship between the activity and the emotion of the subject and a relationship between the activity and the cognition of the subject, wherein the first learning data preferably comprises data generated on the basis of information indicating emotion of at least one subject, information indicating cognition of the at least one subject, and information indicating activity of the at least one subject, wherein said information indicating emotion preferably relate to at least one physiological parameter obtained by means of at least one first sensor, said information indicating cognition preferably relate to at least one parameter indicative of cognition and obtained by means of at least one second sensor, and said information indicating activity preferably relate to at least one physiological parameter obtained by means of at least one third sensor;

a second estimation unit configured to estimate productivity of the subject based on the estimated emotion and cognition each used as a secondary indicator, and second learning data indicating a relationship between the productivity, and the emotion and the cognition of the subject; and

an intervention determination unit configured to determine an intervention to be provided for the subject based on the productivity estimated by the second estimation unit and a predetermined condition for providing an intervention.

It is noted that what is stated for a worker applies to a subject, and viceversa.

ADVANTAGEOUS EFFECTS

The apparatus, method, or program according to the first aspect of the present invention estimates the emotion and the cognition of the worker based on the information indicating the activity of the worker during the operation used as the primary indicator, and the first learning data generated separately from the first indicator, and estimates the productivity of the worker based on the estimated emotion and cognition as the secondary indicators, and the second learning data generated separately from the second indicators. The productivity estimate and the predetermined condition for providing an intervention are then used to determine the intervention (preferably, the timing and the detail thereof) for the worker. This enables an appropriate intervention to be constantly provided for a worker in a timely manner without relying on the experience or the intuition of a manager, and improves and enhances the productivity in a stable manner. Significantly, this is achieved autonomously and in an objective and repeatable way.

The apparatus, method, or program according to the second aspect of the present invention provides the first intervention for the worker at the time when the estimate of the productivity of the worker is determined not to meet the first condition, and provides the second intervention different from the first intervention for the worker at the time when the estimate of the productivity of the worker is determined not to meet the second condition after the first intervention is provided. Thus, the intervention is performed a plurality of times in a stepwise manner in accordance with the estimate of the productivity of the worker. This allows the worker to recover the productivity effectively.

The apparatus, method, or program according to the third aspect of the present invention provides a visual or auditory stimulus to the worker as the first intervention, and a tactile stimulus to the worker as the second intervention. In this manner, gradually stronger interventions are provided in a stepwide manner. This allows the worker to recover the productivity, while reducing the negative effect of any intervention on the mental state of the worker.

The apparatus, method, or program according to the fourth aspect of the present invention instructs the worker to stop the operation at the time when the estimate of the worker productivity is determined not to meet the third condition after the first or second intervention is provided. This allows, for example, the worker in poor physical condition to rest in a timely manner, and effectively maintains both the worker's health and the product quality.

The above aspects of the present invention provide a production management apparatus, a method, and a program that enable an appropriate intervention to be constantly provided for a worker without relying on the experience or the intuition of a manager, and improve and enhance the productivity in a stable manner.

According to further aspects, it is possible improving safety in driving, since the state of the driver can be objectively obtained by means of an apparatus, and the accurate state can be used to provide driving assistance, thus increasing safety. Still further, the accurate state of a person can be objectively obtained by a healthcare support apparatus, so that the health conditions of the person can be improved.

BRIEF DESCRIPTION OF THE DRAWINGS

Fig. 1 is a schematic diagram of a production management system according to an embodiment of the present invention.
Fig. 2 is a diagram showing an example emotion input device and an example measurement device included in the system shown in Fig. 1.
Fig. 3 is a diagram showing another measurement device included in the system shown in Fig. 1.
Fig. 4 is a functional block diagram of a production management apparatus installed in the system shown in Fig. 1.
Fig. 5 is a flowchart showing the procedure and the details of emotion learning performed by the production management apparatus shown in Fig. 4.
Fig. 6 is a flowchart showing the procedure and the details of cognition learning performed by the production management apparatus shown in Fig. 4.
Fig. 7 is a flowchart showing the first half part of the procedure and its details for generating and storing emotion learning data in an emotion learning mode shown in Fig. 5.
Fig. 8 is a flowchart showing the second half part of the procedure and its details for generating and storing the emotion learning data in the emotion learning mode shown in Fig. 5.
Fig. 9 is a flowchart showing the procedure and its details for generating and storing learning data in the cognition learning shown in Fig. 6.
Fig. 10 is a diagram showing an example operation results used for describing cognition estimation.
Fig. 11 is a flowchart showing the procedure and the details of production management performed by the production management apparatus shown in Fig. 4.
Fig. 12 is a flowchart showing emotion estimation and its details in the. procedure shown in Fig. 11.
Fig. 13 is a flowchart showing cognition estimation and its details in the procedure shown in Fig. 11.

Fig. 14 is a flowchart showing intervention control and its details in the procedure shown in Fig. 11.

Fig. 15 is a diagram describing a first example of the intervention control shown in Fig. 14.

Fig. 16 is a diagram describing a second example of the intervention control shown in Fig. 14.

Fig. 17 is a diagram describing the definition of emotion information that is input through the emotion input device shown in Fig. 2.

Fig. 18 is a diagram showing example input results of emotion information obtained through the emotion input device in the system shown in Fig. 1.

Fig. 19 is a diagram showing the classification of emotion information that is input through the emotion input device in the system shown in Fig. 1.

Fig. 20 is a diagram showing variations in emotion information that is input through the emotion input device in the system shown in Fig. 1.

Fig. 21 illustrates a block diagram of a mental state model that is well suited for technical applications wherein a person interacts with a device/machine.

Fig. 22 shows how cognitive and emotional states can be measured by way of objective and repeatable measurements.

Fig. 23 shows examples of objective and repeatable measurements.

DETAILED DESCRIPTION

The present invention is based, amongst others, on the recognition that the human factor influencing for instance productivity (or performance) is based on the mental state of a person. In order to understand this fact, it is preferable using an appropriate model for the person (i.e. of his/her mental state) that takes into account different types of states of a person, wherein the states are directly or indirectly measurable by appropriate sensors. Thus, the mental state can be objectively and systematically observed, as well as estimated in view of the intended technical application.

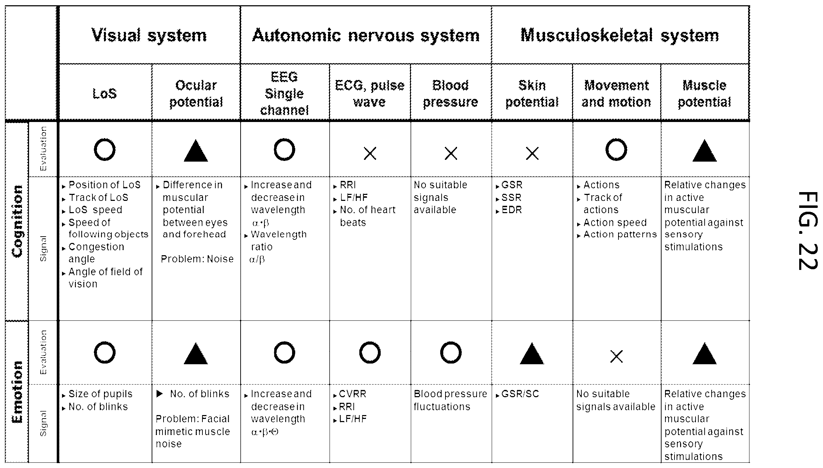

More in detail, in order to allow a technical application that objectively and systematically takes into account a mental state, the latter state can be modeled by a combination of a cognitive state (also cognition, in the following) and an emotional state (also emotion, in the following) of a person. The cognitive state of the person relates to, for example, a state indicating a level of ability acquired by a person in performing a certain activity, for instance on the basis of experience (e.g. by practice) and knowledge (e.g. by training), as also further below discussed. The cognitive state is directly measureable, since it directly relates to the execution of a task by the person. Emotional state has been considered in the past solely as a subjective and psychological state, which could not be established objectively e.g. by technical means like sensors. Other (more recent) studies however led to a revision of such old view, and show in fact that emotional states of a person are presumed to be hard wired and physiologically (i.e. not culturally) distinctive; further, being based also on arousal (i.e. a reaction to a stimuli), emotions can be indirectly obtained from measurements of physiological parameters objectively obtained by means of suitable sensors, as also later mentioned with reference to Figure 22.

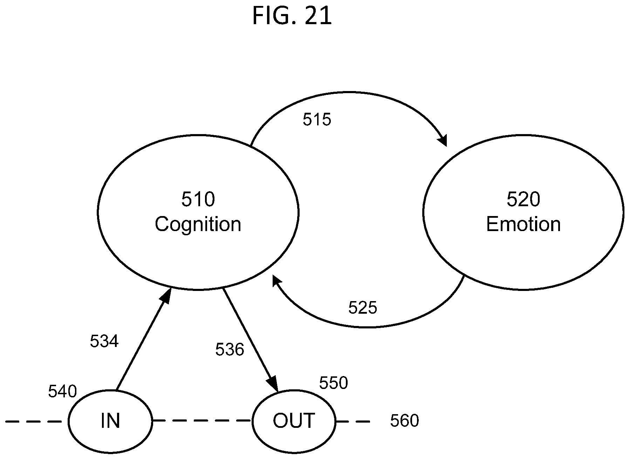

Figure 21 shows a model of a mental state that can be used, according to the inventors, for technical applications dealing for instance with human or men factor influencing for instance productivity. In particular, the model comprises a cognitive part 210 and an emotional part 520 interacting with each other. The cognitive part 510 and the emotional part 520 represent the set of cognitive states and, respectively, the set of emotional states that a person can have, and/or that can be represented by the model. The cognitive part directly interfaces with the outside world (dashed line 560 represents a separation to the outside world), in what the model represents as input 540 and output 550. The input 540 represents any stimuli that can be provided to the person (via the input "coupling port" 540, according to this schematic illustration), and the output 550 (a schematic illustration of an output "coupling port" for measuring physiologic parameters) represents any physiological parameters produced by the person, and as such measurable. An intervention as also later described can be seens as a stimulus provided via the input coupling port 540 of the depicted model. The emotional part can be indirectly measured, since the output depends on a specific emotional state at least indirectly via the cognitive state: see e.g. line 525 (and 515) showing interaction between emotion and cognition, and 536 providing output, according to the model of Figure 21. In other words, an emotional state will be measurable as an output, even if not directly due to the interaction with the cognitive part. It is herein not relevant how the cognitive part and the emotional part interact with each other. What matters to the present discussion is that there are input to the person (e.g. one or more stimuli), and output from the person as a result of a combination of a cognitive state and an emotional state, regardless of how these states/parts interact with each other. In other words, the model can be seen as a black box having objectify measurable input and output, wherein the input and output are causally related to the cognitive and emotional states, though the internal mechanism for such causal relationship are herein not relevant.

Despite the non-knowledge of the internal mechanisms of the model, the inventors have noted that such a model can be useful in practical and technical application in the industry, like for instance when wanting to handle human/men factors influencing productivity, or when wanting to control certain production system parameters depending on human performance, as it will also become apparent in the following.

Figure 22 shows how cognitive and emotional states can be measured by way of objective and repeatable measurements, wherein a circle, triangle and cross indicates that the listed measuring methods are respectively well suitable, less suitable (due for instance to inaccuracies), or (at present) considered not suitable. Other techniques are also available, like for instance image recognition for recognizing facial expressions or patterns of facial expressions that are associated to a certain emotional state. In general, cognitive and emotional states can be measured by an appropriate method, wherein certain variable(s) deemed suitable for measuring the given state are determined, and then measured according to a given method by means of suitable sensor(s). As also evident from Figure 22, the emotional state can be obtained by measuring respective physiological parameter(s) by at least one emotional state sensor, preferably set according to an emotional state sensor configuration, and the cognitive state can be measured by at least one cognitive state sensor preferably set according to a cognitive state sensor configuration, wherein the at least one emotional state sensor is different from the at least one cognitive state sensor and/or the emotional state sensor configuration is different from the cognitive state sensor configuration. In other words, the emotion sensor is a sensor suitable for measuring at least a physiological parameter relating to emotion, and the cognitive sensor is a sensor suitable for measuring at least a physiological parameter relating to cognition. For instance, with reference to Figure 22, LoS (Line of Sight) measurements can be performed for estimating or determining the cognitive state and/or the emotion state, however the configuration of the sensor is different since the parameter(s)/signal(s) used is different depending on whether the emotion or cognition wants to be determined. An example of the sensor for obtaining LoS is represented by a camera and an image processing unit (either integrated or separated from the camera), wherein the camera and/or the processing unit are differently set in order to acquire a signal related to the cognitive state (e.g. any one or a combination of the following examples: the position of LoS, the track of LoS, the LoS speed, the speed of following objects by the eye(s), the congestion angle, and/or the angle of field of vision, etc.) or a signal related to the emotion state (any one or a combination of the following examples: size of pupils, number of blinks, etc.). For example, if the number of blinks wants to be detected, the camera should be set to acquire a given number of images (or a video with a given, preferably high, number frames per second) and image processing unit for recognizing one blink; when the position of LoS wants to be detected, the camera may be set to acquire just one image, even if more is preferable, and the image processing unit to detect the LoS position from the given image(s). Similar considerations apply to other signals relating to LoS for either cognitive state or emotional state; also, similar considerations apply to other types of signals like those relating to the autonomic nervous system or musculoskeletal system as directly evident from Figure 22. With this regard, it is also noted that (at least according to the present knowledge) blood pressure measurements are suitable for detecting the emotional state, but not the cognitive state: thus, in this case, any blood pressure sensor would be suitable for obtaining an emotional state, and any sensor suitable for obtaining blood pressure would be an example of the emotional state sensor regardless of its configuration. Similarly, any sensor suitable for detecting movement and motion (e.g. any or a combination of: actions, track of actions, action speed, action patters, etc., see figure 22) is an example of a cognitive state sensor regardless of its configuration. Thus, as also shown in figure 22, a cognitive state and an emotional state can be detected by a cognitive state sensor and, respectively, emotional state sensor, and/or - when the sensor itself can be the same or similar - by a different configuration of the sensor. Herein, by sensor it is meant a sensing device for detecting physical signals, possibly together (as necessary) with a processing unit for obtaining information on the cognitive or emotion state on the basis of the physical signal.

With reference to the emotional state sensors, it is noted that for instance the emotional state can be obtained on the basis of (i) brain related parameter(s) and/or (ii) appearance related parameter(s) and/or other parameter(s).

    (i) The brain related parameter(s) obtained by suitable sensors and/or sensor configuration(s), see also Figure 22.

The brain related parameter(s) can be represented for example by brain waves obtained by EEG, e.g. by detecting an event-related potential ERP (defined as a stereotyped electrophysiological response to a stimulus). More in particular, using a relationship between the applied stimuli (ex. music, picture for relaxing, excitement, etc.) and the measured EEG pattern corresponding to the ERP induced by a (preliminary learned/known or learned for each user) stimuli, it is possible to determine whether the specific characteristic of the EEG is associated with a known emotional state (e.g. appearances of alpha waves when relaxing). In other words, according to this example, by observing the EEG pattern, and specifically the ERP, it is possible to obtain an indirect measure of the emotional state. For more on ERP, see e.g. An Introduction to the Event-Related Potential Technique, Second Edition, Steven J. Luck, ISBN: 9780262525855.

According to another example, the brain blood flow obtained by fMRI (functional Magnetic Resonance Imaging) can be used as a brain related parameter: the active region of the brain, in fact, can indicate some emotional states; for example, the correlations of BOLD (blood oxygen level dependent) signal with ratings of valence and arousal can be obtained in this way, thus achieving an indirect measure of the emotional state (see e.g. The Neurophysiological Bases of Emotion: An fMRI Study of the Affective Circumplex Using Emotion-Denoting Words, by J. Posner et al, Hum Brain Mapp. 2009 Mar; 30(3): 883-895, doi: 10.1002/hbm.20553).

The above measurements methods/devices can be also combined together. Techniques based on (i) are accurate, but the measurement device may be large and the user's motions may be largely limited.

(ii) Appearance related parameter(s) can be obtained from suitable sensors and/or sensor configurations (see also e.g. Figure 22), for instance on the basis of:

- Facial image analysis of facial expression(s) (as captured for instance by a camera): for instance, using pixel information such as RGB value and intensities, one or more parameters including the angles of the eyebrows, the angle of the mouth, the degree of mouth opening, and/or the degree of eye openings are calculated; the emotion can then be determined (preferably, automatically by a hardware/software unit) based on the combination of one or more such parameters using available set of templates defining the relationship between those parameters and emotions.
- Acoustic analysis of voice expressions: similar to the facial expressions, the emotion can be determined using the available set of templates defining the relationship between the parameters and emotions.

A combination of facial expression and voice expressions can also be used. Emotions estimated on the basis of appearance related parameter(s) are estimated with an higher/increased accuracy when the information amount increases, e.g. when the amount of parameters used increases, or (mathematically speaking) when using a higher dimensional information. In simpler words, when acoustic analysis and facial analysis are both executed, and/or when facial analysis is performed on the basis of multiple analysis on eyebrows, angle of mouth, etc., then accuracy can be increased. The more the parameters used in the analysis, however, the larger the computing resources needed for processing; moreover, providing/arranging camera for each user or requesting the voice utterances may not always be possible depending on the situations. Thus, the higher accuracy comes at a price in the terms of computational resources and/or complexity of the camera/machines used for such analysis.

(iii) Other parameters, possibly obtained by other sensors and/or different configurations of sensors (see e.g. Figure 22), can be used for estimating emotions, like for instance:

- Pupil size by eye image recognition (i.e. an analysis made on image(s) taken of the eye(s) of a subject), wherein the Time Resolution TR is preferably higher than 200Hz, for example;
- Heart electrical activity, detected by ECG, preferably having TR

higher than 500Hz, for example.

Techniques based on (iii) are accurate, but may require large computing resources in analysis.

As anticipated, cognition can be estimated for instance by LoS measurements, either by means of a specific sensor, or by a sensor being substantially the same as the one used for emotion, but differently set (set according to a different configuration) such that physiological parameter(s) are detected corresponding to cognition. More in general, the cognition sensor is a sensor suitable for obtaining physiological parameters related to cognition. For example, such physiological parameters relating to cognition can be one or a combination of LoS parameter(s), EEG parameter(s), movement and/or motion parameter(s) like for example:

- As also anticipated, LoS parameters (including eye movement) relevant to cognition may be obtained by measuring for instance: position of LoS, and/or track of LoS, and/or LoS speed, and/or speed of following object(s), and/or congestion angle, and/or angle of field of vision. These parameters may be detected by eye image recognition with a camera;
- With further reference to figure 22, EEG related parameters can be obtained by measuring for instance: increase and decrease in wavelength $\alpha$ and/or b (alpha and/or beta waves), wavelength ratio $\alpha/b$ ; these parameters may be thus detected by EEG measurements;
- With further reference to figure 22, movement and/or motion parameters relating to cognition can be obtained by measuring for instance: action, and/or tracks of actions, and/or action speed, and/or action patterns, and/or hand movement. These parameters may be detected by measuring with an acceleration sensor acceleration generated by movement of the target, or by movement/motion recognition in a video (sequential images) capturing the target by means of a camera; by comparing or evaluating the taken picture(s) and/or video(s) against a known picture(s) and/or video(s), cognition is obtained for the subject performing the operation. The feature amount in this example may be represented by the number or incidence of misoperations, or by the number of objects (parts) deviated from the predetermined positions, as also further below discussed with reference to factory automation. In the case of a vehicle/driving application, the cognition sensor can be a recording device for recording vehicle operations (such as acceleration, braking, steering operations, etc.) together with vehicle environment images (i.e., images of outside the vehicles). In this case, for instance, the number or incidence of misoperations is obtained by comparing the standard operation (e.g. stop before the stop line) with detected operation in response to the external event occurred in the vehicle environment (e.g, traffic signal turned into yellow or red).

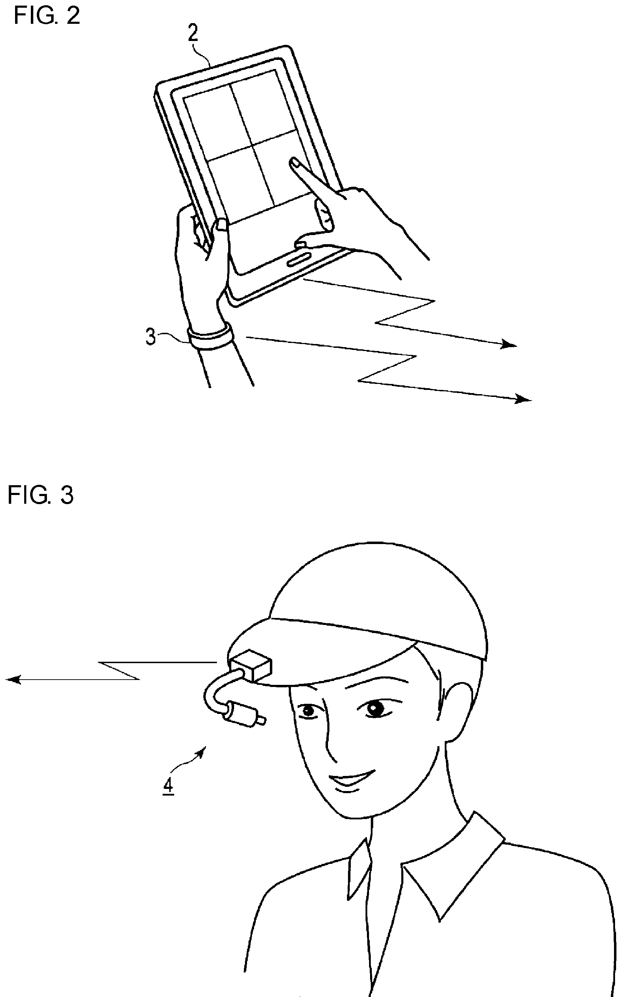

Further, activity can be obtained by means of an activity sensor suitable for measuring vital sign and/or motion related parameters, and includes for example sensors for measuring heart electrical activity H, and/or skin potential activity G, and/or motion BM, and/or an activity amount Ex. An example of an activity sensor is referred as a wearable measurement device 3 as in figure 3, and further later described. Similarly, a camera 4 (see again Figure 3) mounted on a helmet or cap of a subject may be used (by means e.g. of image processing aimed at detecting movement) as a sensor for detecting motion related parameters indicating information about the activity of the subject. Still further, a sensor for detecting blood pressure may be used as an activity sensor. For instance, as also later described, the activity sensor can be a sensor capable of measuring heart electrical activity H, skin potential activity G, motion BM, activity amount Ex, etc. With reference to the example of heart electrical activity H, the activity sensor (or a suitable configuration of a sensor suitable for measuring heart electrical activity) is capable of measuring the heartbeat interval (R-R interval, or RRI), and/or the high frequency components (HF) and/or the low frequency components (LF) of the power spectrum of the RRI, with a required Time Resolution (TR) preferably set to 100Hz - 200Hz. Such parameters can be obtained for instanced by means of an ECG device and/or a pulse wave device. As discussed above, see e.g. the other parameters (iii) used for measuring emotions, heart activity can be used also for estimating emotions; however, the sensors used for measuring heart activity related to emotions must be set differently that the same sensors when used for measuring heart activity related to an activity performed by the subject; in the example herein discussed, for instance, a TR of 100-200Hz suffices for measuring activity, while a TR of 500Hz or more is preferable for measuring emotions. This means that that activity measurement can be achieved with less computational resources than emotion measurements. Regardless of the complexity necessary for obtaining activity information and emotional information, both are used - once obtained - in order to generate learning data indicating a relationship between activity information and emotional information.

Referring to emotions, by any one of or any combination of above techniques, including (i) to (iii), emotional state can be sensed; however, for sensing the emotions accurately, fluctuations of the states, or the continuous variations of the states are important information to consider, which require relatively high time resolution and high dimensional information (thus resulting in high computing resources). Similar considerations apply to cognition sensors. In short, sensing emotion and cognition may require computationally demanding sensor units, and in general complex sensors; Further, such emotion and/or cognition sensors may be cumbersome, or not easy to deploy in certain environments, especially for a daily use or when more subjects are closely interacting.

In contrast thereto, the activity sensor is a sensor that requires smaller information amount, and/or less processing load (including processing time), and/or less time resolution, and/or constructionally simpler and/or less complex than the emotional sensor.

As anticipated, a variety of sensors are suitable for obtaining such measurements, and they are herein not all described since any of them is suitable as long as they provide any of the parameters listed in figure 22, or any other parameters suitable for estimating cognitive and/or emotional states. The sensors can be wearables, e.g. included in a wrist or chest wearable device or in glasses, an helmet like device for measuring brain activity from the scalp (e.g. EEG/NIRS), or a large machine like PET/fMRI.

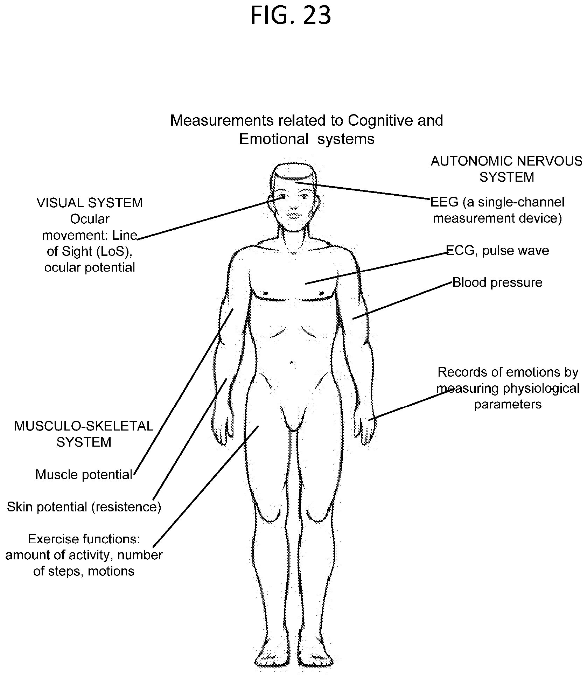

Thus, it possible to model a person, like for instance a factory operator or worker (or a driver of a vehicle, or a person using an healthcare supporting device, etc.), by using a model as illustrated in figure 21, and collect measurements of physiological parameters of the person as shown in figures 22 and 23. In this way, as also shown in the following, it is possible to improve for instance productivity of a production line, increase safety in driving and improving health conditions.

The above explanation is provided as illustrative and propaedeutic to the understanding of the invention and following embodiments/examples, without any limitation on the same.

Turning to the invention, and referring for the sake of illustration to the case of a production line: emotional and cognitive states can be estimated on the basis of first learning data and on information indicating an activity of the worker (i.e. information obtained from measurements on the worker. or in other words information relating to at least one physiological parameter obtained by means of at least one activity sensor as above illustrated, or below further detailed); the worker performance can then be estimated on the basis of the estimated cognition and emotion, and of second learning data. The emotion and cognition estimation allow obtaining an accurate estimation of the overall mental state (see e.g. the above discussed model), and the worker productity/performance can also be more accurately estimated; consequently, an appropriate internveion can be determined to be applied to the worker, such that factory productivity can be increased when taking into account also the human factor. It is significant that this productivity estimation is reached on the basis of objective and repeatable measurements (of the worker activity) that an apparatus can perform, and on specific learning data. Details on the estimation are provided also below, but reference is also made to JP2016-252368 filed on 27 December 2016 as well as to the PCT application PCT/IB2017/055272 (reference/docket number 198 759) filed by same applicant and on the same date as the present one, as well as well as PCT application PCT/IB2017/058414 describing for instance how the emotional state can be estimated.

The first learning data preferably comprises data generated on the basis of information indicating emotion of at least one worker, information indicating cognition of the at least one worker, and information indicating activity of the at least one worker, wherein said information indicating emotion relate to at least one physiological parameter obtained by means of at least one first sensor (e.g. an emotion sensor as above illustrated or below further detailed), said information indicating cognition relate to at least one parameter obtained by means of at least one second sensor (e.g. one cognition sensor as above introduced and later further detailed), and said information indicating activity relate to at least one physiological parameter obtained by means of at least one third sensor (as above illustrated, or further below detailed). As above explained, the sensor(s) required to measure activity is less complex and/or less cumbersome than sensors used to measure emotion and cognition. Thus, emotion and cognition are measured accurately with respective suitable sensors, and the activity is also measured in correspondence of the measured emotion and measured cognition. The collected measurements are then used to generate the first learning data, and thus to generate the relationship between emotion and activity, and the relationship between cognition and activity. The learning data is then "used in the field", e.g. in the manufacturing line, in (or for) the car, or in a healthcare support device, depending on the application also below illustrated. In the field, it is then not necessary to perform the complex measurements on emotion and cognition; it suffices performing the easier measurements on activity, since the emotion and cognition can be estimated on the basis of the first learning data. The estimation is nevertheless accurate, since the first learning data is obtained from accurate measurements. Thus, it is possible to estimate emotion and cognition in the field by means of a reduced number of sensors, and by using simple and non-complex sensors. Once the emotion and cognition are estimated, it is also possible to estimate the performance/productiviy of the subject in a very accurate manner, since not only cognition but also emotion is taken into account, and by using few and simple sensors. It follows that the estimation in productivity/performance, like e.g. manufacturing producvitity or driving performance or performance of a subject, can be accurately obtained by few and simple sensors. In fact, the activity sensor(s) may also be a wearable sensor or included in a wearable device. As further examples, the activity information can be obtained, as also later discussed, by other measurements like for instance based on any one or any combination of:

- Skin potential activity G, e.g. by measuring the galvanic skin response (GSR); this is a parameter easier to obtain, when compared to parameters used to measure an emotional state;
- The eye movement EM, e.g. by measuring the eye movement speed and the pupil size (e.g. based on captured images(s) or video(s) on a subject); in this case, when noting that the same or similar parameters can be used also for obtaining emotions (see (iii) above), the required TR may be equal to or lower than 50Hz (fluctuations or continuous variations of the sensed parameter is not obtained within this range of TR). Similarly to the case of heart activity, the EM measurements related to the activity of the subject is easier to obtain that the EM measurements related to emotions.
- The motion BM, like e.g. the hand movement speed. This is also a parameter that is easier to obtain than parameters related to emotions.

In general, therefore, activity information are easier to obtain (than cognition or emotion) either because they can be obtained by less complex sensors than those required for measuring emotions or cognition, or - when the same type of sensors are used - the configuration of the sensor for acquiring activity information results in less computing resources than the configuration for acquiring emotions or cognition. Thus, by using learning data and the (easily) acquired activity information, it is possible to obtain the emotional state and cognitive state of a subject. As a consequence of obtaining the estimated emotional state and cognitive, a more accurate intervention can be determined or selected, such that safer driving, improved manufacturing, and improved health conditions can be conveniently achieved by easily taking into account the mental state of a subject interacting with a device.

In an illustrative application, the estimated performance can be used to determine an intervention to be provided for the worker based on the productivity estimated by the second estimation unit and a predetermined condition for providing an intervention. Thus, the productivity can be conveniently improved (thanks to the accurately estimated worker state, including the emotional state) and/or the respective production quality can be better controlled and improved. Also here, significantly, the better productivity/quality is achieved on the basis of objective and repeatable measurements, and on specific learning data. In other words, once the productivity is obtained, it is optionally possible to apply an intervention on the worker interacting with the manufacturing line (i.e. one or more of its components), i.e. a feedback is applied based on the productivity estimated objectively with high accuracy and by means of simple sensor(s). By way of the appropriately determined intervention, the overall efficiency of the system, which depends on the interactions between the subject and the system or its components, can be improved. It is therefore possible to improve the system, since the productivity/-performance, on the basis of which the internvention is applied, can be more accurately estimated, and importantly by means of few and simple sensors.

Optionally, at least two amongst the cognitive, emotion, and activity sensors may be different from each other: for instance, as also evident from the present description, it is possible using a camera for measuring emotion (e.g. size of pupils) and cognition, and a sensor for measuring blood pressure or skin potential activity G. It is also possible that the three sensors are different from each other: e.g. a camera is used for determining cognition, an ECG is used for measuring emotion, and a skin potential activity sensor is used as activity sensor. Other configurations are evidently possible, as also explained in the present description.

Optionally, when at least two amongst the emotion, cognition, and activity sensors are substantially the same, then the sensors being substantially the same are set according to different respective configurations. By "substantially the same" (or by "the same") it is herein meant that the sensors are of the same type. The camera is one example of a "substantially the same" sensor used for measuring cognition and emotion: in fact, two distinct cameras being exactly the same can be provided, one for measuring emotion, the other for measuring cognition; alternatively, two different cameras can be used, e.g. with different resolutions, for measuring emotion and cognition, respectively. In such case, the configurations of the two cameras are differently set, so that one produces an emotion measurement, and the other a cognitive measurement. Further, it is also possible having one single camera for measuring emotion and cognition, in which case the processing unit and/or software used on combination with the camera is configured to differently process the image/video taken in order to produce an emotion or cognition measurement. Still further, the same picture(s) and/or video(s) taken by one single camera can be differently elaborated to produce emotion and cognition measurement, according to different configurations for elaborating the image/video. Reference is also made to the above example relating to the different configuration of an ECG for measuring activity or emotion. These are some of the examples of the same sensor being differently configured to produce several respective pieces of information relating to emotion, cognition, and/or activity.

As above summarized, the activity "in the field" is measured by means of an activity sensor. The first learning data is preferably obtained before the deployment of the solution "in the field", or separately (e.g. while the solution is running on an old set of first learning data, a newer set of first learning data is in the process of being separately generated). When obtaining the first learning data, the activity also needs to be measured by an activity sensor. The activity sensor used in the field and the activity sensor used for generating the learning data can be the same, but need not be necessarily the same. For instance, different level of accuracies may want to be used for measuring activity in the field or when collecting data for learning, or different sizes/types of devices depending on their size, complexity, etc.

In order to further illustrate the inter-relationship and differences amongst the different sensors herein discussed, the following non-limiting examples are also given: the emotion sensor can be any sensor suitable for measuring physiological parameters relating to emotion as above discussed, see also figure 22. The cognition sensor can be any sensor for measuring any parameter related to cognition, see e.g. the above discussion and/or figure 22 and/or also the below discussed monitoring camera CM for checking operation results (indicative in fact of the cognition, i.e. the level of skills and capabilities fo the worker). Thus, the cognition sensor is suitable for measuring physiological parameters relating to cognition (see e.g. figure 22), or any parameter relating to an activity and/or execution of an activity and/or result of execution of an activity by the subject (in general, a sensor capable of measuring a parameter indicative of the cognition state of the subject). The emotion sensor and cognition sensor are thus sensors suitable for providing correct values for an emotional state and a cognitive state, respectively, of a person, wherein "correct value" is used to indicate a preferably highly accurate measurement relating to emotion and cognition, respectively, as also further below illustrated. With regard to the activity sensor, as said, the activity sensor and/or the respective configuration used during the learning phase may be the same or different from the activity sensor and/or its configuration used during the estimation phase (i.e. in the field). Further, the activity sensor - either for the learning phase and/or for the estimation (in the field) phase - may be (but not necessarily) different and/or differently set (i.e. with a different configuration) in relation to emotion and cognition. For example, when used in the learning phase in order to gather measurement data for generating the emotion-activity relationship (the first learning data), the activity sensor may be e.g. a sensor(s) like a later described measuring device 3 (e.g. suitable for measuring the heart electrical activity H, the skin potential activity G, the motion BM, and/or the activity amount Ex), or like a later described eye movement (EM) monitoring camera 4. The same sensor may then be used in the field, i.e. in the estimation phase. In other words, the activity sensor may be a sensor suitable for obtaining activity parameters related to an emotional state, and this sensor may be used in the learning phase and/or estimation phase (in the field). Further as example, when used in the learning phase in order to gather measurement data for generating the cognition-activity relationship, the activity sensor may be a triaxial acceleration sensor included e.g. in the measuring device 3 (motion BM indicating e.g. hand movement), or an eye movement monitoring (EM) camera 4. In other words, the activity sensor may be a sensor suitable for obtaining activity parameters related to a cognitive state, and this sensor may be used in the learning phase and/or estimation phase (in the field). When the activity sensor and/or its configuration are different for measuring activities depending on whether the measurement is needed for emotion or cognition, respectively, higher accuracy is achieved. Further, the same two different sensors and/or respective configuration can be used both in the learning phase, and in the estimation phase (in the field): this is however not necessary, as in fact in the learning phase different sensors can be used, while in the field such differentiation may not be used so as to obtain an easier system to implement in the field. The opposite situation is also possible, i.e. different sensors are used in the field, but no in the learning phase. Still further, while the described option configuration may be advantageous, there is no need to differentiate between activity sensors relating to emotion and cognition, as in fact also the same activity sensor and/or respective configuration can be used regardless of whether emotion or cognition wants to be learned/estimated; in this case, a simpler system can be implemented.

Further, the second learning data may optionally and preferably comprise data generated on the basis of information indicating performance, the information indicating emotion of at least one worker, and the information indicating cognition of the at least one worker. The information indicating emotion and cognition may be the same as the ones used for generating the first learning data, i.e. it is not necessary to repeat the measurement. However, this is not strictly necessary, as in fact it is possible taking emotion and cognition measurement for the first learning data, and take emotion and cognition measurement separately for the second learning data. The information indicating performance indicate performance in correspondence of the information indicating emotion and the information indicating cognition, wherein the performance can be measured in known ways as also later explained (e.g. how many articles are manufactured in a unit of time, and/or quality level achieved in the manufacturing; accuracy in driving; level of health conditions, etc.).

The learning data herein discussed can be obtained on the basis of one subject, or of a plurality subjects. In case the data are obtained on the basis of only one subject, the only one subject may be the same on which the later performance estimation is performed, but not necessarily. In addition, the activity information and emotion information (on which the learning process is then performed) can be obtained for a given subject, preferably when the subject is performing a certain task (or also herein operation). Further preferably, the certain task belongs to a set of tasks including at least one task characterized by interaction between the subject and a device. For instance, if the device is a vehicle, the task can be represented by a driving operation of the vehicle (a driving type of task), and the activity, cognition and emotion information (necessary for generating the learning data) are obtained when the subject is driving, e.g. by means of sensors and/or sensor configurations compatible with driving. In another example, the task relates to performing an operation on a production line (a manufacturing type of task), and the emotion, cognition and activity information are obtained while the subject(s) performs the task in the production line. In another example, the task relates to an action performed when the subject is coupled to a health care device (a healthcare related type of task), and the emotion, cognition and activity information are obtained when the user performs such action. The learning process can be performed on data referring to activity and emotion information for one or more subjects performing the same or different types of task.

Further, the line manufacturing apparatus may be included in a system, which also includes an article obtained by means of the manufacturing apparatus.

In other illustrative applications like for instance assisted driving or healthcare support, higher safety in driving, more accurate healthcare monitoring, or improved health conditions can be reached on the basis of objective and repeatable measurements, and on specific learning data. What has been said above applies also to the following embodiments, such that repetitions will be avoided.

Embodiments of the present invention will now be described with reference to the drawings.

Embodiment 1

Principle

As anticipated, factors that may influence the productivity of a production line include 4M (machines, methods, materials, and men) factors. In the present embodiment, the factor "men", which may influence the productivity, may be defined as emotion and cognition based on the neural activity of the brain. The emotion is, for example, human motivation and mood (comfort or discomfort) for an operation, and varies during a relatively short period such as hours or days. The cognition is a human baseline ability. This ability is associated with, for example, human attention to and judgment about an operation, and varies during a relatively long period such as months or years.

In the present embodiment, information indicating the human activity correlated with the neural activity of the brain, such as vital signs and motion information, is used as a primary indicator (for example, when using regression analysis, an indicator as herein used can be represented by an independent variable; in other words, information indicating human activity may represent independent variable(s) when using regression analysis). The information indicating the activity and the emotion correct value, as for instance input by the worker, are used to estimate the emotion. Examples of the information indicating the activity include vital signs and motion information such as the heart electrical activity, the skin potential activity, the motion, and the amount of exercise. With emotion correct value it is herein meant a value indicating the emotional state of the person (e.g. worker), which value is considered correct or highly accurate. In other words, the emotion correct value is (preferably, highly) accurate information on the emotional state of a person. The emotion correct value can be obtained, in one example, by means of an emotion input device 2. For simplicity, as later described in the example referring to figure 2, the emotion input device 2 can be represented by a device to which the person (e.g. worker) can input his/her current emotion. However, the emotion input device 2 can be represented for instance by a measurement apparatus and/or sensor (or combination of a plurality of such measurement apparatuses and/or sensors) capable of acquiring an emotion correct value (i.e. a highly accurate information on the emotional state), i.e. by means of suitable measurements made on the subject, see also the above discussion in relation to Figures 22 and 23. In particular and preferably, the correct emotion value is acquired by means of devices suitable for determining such state with high precision/accuracy (regardless of the size and complexity of the sensor or device used; preferably, such sensors are large and complex devices achieving higher accuracy than other sensors as those included in wearables). Also, a combination of both an indirect (by means of accuracte measurements) and direct (e.g. by means of user inputting his/her own state into a device) determination of the emotional state is possible. The correct emotion value herein discussed can be acquired for each of a plurality of workers, as also further later illustrated. In general, the emotion correct value and the cognition correct value can be obtained by at least one emotion sensor, and, respectively, by a cognition sensor, wherein such sensors are as above explained.

The cognition is estimated using, as primary indicators (when using for example regression analysis, the independent variable(s) may be given by such indicator(s)), the feature quantities of, for example, eye movement and hand movement representing the attention and judgment in the information indicating the human activity. The feature quantities of eye movement and hand movement, and the cognition correct value are used to estimate the cognition. Examples of the feature quantities representing eye movement include the eye movement speed, the gaze coordinates and the gaze duration, the number of blinks, and changes in the pupil size. Examples of the feature quantities representing hand movement include triaxial acceleration. With cognition correct value it is herein meant (preferably highly, accurate) information indicative of the cognitive state of the person, which information is acquired by means of one of more apparatuses, devices and/or sensors capable of determining whether an operation by the person is as expected, e.g. whether a detected operation (as acquired by such device/apparatus/sensor) is according to a predetermined pattern and/or template for such operation. An example for such device/apparatus/sensor is given by a work monitoring camera CM also later described. Further examples are given above, see the discussion on cognition sensors. When using for example regression analysis, the cognition correct values may be represented as dependent variable(s). Thus, when using regression analysis for emotion or cognition, a relationship can be found between dependent variable(s) and independent variable(s), wherein the dependent variable(s) represent the correct values for emotion and, respectively, cognition, and the independent variable(s) represent indications of human activity as appropriately measured.

In the present embodiment, the emotion learning data and the cognition learning data are preliminarily generated for each worker. These learning data items are generated based on the above correct values (e.g. dependent variables) and primary indicators (e.g. independent variables). A change in the activity of the worker is measured during operation, and the measurement data is used as a primary indicator. This primary indicator and the learning data are used to estimate a change in each of the emotion and the cognition of the worker. In other words, (first) learning data is generated for instance by regression analysis between activity indication values (independent variables) and correct values (dependent variable) of emotion and, respectively, cognition - on the basis of data available for one or more persons, for instance. Once the learning data has been obtained, the emotion and/or cognition can be estimated on the basis of the (previously generated) learning data and the current activity as detected for a person at a certain point in time when the emotion/estimation wants or needs to be estimated.

In addition, relational expressions representing the correlation between the changes in the emotion and cognition and a change in the worker productivity (or, more in general, correlation between emotion and the cognition, and productivity/performance) are preliminarily generated for each worker as learning data for estimating the productivity. In an example using regression analysis, the performance (or change in performance), may be represented as dependent variable(s). Information indicating performance or change in performance may be obtained for instance by measuring speed of producing an item, and/or how many items are produced per hour, and/or quality in producing item(s), etc. as also later explained. The estimated changes in the worker's emotion and cognition are used as secondary indicators; in the example of regression analysis, the secondary indicator(s) may be represented as independent variable(s). The secondary indicators and the relational expressions are used to estimate a change in the worker's current or future productivity. In other words and as an example, (second) learning data is generated using regression analysis between performance information (as dependent variable(s)) and estimated emotion and/or cognition (as independent variable(s)). Once the (second) learning data is obtained, the actual performance can be estimated based on the emotion and/or cognition as estimated for a person at a certain point in time.

The productivity information is typically defined by the quality and the number of products. In the present embodiment, this information is more specifically represented by skill level information and misoperation frequency information. The skill level information is represented by, for example, a difference between a standard operation time and an actual operation time. The misoperation frequency information is represented by, for example, deviations of the actual operation time from an average operation time.

In the present embodiment, the information about the difference between the standard operation time and the actual operation time, and the information indicating deviations of the actual operation time from the average operation time are estimated for each worker as the productivity information during operation. The estimated productivity information and a predetermined condition for providing an intervention are used to determine the timing and the details of the intervention for the worker.

What has been explained above for a worker, equally applies to persons like a driver, or a person using a healthcare device.

In the case of a driver, for instance, correct values used for cognition estimation may be represented by how correctly the driving task is executed, which can be obtained e.g. by measuring certain driving parameters like how correctly the vehicle follows certain predetermined routes (e.g. comparing how smoothly the actual driving route correspond to an ideal route obtained from a navigation system), how smooth the control of the vehicle is (e.g. whether or how often any sudden change of direction occurs), on the degree of the driver recognizing an obstacle, etc. The performance values of one driver (in the sense of performance in executing driving, to be used for obtaining learning data by way of regression analysis) can e.g. be obtained by comparing for instance the distance covered over a certain period over an expected distance for a given period, or whether in reaching two points a certain route has been followed compared to predetermined available routes, etc.

In the case of a person using a healthcare assistance device, the correct values for cognition estimation may be obtained by measuring how certain tasks are executed: for instance, how straight and balanced the person's body position is when walking, running or sitting (e.g. over predetermined patterns); how smoothly certain movements are made over predetermined patterns; etc. The performance values of the person (to be used for obtaining learning data by way of regression analysis) can e.g. be obtained by measuring efficiency and/or quality in completing a certain task of number of tasks, like for instance measuring the distance covered on foot over an expected distance; measuring the time for accomplishing a task over a predetermined time (e.g. completing a housecleaning or hobby-related operation, number of such operations performed in an hour or day), etc.

Other values and considerations apply as in the case of a worker.

System Configuration

A production management system according to an embodiment of the present invention is a cell production system. The cell production system divides the product manufacturing process into multiple sections. The production line has working areas, called cells, for these sections. In each cell, a worker performs the operation of the assigned section.

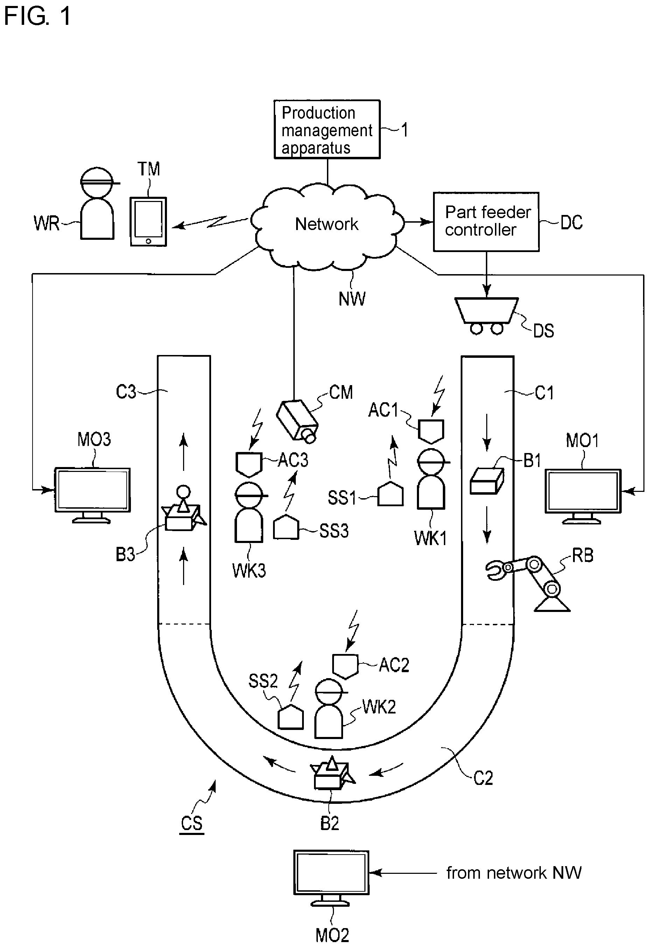

Fig. 1 shows an example cell production system, which includes a U-shaped production line CS. The production line CS includes, for example, three cells C1, C2, and C3 corresponding to different sections on the course of the products. Workers WK1, WK2, and WK3 are assigned to the cells C1, C2, and C3, respectively. In addition, a skilled leader WR is placed to supervise the overall operation on the production line CS. The leader WR has a portable information terminal TM, such as a smartphone or a tablet terminal. The portable information terminal TM is used to display information for managing the production operation provided to the leader WR.

A part feeder DS and a part feeder controller DC are located most upstream of the production line CS. The part feeder DS feeds various parts for assembly onto the line CS at a specified rate in accordance with a feed instruction issued from the part feeder controller DC. Additionally, the cell C1, which is a predetermined cell in the production line CS, has a cooperative robot RB. In accordance with an instruction from the part feeder controller DC, the cooperative robot RB assembles a part into a product B1 in cooperation with the part feed rate.

The cells C1, C2, and C3 in the production line CS have monitors MO1, MO2, and MO3, respectively. The monitors MO1, MO2, and MO3 are used to provide the workers WK1, WK2, and WK3 with instruction information about their operations and an intervention message corresponding to one form of intervention.

A work monitoring camera CM is installed above the production line CS. The work monitoring camera CM captures images to be used for checking the results of the production operations for the products B1, B2, and B3 performed by the workers WK1, WK2, and WK3 in the cells C1, C2, and C3. The results of the production operations are used as correct values when learning data for cognition estimation is generated.

To estimate the emotions and cognition of the workers WK1, WK2, and WK3, the workers WK1, WK2, and WK3 have input and measurement devices SS1, SS2, and SS3, respectively. The input and measurement devices SS1, SS2, and SS3 each include an emotion input device 2 for receiving an emotion correct value, a measurement device 3 and/or an eye monitoring camera 4 for measuring the worker's activity used as a primary indicator for estimating the emotion and cognition.

The emotion input device 2, which is for example a smartphone or a tablet terminal as shown in Fig. 2, displays an emotion input screen under control with application programs. The emotion input screen shows emotions using a two-dimensional coordinate system with emotional arousal on the vertical axis and emotional valence on the horizontal axis. When a worker plots the position corresponding to his or her current emotion on the emotion input screen, the emotion input device 2 recognizes the coordinates indicating the plot position as information indicating the emotion of the worker.

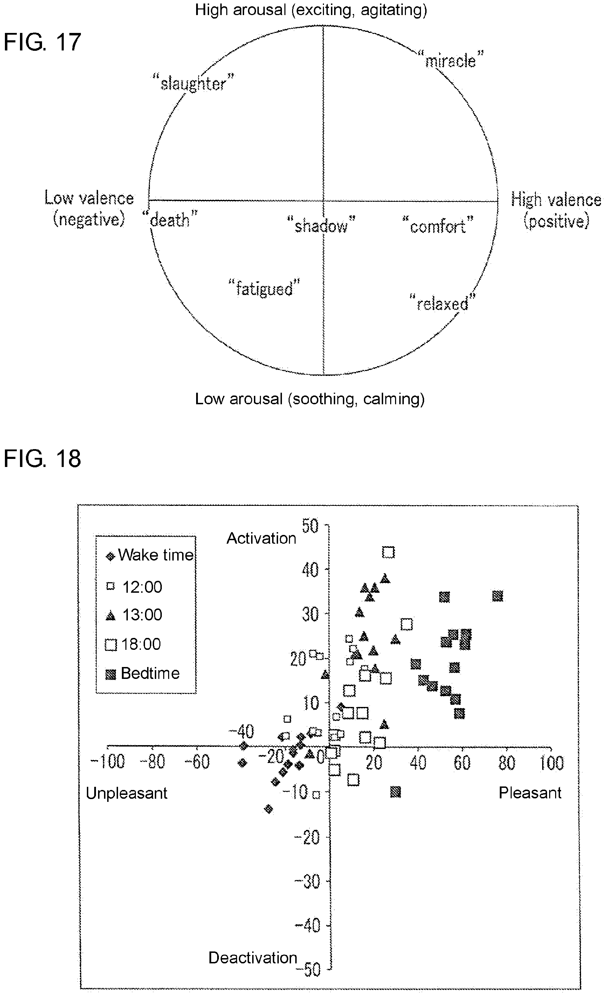

This technique of expressing the emotions using arousal and valence on the two-dimensional coordinate system is known as the Russell's circumplex model. Fig. 17 schematically shows this model. Fig. 18 is a diagram showing example input results of emotion at particular times obtained through the emotion input device 2. The arousal indicates the emotion either being activated or deactivated and the degree of activation to deactivation, whereas the valence indicates the emotion either being comfortable (pleasant) or uncomfortable (unpleasant) and the degree of being comfortable to uncomfortable.

The emotion input device 2 transforms the position coordinates detected as the emotion information to the arousal and valence values and the information about the corresponding quadrant of the two-dimensional arousal-valence coordinate system. The resultant data, to which the time stamp data indicating the input date and time is added, is transmitted as emotion input data (hereinafter referred to as scale data) to a production management apparatus 1 through a network NW using a wireless interface. However, as above explained, the emotional state can be obtained by other emotion sensors (as above explained) than the device 2, or in combination with device 2.

The measurement device 3 (an example of an activity sensor, in particular an example of an activity sensor used when learning and/or an example of an activity sensor used when estimating) is, for example, incorporated in a wearable terminal, and is mounted on a wrist of the worker as shown in Fig. 3. The measurement device 3 may not be incorporated in a wearable terminal, and may be mountable on clothes, a belt, or a helmet. The measurement device 3 measures information indicating human activity correlated with human emotions and cognition. The information indicating human activity includes vital signs and motion information. To measure the vital signs and the motion information, the measurement device 3 includes various vital sign sensors and motion sensors. Examples of the vital sign sensors and the motion sensors include sensors for measuring heart electrical activity H, skin potential activity G, motion BM, and an activity amount Ex.

The heart electrical activity sensor measures the heart electrical activity H of the worker in predetermined cycles or at selected timing to obtain the waveform data, and outputs the measurement data. The skin potential activity sensor, which is for example a polygraph, measures the skin potential activity G of the worker in predetermined cycles or at selected timing, and outputs the measurement data. The motion sensor, which is for example a triaxial acceleration sensor, measures the motion BM, and outputs the triaxial acceleration measurement data indicating hand movement of the worker. The sensor for measuring the activity amount Ex, which is an activity sensor, outputs the measurement data indicating the intensity of physical activity (metabolic equivalents, or METs) and the amount of physical activity (exercise). Another example of the vital sign sensors may be an electromyograph for measuring electric charge in the muscle.

The eye movement monitoring camera 4 (an example of an activity sensor, in particular an example of an activity sensor used when learning and/or an example of an activity sensor used when estimating cognition; also, the EM camera 4 can be optionally and preferably used as an activity sensor for detecting activity information relating to cognition, as above explained) is a small image sensor, and is mounted on, for example, the cap worn by each of the workers WK1, WK2, and WK3 as shown in Fig. 3, or on the frame of glasses or goggles. The eye movement monitoring camera 4 captures the eye movement (EM) of the worker, and transmits the captured image data to the production management apparatus 1 as measurement data.

Each of the measurement device 3 and the eye movement monitoring camera 4 adds the time stamp data indicating the measurement date and time to its measurement data. The measurement device 3 and the eye movement monitoring camera 4 each transmit the measurement data to the production management apparatus 1 through the network NW using a wireless interface.

The wireless interface complies with, for example, low-power wireless data communication standards such as wireless local area networks (WLANs) and Bluetooth (registered trademark). The interface between the emotion input device 2 and the network NW may be a public mobile communication network, or a signal cable such as a universal serial bus (USB) cable.

To provide a tactile intervention for the workers WK1, WK2, and WK3, the workers WK1, WK2, and WK3 have stimulus devices AC1, AC2, and AC3, respectively. The stimulus devices AC1, AC2, and AC3 include, for example, a vibrator, and vibrate in response to a drive signal transmitted from the production management apparatus 1 described below.

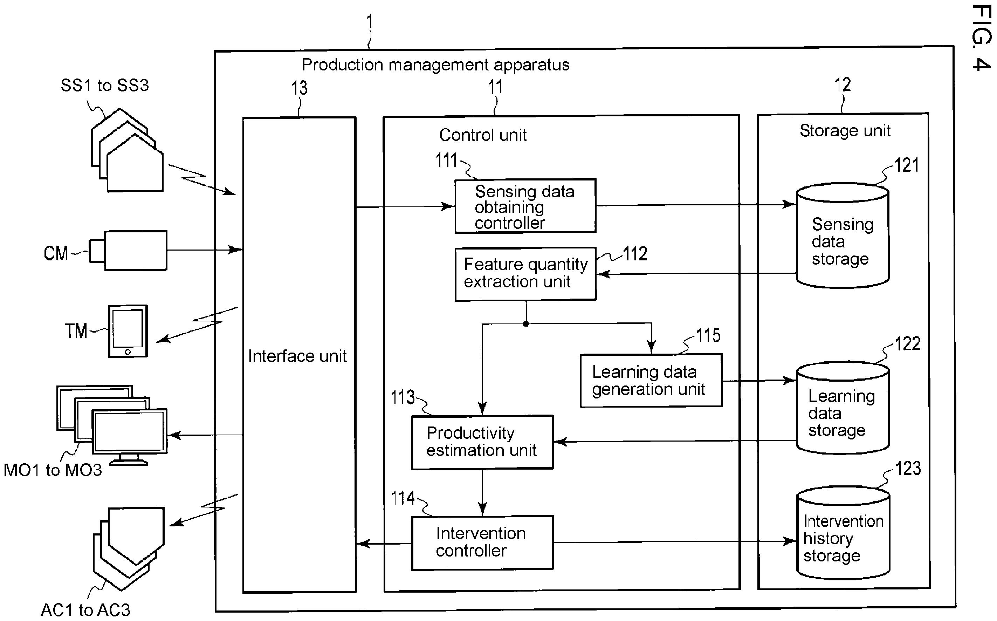

The structure of the production management apparatus 1 will now be described. Fig. 4 is a functional block diagram of the apparatus. The production management apparatus 1 is, for example, a personal computer or a server computer, and includes a control unit 11, a storage unit 12, and an interface unit 13.

The interface unit 13, which allows data communication in accordance with a communication protocol defined by the network NW, receives the measurement data transmitted from the input and measurement devices SS1, SS2, and SS3 through the network NW. The interface unit 13 transmits display data output from the control unit 11 to the portable information terminal TM and the monitors MO1, MO2, and MO3, and also transmits a control command for the production line CS output from the control unit 11 to the part feeder controller DC. The interface unit 13 also includes a man-machine interface function. The man-machine interface function receives data input from an input device, such as a keyboard or a mouse, and outputs display data input from the control unit 11 to a display (not shown) on which the data will appear.

The storage unit 12 is a storage medium, and is a readable and writable non-volatile memory, such as a hard disk drive (HDD) or a solid state drive (SSD). The storage unit 12 includes a sensing data storage 121, a learning data storage 122, and an intervention history storage 123 as storage areas used in the embodiment.

The sensing data storage 121 stores data transmitted from the input and measurement devices SS1, SS2, and SS3 in a manner associated with the identifiers of the workers WK1, WK2, and WK3 that have transmitted the corresponding data. The transmitted and stored data includes scale data indicating the worker's emotion input through the emotion input device 2, measurement data obtained through the sensors of the measurement device 3, and image data input from the eye movement monitoring camera 4. The sensing data storage 121 also stores image data about the results of the operation for a product transmitted from the work monitoring camera CM.

The learning data storage 122 stores learning data to be used for emotion estimation, learning data to be used for cognition estimation, and learning data to be used for productivity estimation, which are generated by the control unit 11 for each of the workers WK1, WK2, and WK3.

The intervention history storage 123 stores information indicating the results of an intervention provided for one of the workers WK1, WK2, and WK3 by the control unit 11, or information indicating the timing and the details of the intervention as an intervention history event.

The control unit 11 includes a central processing unit (CPU) and a working memory. The control unit 11 includes a sensing data obtaining controller 111, a feature quantity extraction unit 112, a productivity estimation unit 113, an intervention controller 114, and a learning data generation unit 115 as control functions used in the embodiment. Each of these control functions is implemented by the CPU executing the application programs stored in program memory (not shown).

The sensing data obtaining controller 111 obtains, through the interface unit 13, data transmitted from each of the input and measurement devices SS1, SS2, and SS3, or scale data output from the emotion input device 2, measurement data output from the measurement device 3, and image data output from the eye movement monitoring camera 4, and stores the obtained data into the sensing data storage 121. The sensing data obtaining controller 111 also obtains, through the interface unit 13, work monitoring image data about the results of the operations performed by the workers WK1, WK2, and WK3 transmitted from the work monitoring camera CM, and stores the obtained data into the sensing data storage 121.

In a learning mode, the feature quantity extraction unit 112 reads, from the sensing data storage 121, the scale data, the measurement data, and the image data for each of the workers WK1, WK2, and WK3 within each of the windows that are arranged at time points chronologically shifted from one another. The feature quantity extraction unit 112 extracts the feature quantities (extracted data, or extracted sensing data) from the read scale data, measurement data, and image data, calculates the variation between the feature quantities, and transmits the calculation results to the learning data generation unit 115.

The windows each have a predetermined unit duration. The windows are defined in a manner shifted from one another by the above unit duration to avoid overlapping between chronologically consecutive windows, or in a manner shifted by a time duration shorter than the above unit duration to allow overlapping between chronologically consecutive windows. The unit duration of each window may be varied by every predetermined value within a predetermined range.

The learning data generation unit 115 performs multiple regression analysis for each of the workers WK1, WK2, and WK3 with correct values

(supervisory data) being the variations among the feature quantities in the scale data for arousal and for valence that are extracted by the feature quantity extraction unit 112 and variables being the variations among the feature quantities of the measurement data. This generates first regression equations for arousal and for valence representing the relationship between the emotion and the feature quantities of measurement data. The learning data generation unit 115 associates the generated regression equations with window identifiers that indicate the time points of the corresponding windows, and stores the equations into the learning data storage 122 as learning data to be used for emotion estimation.

The learning data generation unit 115 also performs multiple regression analysis for each of the workers WK1, WK2, and WK3 with correct values being the operation result data extracted from the captured image data obtained through the work monitoring camera CM (e.g. whether the images acquired by the camera are according to a predetermined pattern or template for an operation performed by the worker) and variables being the eye movement data and hand movement data. The eye movement data is extracted by the feature quantity extraction unit 112 from the captured image data obtained through the eye movement monitoring camera 4. The hand movement data is extracted by the feature quantity extraction unit 112 from the measurement data obtained through the triaxial acceleration sensor included in the measurement device 3. In this manner, the learning data generation unit 115 generates a second regression equation for each of the workers WK1, WK2, and WK3 representing the relationship between the cognition, and the eye movement and hand movement of each worker. The learning data generation unit 115 stores the generated second regression equations into the learning data storage 122 as learning data to be used for cognition estimation.

The learning data generation unit 115 further uses the estimated changes in the emotion and the cognition of each of the workers WK1, WK2, and WK3 as secondary indicators, and generates a relational expression for each worker representing the correlation between each secondary indicator and a change in the productivity of each worker. The learning data generation unit 115 stores the generated relational expressions into the learning data storage 122 as learning data to be used for productivity estimation.

More specifically, skill level information and misoperation frequency information are defined as productivity information. The skill level information is represented by, for example, a difference between a standard operation time and an actual operation time. The misoperation frequency information is represented by, for example, deviations of the actual operation time from an average operation time. The learning data generation unit 115 generates relational expressions for estimating the skill level information and the misoperation frequency information based on the estimates of the changes in the emotion and the cognition, and stores the relational expressions into the learning data storage 122.

In a productivity estimation mode, the feature quantity extraction unit 112 reads, from the sensing data storage 121, the measurement data and the image data for each of the workers WK1, WK2, and WK3 within each of the windows that are arranged at time points chronologically shifted from one another. The feature quantity extraction unit 112 extracts the changes in the feature quantities from the read measurement data and image data for emotion and cognition estimation, and transmits the changes in the feature quantities to the productivity estimation unit 113.

For each of the workers WK1, WK2, and WK3, the productivity estimation unit 113 receives the changes in the feature quantities for emotion and cognition estimation extracted by the feature quantity extraction unit 112, and reads the first regression equations for estimating the emotion and the second regression equations for estimating the cognition from the learning data storage 122. The productivity estimation unit 113 uses the changes in the feature quantities received from the feature quantity extraction unit 112 and the first and second regression equations to estimate a change in each of the emotion and the cognition.

For each of the workers WK1, WK2, and WK3, the productivity estimation unit 113 also reads the relational expressions for productivity estimation from the learning data storage 122. The productivity estimation unit 113 uses the read relational expressions and the estimates of the changes in the emotion and the cognition to estimate the productivity of each of the workers WK1, WK2, and WK3. More specifically, the productivity estimation unit 113 estimates the difference between the standard operation time and the actual operation time representing the skill level, and the deviations of the actual operation time from the average operation time representing the misoperation frequency.

For each of the workers WK1, WK2, and WK3, the intervention controller 114 compares the productivity estimation results from the productivity estimation unit 113 with a predetermined condition for providing an intervention, and determines an intervention to be provided to each of workers so as to increase productivity. For example and preferably, the timing and/or the details of intervention for each of the workers WK1, WK2, and WK3 are determined based on the comparison results.

Examples of the intervention include a visual or auditory stimulus to the worker, a tactile stimulus to the worker, and an instruction to the worker to stop the operation (or rest). Further, intervention can be optionally defined or identified according to characteristics (or parameters) like for instance: type of intervention (e.g. audio, visual, audiovisual, tactile, etc. type), intensity (e.g. volume strength of audiovisual intervention, strength of tactile intervention, light intensity of visual intervention, etc.), timing (during operation, after, etc.), time frequency of application of the intervention, etc. Thus, the device is capable of determining one or more intervention characteristics (i.e. any combination of the characteristics/parameters of an intervention) depending on the estimated productivity/performance, e.g. as a function of the estimated performance. For instance, if the estimated performance is low (e.g. below a predetermined threshold at a certain point in time, of for a certain time interval), a characteristic like increased strength or increased time frequency is chosen; further as example, if productivity is low (e.g. below a predetermined threshold at a certain point in time, of for a certain time interval), an intervention is chosen which has different characteristic (e.g. of a different type) than those of a previously applied intervention. The intervention controller 114 selects one of the intervention (preferably, its details or characteristics/parameters as just illustrated) depending on the number of interventions already provided, and displays an intervention message on the monitor MO1, MO2, or MO3 or drives the stimulus device AC1, AC2, or AC3 to vibrate.

Instead of or in addition to displaying an intervention message on the monitor MO1, MO2, or MO3, a synthetic voice message or a chime may be produced. Operation

The operation of the production management apparatus 1 with the above structure will now be described in association with the operation of the overall system.

(1) Learning Data Generation

Before the process for estimating the productivity of the workers WK1, WK2, and WK3, the production management apparatus 1 generates, for each of the workers WK1, WK2, and WK3, the learning data to be used for productivity estimation in the manner described below.

1-1: Generation of Learning Data for Emotion Estimation

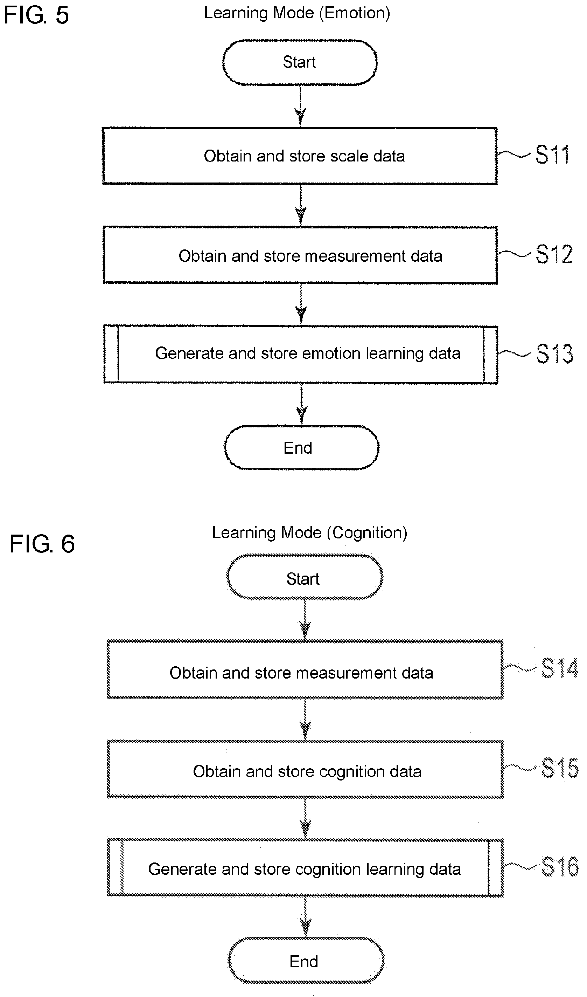

The production management apparatus 1 generates, for each of the workers WK1, WK2, and WK3, the learning data to be used for emotion estimation in the manner described below. Fig. 5 is a flowchart showing the procedure and its details.

More specifically, each of the workers WK1, WK2, and WK3 inputs his or her current emotions with the emotion input device 2 at predetermined time intervals or at selected timing while working.

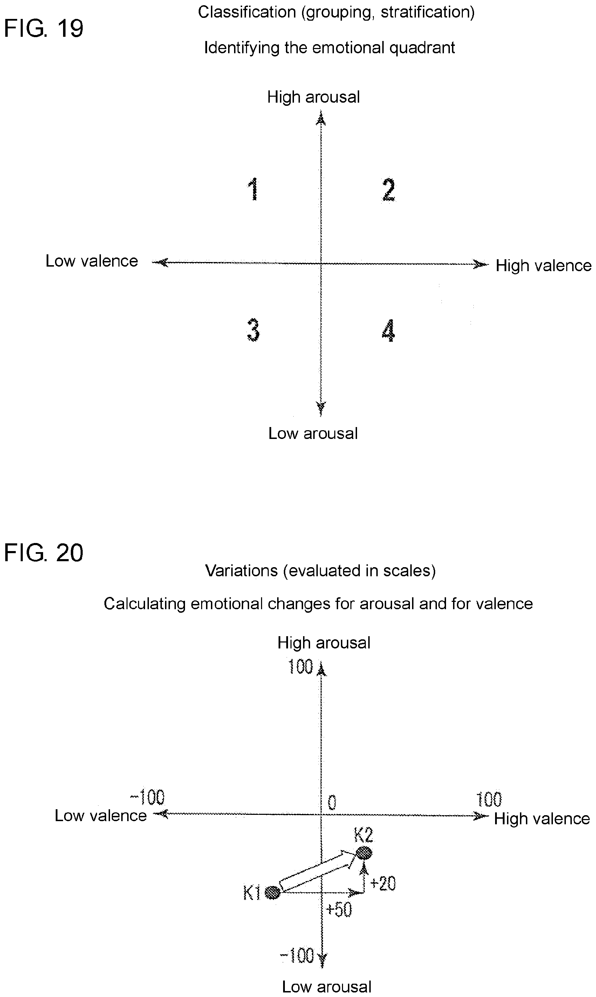

As described above, the emotion input device 2 displays the emotion of the worker in the two-dimensional coordinate system for emotional arousal and emotional valence, and detects the coordinates of a position plotted by the worker WK1, WK2, or WK3 on the two-dimensional coordinate system. The two-dimensional coordinate system used in the emotion input device 2 has the four quadrants indicated by 1, 2, 3, and 4 as shown in Fig. 19, and the arousal and valence axes each representing values from -100 to +100 with the intersection point as 0 as shown in Fig. 20. The emotion input device 2 transforms the detected coordinates to the information about the corresponding quadrant and to the corresponding values on both the arousal and valence axes. The emotion input device 2 adds the time stamp data indicating the input date and time and the identifier (worker ID) of the worker WK1, WK2, or WK3 to the resultant information, and transmits the data to the production management apparatus 1 as scale data. As above illustrated, the emotion input device 2 is not limited to a device to which the worker inputs his/her emotion (which is herein described for simplicity only), but includes in fact also devices capable of accurately determining an emotion state on the basis of accurate measurement(s).

In parallel with this, the measurement device 3 measures the heart electrical activity H, the skin potential activity G, the motion BM, and the activity amount Ex of the worker WK1, WK2, or WK3 at predetermined time intervals. The measurement data is transmitted to the production management apparatus 1 together with the time stamp data indicating the measurement time and the worker ID of the worker WK1, WK2, or WK3. Additionally, the eye movement EM of the worker WK1, WK2, or WK3 is captured by the eye movement monitoring camera 4. The image data is also transmitted to the production management apparatus 1 together with the time stamp data and the identifier (worker ID) of the worker WK1, WK2, or WK3.

In step S11, the production management apparatus 1 receives, for each of the workers WK1, WK2, and WK3, the scale data transmitted from the emotion input device 2 though the interface unit 13 as controlled by the sensing data obtaining controller 111, and stores the received scale data into the sensing data storage 121.

In step S12, the production management apparatus 1 also receives, for each of the workers WK1, WK2, and WK3, the measurement data transmitted from the measurement device 3 and the image data transmitted from the eye movement monitoring camera 4 through the interface unit 13 as controlled by the sensing data obtaining controller 111, and stores the received measurement data and image data into the sensing data storage 121.

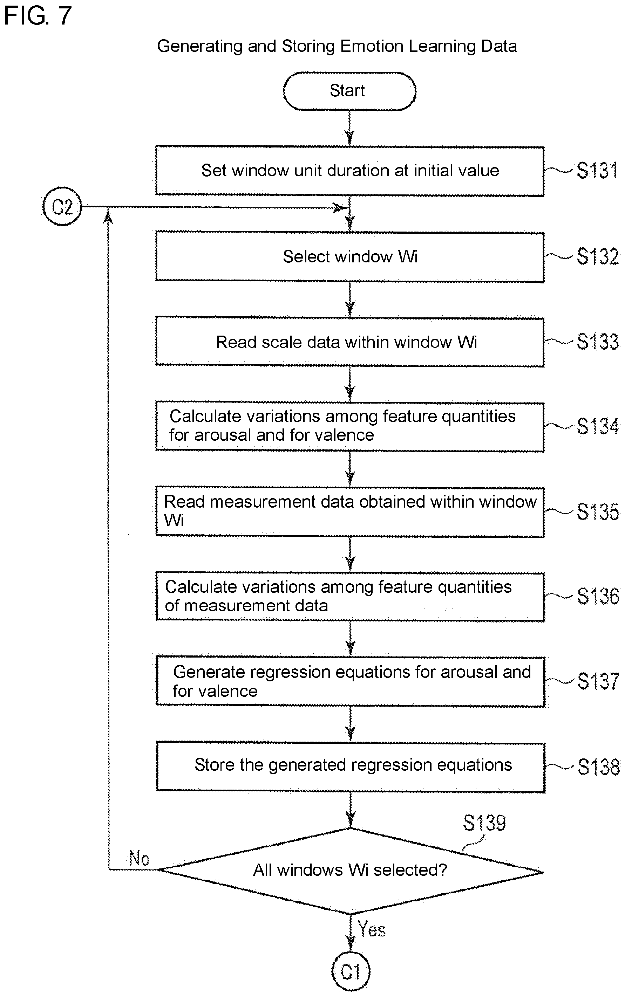

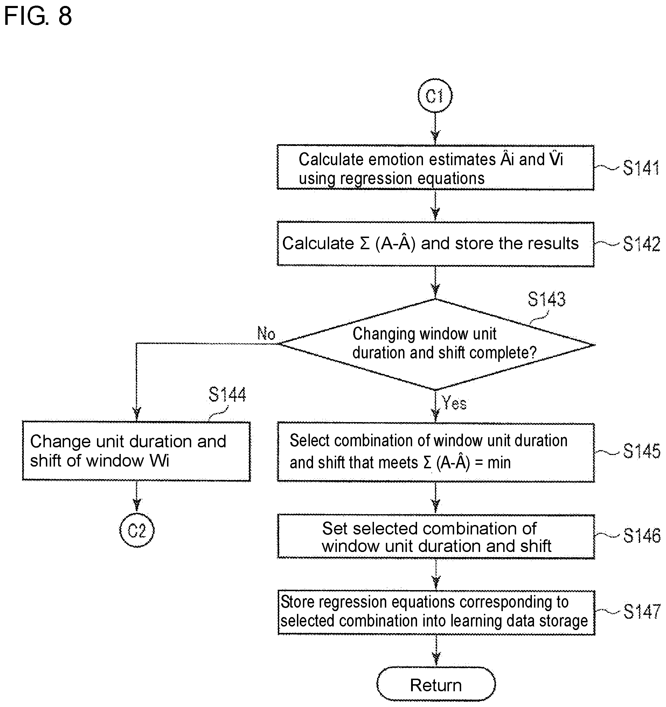

In step S13, when the scale data, the measurement data, and the image data accumulate for a predetermined period (e.g., one day or one week), the production management apparatus 1 generates learning data to be used for emotion estimation, as controlled by the feature quantity extraction unit 112 and the learning data generation unit 115 in the manner described below. Figs. 7 and 8 are flowcharts showing the procedure and its details.

In step S131, the unit duration of the window Wi (i = 1, 2, 3, ...) is set at an initial value. In step S132, the first window (i = 1) is selected. In step S133, the feature quantity extraction unit 112 reads a plurality of sets of scale data within the first window from the sensing data storage 121. In step S134, the feature quantity extraction unit 112 calculates the variations among the feature quantities for arousal and for valence.

For example, when scale data K1 and scale data K2 are input within the unit duration of one window as shown in Fig. 20, the variations are calculated as the change from the third to the fourth quadrant, and as the increment of 20 (+20) for arousal and the increment of 50 (+50) for valence. For a change to a diagonally opposite quadrant, for example, for a change from the third to the second quadrant, the variations among the resultant feature quantities may be calculated for arousal and for valence.

In step S135, the feature quantity extraction unit 112 reads the measurement data and image data obtained within the unit duration of the first window, which are the measurement data about the heart electrical activity H, the skin potential activity G, the motion BM, and the activity amount Ex, and the image data about the eye movement EM, from the sensing data storage 121. In step S136, the feature quantity extraction unit 112 extracts the feature quantities from the measurement data and the image data.

For example, the heart electrical activity H has the feature quantities that are the heartbeat interval (R-R interval, or RRI), and the high frequency components (HF) and the low frequency components (LF) of the power spectrum of the RRI. The skin potential activity G has the feature quantity that is the galvanic skin response (GSR). The motion BM has feature quantities including the hand movement directions and speed. The hand movement directions and speed are calculated based on, for example, the triaxial acceleration measured by the triaxial acceleration sensor. The activity amount Ex has the feature quantities that are the intensity of physical activity (METs) and the exercise (EX). The exercise (EX) is calculated by multiplying the intensity of physical activity (METs) by the activity duration. The eye movement EM has the feature quantities including the eye movement speed, the gaze coordinates and the gaze duration, the number of blinks, and changes in the pupil size.

The feature quantity extraction unit 112 calculates the variations among the extracted feature quantities that are the heart electrical activity H, the skin potential activity G, the motion BM, the activity amount Ex, and the eye movement EM within the unit duration of the window.

In step S137, the learning data generation unit 115 generates learning data for arousal and learning data for valence based on the variations calculated in step S134 among the scale data feature quantities and the variations calculated in step S136 among the measurement data and image data feature quantities.

For example, the learning data generation unit 115 performs multiple regression analysis using the variations among the scale data feature quantities for arousal and for valence as supervisory data, and the variations among the measurement data and image data feature quantities as independent variables, which are primary indicators. The learning data generation unit 115 then generates regression equations for each of the workers WK1, WK2, and WK3 for arousal and for valence representing the relationship between the change in the emotion of each worker and the changes in the measurement data and image data feature quantities.

The regression equations corresponding to the i-th window are as follows:

$$X\hat{A}i = f(\alpha 1Hi, \alpha 2Gi, \alpha 3EMi, \alpha 4BMi, \alpha 5Exi),$$

and

$$X\hat{V}i = f(\alpha 1Hi, \alpha 2Gi, \alpha 3EMi, \alpha 4BMi, \alpha 5Exi) \quad (1)$$

where $X\hat{A}i$ is the estimate of the arousal change, $X\hat{V}i$ is the estimate of the valence change, $\alpha 1$, $\alpha 2$, $\alpha 3$, $\alpha 4$, and $\alpha 5$ are the weighting coefficients for the feature quantities of the measurement data items Hi, Gi, EMi, BMi, and Ex, and f is the sum of the indicators obtained from the feature quantities of the measurement data items Hi, Gi, EMi, BMi, and Ex, which are primary indicators. The weighting coefficients may be determined by using, for example, the weighted average based on the proportions in the population data obtained in the learning stage. Equations (1) are an example of a relationship between activity and emotion of a person. In one example, first learning data (also above discussed) may include, indicate or be based on equations (1) above, representing a relationship between activity and emotion.

In step S138, the learning data generation unit 115 stores the generated regression equations for arousal and for valence corresponding to the i-th window into the learning data storage 122. In step S139, the learning data generation unit 115 determines whether all the windows Wi have been selected for generating regression equations. When any window remains unselected, the processing returns to step S132, where the unselected window is selected, and the processing in steps S133 to S139 for generating the learning data for emotion estimation is repeated for the next selected window.

The feature quantity extraction unit 112 and the learning data generation unit 115 change the window unit duration by every predetermined value and the chronological shift of the window by every predetermined amount to determine the optimum window unit duration and the optimum shift. Of all the combinations of the unit durations and the shifts, the learning data generation unit 115 selects a combination that minimizes the difference between the emotion estimates obtained using the regression equations and the emotion information correct values input through the emotion input device 2. The learning data generation unit 115 then sets, for the emotion estimation, the selected window unit duration and the selected shift, as well as the regression equations generated for this combination.

An example of the processing of selecting the optimum window will now be described. Fig. 8 is a flowchart showing the procedure and its details.

In step S141, the learning data generation unit 115 calculates the emotion estimates $X\hat{A}i$ and $X\hat{V}i$ using the regression equations generated for each window Wi, and calculates the sum of the calculated estimates $X\hat{A}i$ as $X\hat{A}$ and the sum of the calculated estimates $X\hat{V}i$ as $X\hat{V}$. In step S142, the learning data generation unit 115 calculates the differences between the sums of the emotion estimates $X\hat{A}$ and $X\hat{V}$, and the sums of the true values XA and XV of the emotion information input through the emotion input device 2 in the manner described below.

$$\Sigma(XA - X\hat{A}) \text{ and } \Sigma(XV - X\hat{V})$$

The calculation results are stored into the learning data storage 122. For simplifying the flowchart, Fig. 8 only shows $\Sigma(XA - X\hat{A})$.

In step S143, the learning data generation unit 115 determines whether changing the window unit duration and the shift has been complete, or in other words, whether regression equations have been generated for all combinations of the window unit durations and the shifts. When this process is incomplete, the processing advances to step S144, in which the unit duration and the shift of the window Wi is changed by the predetermined amount. The processing then returns to step S132 shown in Fig. 7, and then the processing in steps S132 to S143 is performed. In this manner, the processing in steps S132 to S144 is repeated until the regression equations are generated for all the combinations of the window unit durations and the shifts.

When the regression equations have been generated for all the combinations of the window unit durations and the shifts, the learning data generation unit 115 compares the differences, calculated for all the combinations of the window unit durations and the shifts, between the sums of the emotion information true values XA and XV, and the sums of the emotion estimates $X\hat{A}$ and $X\hat{V}$, which are $\Sigma(XA - X\hat{A})$ and $\Sigma(XV - X\hat{V})$, in step S145. The learning data generation unit 115 then selects the combination of the window unit duration and the shift that minimizes the values of $\Sigma(XA - X\hat{A})$ and $\Sigma(XV - X\hat{V})$.

In step S146, the learning data generation unit 115 sets the selected combination of the window unit duration and the shift in the feature quantity extraction unit 112. In step S147, the learning data generation unit 115 stores the regression equations corresponding to the selected combination into the learning data storage 122. The process of generating the learning data to be used for emotion estimation ends.

1-2: Generation of Learning Data for Cognition Estimation

The learning data generation unit 115 generates the learning data to be used for cognition estimation in the manner described below. Fig. 6 is a flowchart showing the procedure and its details.

More specifically, the motion BM of each of the workers WK1, WK2, and WK3 indicating hand movement is measured by the triaxial acceleration sensor included in the measurement device 3. The measurement data is then transmitted to the production management apparatus 1. In parallel with this, the eye movement EM indicating eye movement during operation is captured by the eye movement monitoring camera 4. The captured image data is transmitted to the production management apparatus 1.

In step S14, the production management apparatus 1 receives, for each of the workers WK1, WK2, and WK3, the measurement data about the motion BM indicating the hand movement transmitted from the measurement device 3 and the image data about the eye movement EM transmitted from the eye movement monitoring camera 4 through the interface unit 13 as controlled by the sensing data obtaining controller 111, and stores the received measurement data and image data into the sensing data storage 121. The measurement data about the motion BM and the image data about the eye movement EM may be the corresponding data obtained during the process of generating the learning data to be used for emotion estimation.

In the cells C1, C2, and C3 of the production line CS, the results of the operations performed by the workers WK1, WK2, and WK3 are captured by the work monitoring camera CM. The captured image data is transmitted to the production management apparatus 1. In step S15, the production management apparatus 1 receives the image data transmitted from the work monitoring camera CM through the interface unit 13 as controlled by the sensing data obtaining controller 111, and stores the received image data into the sensing data storage 121.

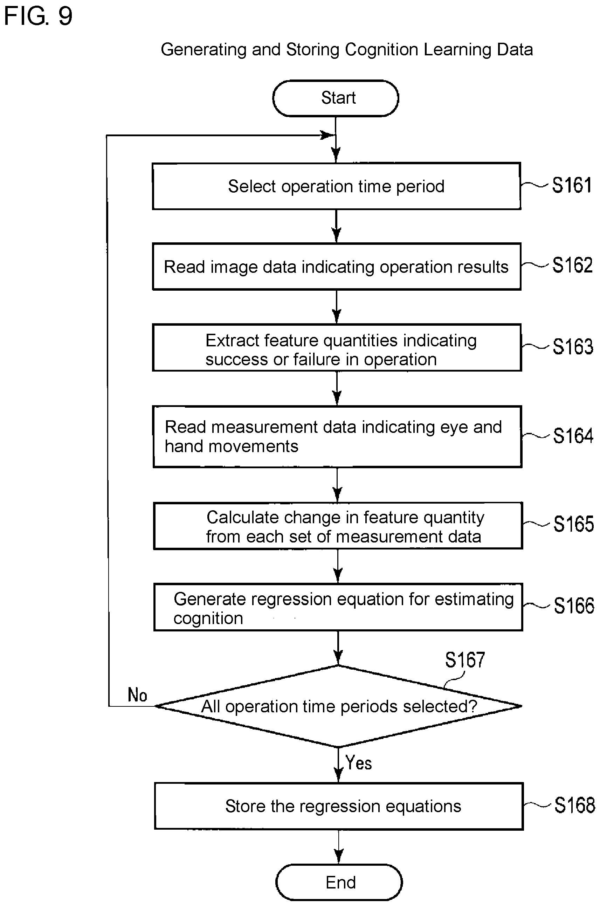

In step S16, the production management apparatus 1 generates the learning data to be used for cognition estimation as controlled by the feature quantity extraction unit 112 and the learning data generation unit 115 in the manner described below. Fig. 9 is a flowchart showing the procedure and its details.

In step S161, the production management apparatus 1 selects an operation time period (e.g., one day or one week). In step S162, the feature quantity extraction unit 112 reads the image data indicating the operation results from the sensing data storage 121. In step S163, the feature quantity extraction unit 112 extracts the feature quantities indicating the success or failure in the operation from the read image data indicating the operation results by, for example, pattern recognition (this is an example of obtaining correct values indicating whether the operation results suggest a correctly performed operation, wherein images taken by a camera are compared to a pattern to establish whether the operation was correctly performed or not). The feature quantities are, for example, represented by the number or incidence of misoperations during the selected time period. The feature quantity extraction unit 112 uses the extracted feature quantities as correct values of the cognition.

In step S164, the feature quantity extraction unit 112 reads the measurement data obtained by the triaxial acceleration sensor included in the measurement device 3. In step S165, the feature quantity extraction unit 112 extracts the feature quantities indicating the hand movement of the worker from the read measurement data. In parallel with this, the feature quantity extraction unit 112 reads the image data obtained through the eye movement monitoring camera 4 in step S164, and extracts the feature quantities indicating the eye movement of the worker (eye movement EM) from the read image data in step S165. The extracted eye movement EM is represented by, for example, the eye movement speed, the gaze coordinates and the gaze duration, the number of blinks, and changes in the pupil size as described above. The feature quantities of the motion BM and the eye movement EM may be the corresponding feature quantities extracted during the process of generating the learning data to be used for emotion estimation.

In step S166, the learning data generation unit 115 performs multiple regression analysis with correct values (supervisory data) being the feature quantities indicating the success or failure in the operation and variables being the feature quantities indicating the hand movement and the feature quantities indicating the eye movement EM. This generates a regression equation. The learning data generation unit 115 stores the generated regression equation into the learning data storage 122 as learning data to be used for cognition estimation. An example regression equation used for cognition estimation is as follows:

$$\hat{Y}i = f(\beta 1 EMi, \ \beta 2 BMi) \ (2)$$

where Yi is the estimate of the cognition change, $\beta 1$ is the weighting coefficient for the feature quantities of the eye movement EMi, β2 is the weighting coefficient for the feature quantities of the motion BMi, and f is the sum of the indicators obtained from the feature quantities of the eye movement EMi and the motion BMi, which are primary indicators. The weighting coefficients may be determined by using, for example, the weighted average based on the proportions in the population data obtained in the learning stage. Equation (2) is an example of a relationship between activity and cognition. In one example, first learning data (also above discussed) may include, indicate or be based on equation (2) above, indicating in fact a relationship between activity and cognition. In a further example, first learning data (also above discussed) may include, indicate or be based on equations (1) and equation (2) above.

In step S167, the learning data generation unit 115 determines whether all the operation time periods have been selected for generating regression equations. When any operation time period remains unselected, the processing returns to step S161, and the regression equation generation process is repeated. When the regression equations have been generated for all the operation time periods, the learning data generation unit 115 associates, in step S168, the generated regression equations with the information indicating their corresponding operation time periods, and stores the regression equations into the learning data storage 122.

1-3: Generation of Learning Data for Productivity Estimation

When the learning data for emotion estimation and the learning data for cognition estimation have been generated for each of the workers WK1, WK2, and WK3, the learning data generation unit 115 generates the learning data to be used for productivity estimation in the manner described below.

More specifically, the learning data generation unit 115 defines the productivity information by using skill level information and misoperation frequency information. The skill level information is represented by, for example, a difference between a standard operation time and an actual operation time. The misoperation frequency information is represented by deviations of the actual operation time from an average operation time.

The learning data generation unit 115 uses the emotion estimates and the cognition estimates as secondary indicators, and generates a relational expression for estimating the skill level of the worker based on the difference between the current and past secondary indicators. An example of the relationship is described below.

A skill level Quality-A is expressed using the formula below.

$$\text{Quality-A} = \sqrt{\{(\gamma a1(X2 - x1))^2\}} + \sqrt{\{(\gamma a2(Y2 - y1))^2\}} \ (3)$$

In the formula, x1 is the current emotion estimate, y1 is the current cognition estimate, X2 is the average of past emotion estimates, Y2 is the average of past cognition estimates, γa1 is the weighting coefficient for emotion, and γa2 is the weighting coefficient for cognition.

The learning data generation unit 115 also uses the emotion estimates and the cognition estimates as secondary indicators, and generates a relational expression for estimating the misoperation frequency of the worker based on the variatons among the past and current secondary indicators. An example of the relationship is described below.

A misoperation frequency Quality-B is expressed using the formula below.

$$\text{Quality-B} = \gamma b1\sqrt{\{((X1 - x1) / \Sigma(X - xi))^2\}} + \gamma b2\sqrt{\{((Y1 - y1) / \Sigma(Y - yi))^2\}} \ (4)$$

In the formula, x1 is the current emotion estimate, y1 is the current cognition estimate, X1 is the average of past emotion estimates, Y1 is the average of past cognition estimates, γb1 is the weighting coefficient for emotion, and γb2 is the weighting coefficient for cognition.

The weighting coefficients γa1, γa2, γb1, and γb2 may be determined for each of the workers WK1, WK2, and WK3 by using, for example, multiple regression analysis or questionnaires to the workers WK1, WK2, and WK3. In one example, each or both equations (3) and (4) indicate a relationship between performance, and emotion and cognition. In a further example, second learning data (also above discussed) may include, indicate or be based on equation (3) and/or (4) above, indicating in fact a relationship between performance and activity.

(2) Productivity Estimation

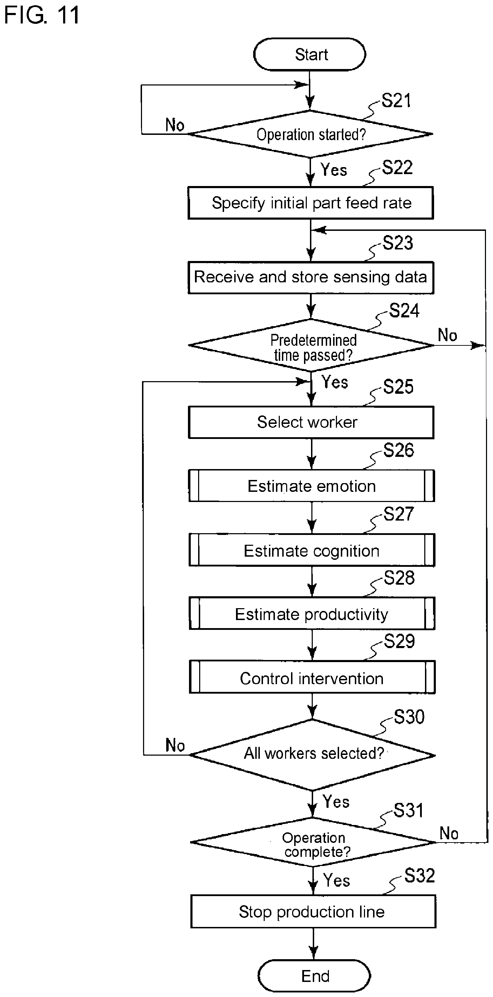

After the learning data for productivity estimation is generated, the production management apparatus 1 uses the learning data to estimate the productivity of the workers WK1, WK2, and WK3 during operation in the manner described below. Fig. 11 is a flowchart showing the estimation process and its details.

2-1: Collecting Worker's Sensing Data

When detecting an input operation start command in step S21, the production management apparatus 1 specifies an initial part feed rate in the part feeder controller DC in accordance with the preliminarily input information specifying the production amount (e.g., 100 products/day) in step S22. The part feeder controller DC then instructs the part feeder DS to feed the sets of parts for the products to be manufactured to the production line CS at the specified rate. In response to the fed sets of parts, the workers WK1, WK2, and WK3 in their assigned cells start their operations for assembling products.

During the operation, the measurement device 3 in each of the input and measurement devices SS1, SS2, and SS3 of the workers WK1, WK2, and WK3 measures the heart electrical activity H, the skin potential activity G, the motion BM, and the activity amount Ex of the worker at predetermined time intervals or at selected timing. The measurement data is transmitted to the production management apparatus 1. The eye movement EM of each of the workers WK1, WK2, and WK3 is also captured by the eye movement monitoring camera 4. The captured image data is transmitted to the production management apparatus 1.

In step S23, the production management apparatus 1 receives the measurement data and the image data transmitted from the input and measurement devices SS1, SS2, and SS3 through the interface unit 13 as controlled by the sensing data obtaining controller 111. The production management apparatus 1 stores the received data into the sensing data storage 121.

2-2: Estimating Worker's Emotion

When determining that a predetermined time (e.g., one hour) has passed in step S24, the production management apparatus 1 selects one of the workers WK1, WK2, and WK3 in step S25. The feature quantity extraction unit 112 then reads the measurement data and the image data associated with the selected worker from the sensing data storage 121, and extracts the feature quantities from both the measurement data and the image data.

For example, the feature quantity extraction unit 112 extracts the feature quantities of the heart electrical activity $H_i$, the skin potential activity $G_i$, the motion $BM_i$, the activity amount $Ex_i$, and the eye movement $EM_i$, which are correlated with emotional changes, from the measurement data for the heart electrical activity H, the skin potential activity G, the motion BM, and the activity amount Ex and the image data for the eye movement EM. In parallel with this, the feature quantity extraction unit 112 extracts the feature quantities correlated with cognition changes from the motion BM measurement data and the eye movement EM image data. The extracted feature quantities are the same as those extracted in the learning data generation process described above, and will not be described in detail.

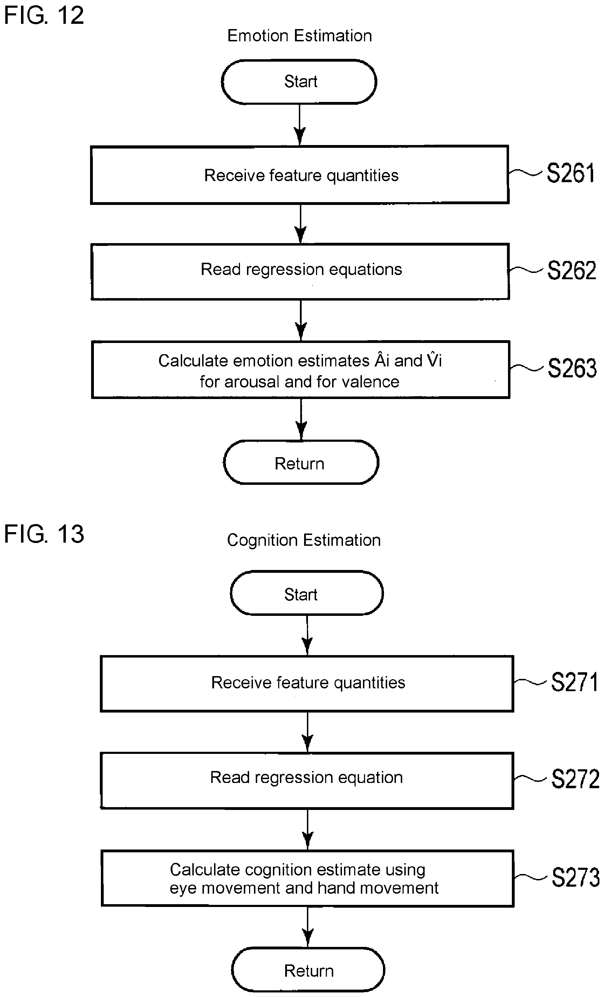

In step S26, the production management apparatus 1 estimates emotional changes in the worker as controlled by the productivity estimation unit 113. Fig. 12 is a flowchart showing the procedure and its details.

In step S261, the productivity estimation unit 113 receives the feature quantities to be used for emotion estimation from the feature quantity extraction unit 112. In step S262, the productivity estimation unit 113 reads, from the learning data storage 122, the regression equations (1) for emotion estimation for arousal and for valence corresponding to the predetermined time period described above. In step S263, the productivity estimation unit 113 calculates the estimates of emotional changes $X\hat{A}_i$ and $X\hat{V}_i$ for the worker in the predetermined time period described above using the feature quantities to be used for the emotion estimation and the regression equations for arousal and for valence.

2-3: Estimating Worker's Cognition

The feature quantity extraction unit 112 included in the production management apparatus 1 extracts the feature quantities correlated with cognition from each of the motion $BM_i$ measurement data and the eye movement $EM_i$ image data obtained during the predetermined time described above.

In step S27, the production management apparatus 1 estimates the cognition of the worker as controlled by the productivity estimation unit 113. Fig. 13 is a flowchart showing the procedure and its detail.

In step S271, the productivity estimation unit 113 receives, from the feature quantity extraction unit 112, the feature quantities of the eye movement $EM_i$ and the motion $BM_i$ to be used for cognition estimation corresponding to the predetermined time period described above. In step S272, the productivity estimation unit 113 reads, from the learning data storage 122, the regression equation (2) for cognition estimation corresponding to the predetermined time period described above. In step S273, the productivity estimation unit 113 calculates the cognition estimate $Y_i$ for the worker using the feature quantities of the eye movement $EM_i$ and the motion $BM_i$ to be used for the cognition estimation and the regression equation for the cognition estimation.

(2-4) Productivity Estimation

In step S28, the production management apparatus 1 estimates the productivity of the worker in the manner described below using the calculated emotional change estimates and the cognition estimates, and the relational expressions (3) and (4) for productivity estimation stored in the learning data storage 122, as controlled by the productivity estimation unit 113.

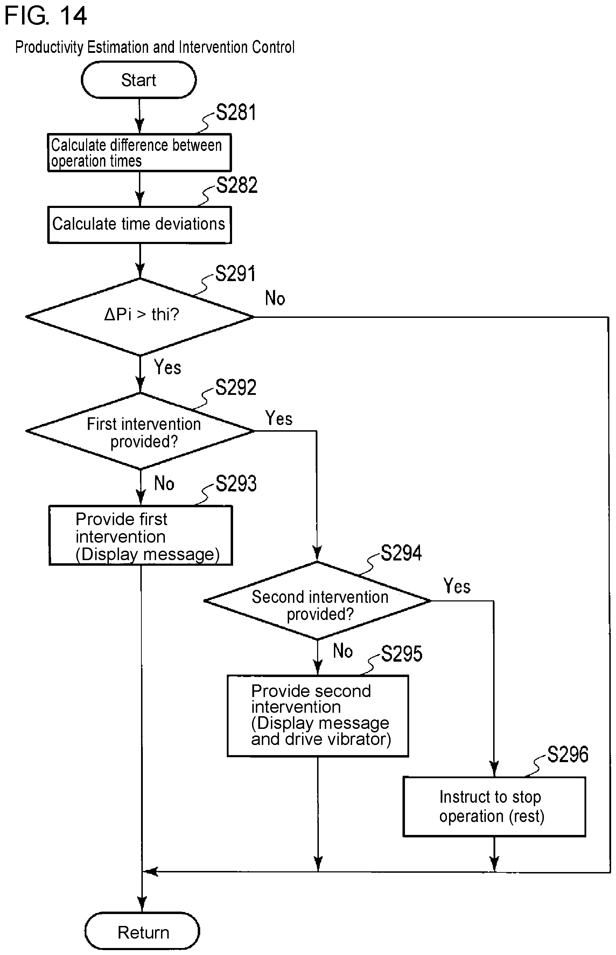

In step S281 shown in Fig. 14, the production management apparatus 1 first calculates the difference between the standard operation time and the actual operation time using the relational expression (3), and outputs the calculated difference in operation time as information indicating the skill level Quality-A of the worker. In step S282, the production management apparatus 1 calculates the deviations of the actual operation time from the average operation time using the relational expression (4), and outputs the calculated values as information indicating the misoperation frequency Quality-B of the worker.

The production management apparatus 1 then adds the calculated skill level Quality-A to the misoperation frequency Quality-B, and uses the resultant value as a worker productivity estimate P. Although the skill level Quality-A may be simply added to the misoperation frequency Quality-B, they may be weighted by their significance in productivity and then may be added to each other.

(3) Controlling Intervention for Worker Based on Worker Productivity Estimates

When obtaining the productivity estimates, the production management apparatus 1 controls, in step S29, interventions for the worker WK1, WK2, or WK3 based on the worker productivity estimates as controlled by the intervention controller 114 in the manner described below by way of non-limiting example.

In step S291 shown in Fig. 14, the intervention controller 114 first calculates the variation $\Delta Pi$ of the productivity estimate calculated in step S28. The variation $\Delta Pi$ is calculated as, for example, a variation from a productivity target value set for each worker or the same productivity target value set for all the workers. The intervention controller 114 then compares the variation $\Delta Pi$ with a threshold thi indicating the productivity permissible level predetermined as a condition for providing an intervention. The threshold thi indicating the permissible level may be a value set for each worker or may be the same value set for all the workers. The index i in the variation $\Delta Pi$ and the threshold thi is an integer representing the number of interventions already provided.

The comparison may show that the productivity estimate has decreased and the variation $\Delta Pi$ exceeds the threshold thi. In this case, the intervention controller 114 determines whether the first intervention has been provided in step S292. When the first intervention has not been provided, the intervention controller 114 determines and performs intervention control in step S293 at the time when the variation $\Delta Pi$ is determined to exceed the threshold thi.

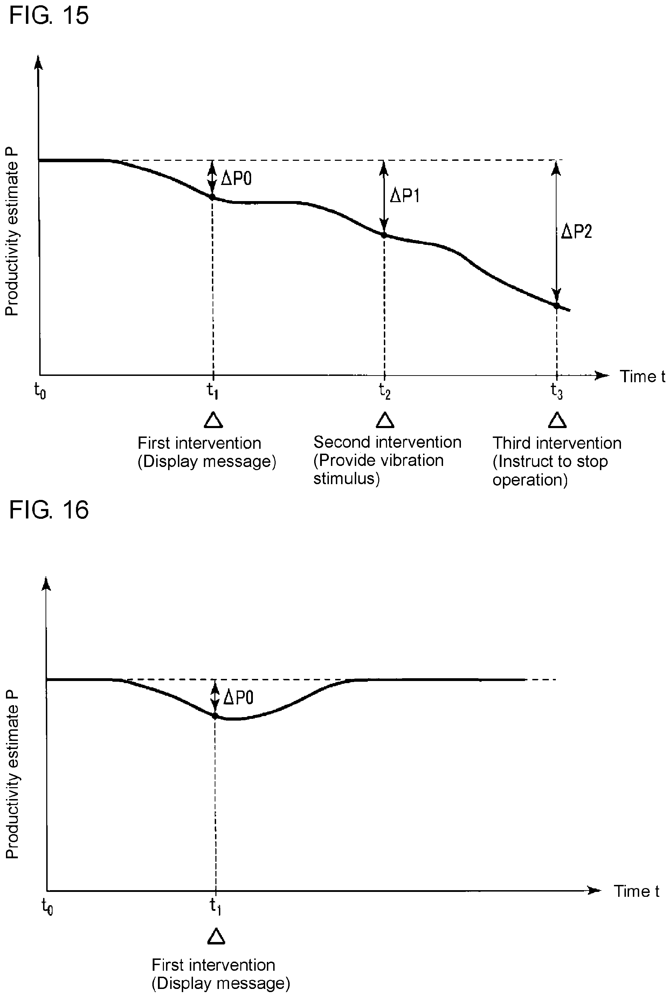

For example, when the productivity estimate P of the worker WK1 decreases during the operation, and the decrease $\Delta P0$ exceeds a first threshold th0 as shown in Fig. 15 or 16, the first intervention is determined to be provided, and is provided at the time t1. In the first intervention, for example, a message intended to improve the motivation of the worker WK1 is generated and displayed on the monitor MO1 arranged in front of the worker WK1. Instead of or in addition to displaying the message, a voice message having the same information may be output from a speaker or headphones (not shown) for the worker WK1.

After the first intervention, the intervention controller 114 continues to compare a variation $\Delta P1$ of the productivity estimate with a second threshold th1. The second threshold th1 used after the first intervention is set at a value larger than the first threshold th0, which is used before the first intervention.

The comparison may show that, for example, the productivity estimate P of the worker WK1 has decreased further as shown in Fig. 15, and the decrease $\Delta P1$ exceeds the second threshold th1. In this case, the intervention controller 114 determines whether the second intervention has been provided in step S294. When the intervention has not been provided, the intervention controller 114 determines and performs the second intervention control in step S295 at the time t2 when the variation $\Delta P1$ is determined to exceed the second threshold th1.

In the second intervention control, for example, a message intended to strongly demand the worker WK1 to recover the production efficiency is generated and displayed on the monitor MO1 arranged in front of the worker WK1. Additionally, the stimulus device AC1 carried by the worker WK1 is driven to vibrate for the worker WK1. Instead of or in addition to displaying the message, a voice message having the same information may be output from a speaker or headphones (not shown) for the worker WK1.

The first intervention may motivate the worker WK1 to recover the productivity as shown in, for example, Fig. 16. In this case, the variation $\Delta P1$ of the productivity estimate P of the worker WK1 does not exceed the second threshold th1, and thus the second intervention is not provided.

After the second intervention, the intervention controller 114 continues to compare a variation $\Delta P2$ of the productivity estimate with a third threshold th2. The third threshold th2 used after the second intervention is set at a value larger than the second threshold th1, which is used before the second intervention.

The comparison may show that, for example, the productivity estimate P of the worker WK1 has decreased further as shown in Fig. 15, and the decrease $\Delta P2$ exceeds the third threshold th2. In this case, the processing immediately proceeds to step S296, in which the intervention controller 114 determines and performs the third intervention control at the time t3 when the variation $\Delta P2$ is determined to exceed the third threshold th2.

For example, recovery of the productivity is determined impossible in this case, and a message for instructing the worker WK1 to stop the operation and rest is generated and displayed on the monitor MO1. Additionally, a message for instructing the leader WR to replace or change the worker is transmitted to and displayed on the portable information terminal TM held by the leader.

When the production management apparatus 1 completes the processing from the emotion estimation to the intervention control for one worker WK1, the production management apparatus 1 determines, in step S30, whether all the workers have been selected for the processing. When any worker remains unselected, the processing returns to step S25, in which the unselected worker is selected, and the processing in steps S25 to S29 is repeated for the next selected worker.

When the processing has been completed for all the workers WK1, WK2, and WK3, the production management apparatus 1 determines whether it has reached the closing time for the production line CS in step S31. At the closing time, the production management apparatus 1 stops the production line CS in step S32.

When the intervention control is performed, the intervention controller 114 generates information indicating the date and time and details of the intervention (e.g. the characteristics describing the intervention as also further above discussed), and stores the information associated with the worker ID into an intervention history storage 123. The information indicating the intervention control history stored in the intervention history storage 123 is, for example, used for the healthcare management and productivity assessment for the workers WK1, WK2, and WK3. The above first, second and subsequent interventions represent non-limiting examples, as in fact other interventions may be used. In particular, in the above examples, two (or more) subsequent (or one following the other over time) interventions are different from each other, regardless of which characteristics or parameters make one intervention different from the following intervention.

Advantageous Effects of Embodiment

As described in detail in the above embodiment, vital sign measurement data and motion measurement data obtained from the workers WK1, WK2, and WK3 during operation are used as primary indicators. The primary indicators and the learning data generated separately are used to estimate the emotion and the cognition of the worker. The estimated emotion and cognition are used as secondary indicators. The secondary indicators and the relational expressions generated separately are used to estimate the productivity of the worker. The variation of the productivity estimate is compared with a threshold that defines the condition for providing an intervention. When the variation of the productivity estimate is determined to exceed the threshold, the intervention is provided for the worker.

The embodiment thus enables an appropriate intervention to be provided for a worker in a timely manner without relying on the experience or the intuition of a manager, and improves and enhances the productivity in a stable manner.

The intervention control is performed a plurality of times, preferably in a stepwise manner while the variation of the worker productivity estimate is monitored. In this manner, gradually stronger interventions are provided in a stepwise manenr. This allows the physical and mental states of a worker to be maintained positive while effectively restoring the productivity.

After the first or second intervention, the worker is instructed to stop the operation at the time when the variation of the worker productivity estimate is determined to exceed a third threshold. This allows, for example, a worker in poor physical condition to rest in a timely manner, and effectively maintains both the worker's health and the product quality.

Emotional changes are expressed as arousal and valence variations and the quadrants of the two-dimensional arousal-valence coordinate system. This allows the emotional changes to be estimated easily and accurately.

The learning data for cognition estimation is generated with correct values (supervisory data) being the feature quantities indicating the success or failure in the operation extracted from the image data obtained by the work monitoring camera CM, and variables being the feature quantities indicating hand movement and the feature quantities indicating eye movement EM. This allows the worker's cognition about the production operation to be estimated more accurately.

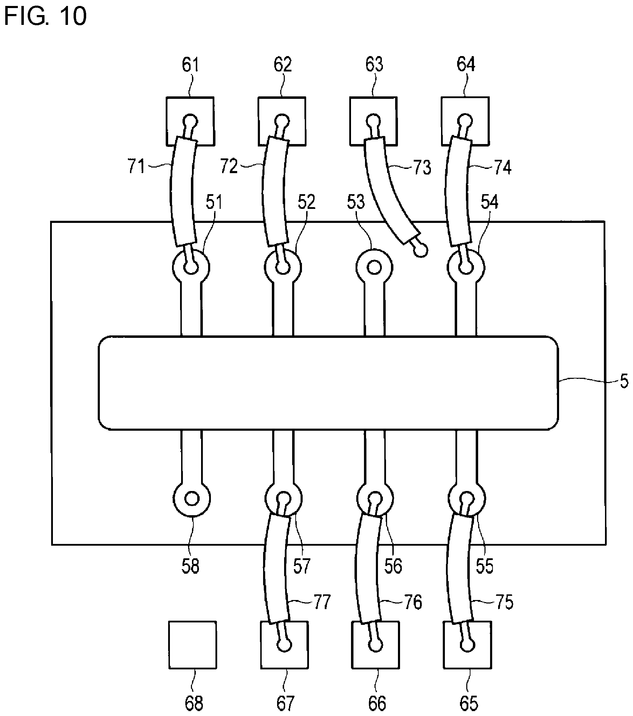

In one example, a worker is currently connecting parts. The image data about the operation results is as shown in Fig. 10. In this example, the operation ends with a terminal 53 and a terminal 63 unsuccessfully connected using a lead 73, and a terminal 58 and a terminal 68 unconnected. In the present embodiment, supervisory data indicating the worker's cognition includes the feature quantities indicating the success or failure in the operation, and variables are primary indicators related to the worker's cognition obtained in parallel within the same time period, or in other words, the feature quantities indicating the hand movement of the worker and the feature quantities indicating the eye movement (EM). The supervisory data and the variables are used to generate a relational expression for estimating the cognition. With the measurement data including the feature quantities indicating hand movement and the feature quantities indicating eye movement, the estimation of the worker's cognition using the relational expressions enables the estimation of the possibility of misoperation by the worker as shown in Fig. 10.

The information indicating the productivity of the worker is defined by the skill level represented by a difference between a standard operation time and an actual operation time, and the misoperation frequency represented by deviations of the actual operation time from an average operation time. The worker productivity is estimated with learning data prepared for both the skill level and the misoperation frequency. This allows the productivity of the worker to be accurately estimated in accordance with the assessment indicator at a production site.

Other Embodiments (e.g. variations of embodiment 1)

In the embodiment described above, the intervention has three stages. However, the intervention may have one, two, or four or more stages. In the embodiment described above, the variation $\Delta$Pi of the productivity estimate is calculated as a variation from a productivity target value set for each worker or the same productivity target value set for all the workers. However, at the second or subsequent interventions, the variation $\Delta$Pi may be calculated as a variation from the productivity estimate at the previous intervention. When the intervention is performed a plurality of times in a stepwise manner, the same intervention may be performed.

The condition for providing an intervention may be determined for each worker in accordance with the worker's baseline productivity. A change in the baseline productivity may be detected based on the estimate of the worker's skill level, and the condition for providing an intervention may be updated in accordance with the detected change. The number or details of interventions may also be determined for each worker in accordance with the worker's baseline productivity.

The relationship between human emotions and vital signs, or the relationship between human emotions and motion information may change depending on the date, the day of the week, the season, the environmental change, and other factors. The learning data to be used for emotion estimation may thus be updated regularly or as appropriate. When the difference calculated between a correct value of an emotion and an estimate of the emotion obtained by the productivity estimation unit 113 exceeds a predetermined range of correct values, the learning data stored in the learning data storage 122 may be updated. In this case, the correct value can be estimated based on the trends in the emotion estimates. In another embodiment, the correct value of the emotion may be input regularly by the subject through the emotion input device 2, and the input value may be used.

Similarly, when the difference calculated between the correct value of cognition and the estimate of the cognition obtained by the productivity estimation unit 113 exceeds a predetermined range of correct values, the learning data stored in the learning data storage 122 may be updated. In this case, the correct value can be estimated based on the trends in the cognition estimates.

The relational expression representing the relationship between the productivity, and the emotion and the cognition may also be modified based on the productivity estimate. In this case as well, the correct value can be estimated based on the trends in the cognition estimates.

In the embodiment described above, the information indicating the emotion of the worker is input into the production management apparatus 1 through the emotion input device 2, which is a smartphone or a tablet terminal. The information may be input in any other manner. For example, the worker may write his or her emotion information on print media such as a questionnaire form, and may use a scanner to read the emotion information and input the information into the production management apparatus 1.

Further, a camera may be used to detect the facial expression of the worker. The information about the detected facial expression may then be input into the production management apparatus 1 as emotion information. A microphone may be used to detect the worker's voice. The detection information may then be input into the production management apparatus 1 as emotion information. Emotion information may be collected from a large number of unspecified individuals by using questionnaires, and the average or other representative values of the collected information may be used as population data to correct the emotion information from an individual. Any other technique may be used to input the information indicating human emotions into the production management apparatus 1.

The above embodiment describes the two-dimensional arousal-valence system for expressing the information about the worker's emotion. Another method may be used to express the worker's emotion information.

In the embodiment described above, the measurement data items, namely, the heart electrical activity H, the skin potential activity G, the eye movement EM, the motion BM, and the activity amount Ex are input into the production management apparatus 1 as information indicating the activity of the worker, and all these items are used to estimate the emotions. However, at least one item of the measurement data may be used to estimate the emotions. For example, the heart electrical activity H is highly contributory to emotions among the other vital signs. The measurement data about the heart electrical activity H, which is highly contributory to emotions among the other vital signs, may be solely used to estimate the emotions. Vital signs other than the items used in the embodiment may also be used.

Additionally, measurement data other than the hand movement and the eye movement may also be used as a primary indicator to estimate the cognition.

In addition, the number of cells in the production line CS and the types of products assembled in each cell may also be modified variously without departing from the scope of the invention.

EMBODIMENT 2

In embodiment 1, a production management apparatus has been presented, which is suitable to determine an intervention to apply to a worker, so that productivity can be increased or maintained at high levels. Present embodiment 2 is directed to a drive assisting apparatus for providing vehicle driving assistance, wherein an intervention is provided, when the driver is driving the vehicle, based on the estimation result of the performance of the driver. The estimation result of the performance of the driver can be obtained as described in embodiment 1, and for instance as represented in figure 4 (wherein, in the case of the present embodiment, the productivity estimation unit 113 is substituted by a driving performance estimation unit 113; the same sensors or devices SS1 to SS3 can be used, when conveniently installed in view of the driver position etc.). The intervention controller 114 of Figure 4 is, according to the present embodiment, configured to provide an intervention relating to driving the vehicle. Thus, the intervention in the present embodiment can be seen as a driving assistance, in that it supports increasing safety and efficiency of driving. As an example, in the present embodiment, correct values used for cognition estimation may be represented by how correctly the driving task is executed, which can be obtained e.g. by measuring certain driving parameters like how correctly the vehicle follows certain predetermined routes (e.g. comparing how smoothly the actual driving route correspond to an ideal route obtained from a navigation system), how smooth the control of the vehicle is (e.g. whether or how often any sudden change of direction occurs), on the degree of the driver recognizing an obstacle, etc. Suitable sensors could be provided (as represented by CM in figure 4), including for instance positioning measurement systems, camera for recognizing driving paths or patterns, vehicle speed sensors, vehicle inertial systems for obtaining information on current driving parameters, etc. The performance values of one driver (in the sense of performance in executing driving, to be used for obtaining learning data by way of regression analysis) can e.g. be obtained by comparing for instance the distance covered over a certain period over an expected distance for a given period, or whether in reaching two points a certain route has been followed compared to predetermined available routes, etc. The intervention controller is configured to determine an intervention to be provided for the subject (e.g. the driver) based on the performance estimated and a predetermined condition for providing an intervention. Preferably, the intervention (as determined by the controller) may include providing the driver of the vehicle with at least a feedback during driving depending on the performance level estimated. For instance, the message may include a message (as an example of the feedback) to the driver suggesting to make a stop and take a rest. Another example of driving assistance (or driving assistance feedback) is represented by a sound, melody, music, or audio message in general; in this way, the driver may be alerted so that the hazardous situation is avoided, and alerted in a way that is appropriate to the estimated performance level. Other types of driving assistance feedback are of course suitable (e.g. a tactile stimulus, or electrical physiological stimulus, etc.), ad in fact the intervention includes any stimulus that can be provided to the driver, and that is deemed suitable for increading the efficiency in driving, which leads also to increased safety. The intervention controller may be installed in the vehicle. However, the determination of the intervention to apply based on the estimated result may be indifferently performed within the vehicle or outside of the vehicle; in the latter case, the determined intervention is communicated to the control unit within the vehicle, which provides the (outside determined) to the driver. Thus, in the present embodiment, thanks to an accurate estimation of the performance, an intervention for the driver can be appropriately determined, so that the driver's performance in driving can be increased, and consequently safety. The intervention can thus be seen in the sense of a driving assistance, since it helps the driver in reaching a better and safer driving. Reference is thenalso made to embodiment 1 (and corresponding figures), illustrating details of devices, methods and of respective features or terms, that are equally and optionally applicable to the present embodiment.

Embodiment 3

Present embodiment 3 is directed to an apparatus for healthcare support of a subject, wherein the device is preferably coupled to the subject. By coupled to the subject it is meant that the device is within range of interaction with the subject, e.g. capable of making measurements on the subject, and/or providing a stimulus (intervention) to the subject, and/or receiving inputs from (e.g. commands) and providing output (e.g. response to the command) to the subject. The healthcare support apparatus includes a controller for providing the subject with an intervention based on an estimated performance of the subject. The estimated performance refers to the performance in executing an operation by the person. Preferably, the operation includes an operation of a device by the person; the operation includes however also a physical or intellectual exercise of the subject. Thus, the operation refers to an action executed by the subject. The estimated performance may be an estimation of the result of the performance (by the subject when executing the operation); the result may be obtained by a performance estimation unit, represented for instance by the second estimation unit illustrated also above. More in particular, the estimation result of the performance of the subject can be obtained as described in embodiment 1, and for instance as represented in figure 4 (wherein, in the case of the present embodiment, the productivity estimation unit 113 is substituted by a performance estimation unit 113; the same sensors or devices SS1 to SS3 can be used, when conveniently installed in view of the subject, and preferably when having regard of one or more types of operation/action executed by the subject). The intervention controller 114 is configured to determine an intervention to be provided for the subject based on the performance estimated by the second estimation unit and a predetermined condition for providing an intervention. In particular, the intervention controller 114 is configured to determine an intervention to be provided to a person in order to improve his/her health conditions or for maintaining good health conditions. As an example, in the present embodiment, correct values for cognition estimation may be obtained by measuring how one or more task (i.e. an operation or action) is executed by the subject: for instance, how straight and balanced the person's body position is when walking, running or sitting (e.g. over predetermined patterns); how smoothly certain movements are made over predetermined patterns; etc. This can be obtained for instance by comparing an image (obtained e.g. via camera CM) with a predetermined pattern, or by making other suitable measurements and comparing the same with predetermined values and/or pattern of values. The performance values of the person (to be used for obtaining learning data by way of regression analysis) can e.g. be obtained by measuring efficiency and/or quality in completing a certain task (i.e. the operation or action above explained) or number of tasks, like for instance measuring the distance covered on foot over an expected distance; measuring the time for accomplishing a task over a predetermined time (e.g. completing a housecleaning or hobby-related operation, number of such operations performed in an hour or day), etc.

The intervention may be represented for instance by one or more messages (in the form of text, audio, and/or video, etc.) suggesting certain activities to undertake or lifestyle to follow, or one or more stimuli signals induced on the subject (for instance, audio/video signal to induce stimulation on the subject, and/or an electric signal inducing stimulation on the subject, etc. ). Other types of intervention are of course suitable. In general, the intervention in the present embodiment can be seen as a healthcare support feedback that leads to improved health conditions or to maintaining good health conditions. Since the performance can be accurately estimated, a (healthcare) intervetnion can be accurately provided for instance when it is really needed (e.g. in correspondence of a predetermined performance value, which can herein be accurately estimated), or chosen in dependence of the estimated performance; for instance, if the performance decreases, a particular intervention can be chosen for prompting an improvement of health conditions; when performance increases, another type of feedback may be given to maintain the same level of performance, and for prompting maintenance of good health conditions also in the long term. In this way, it is possible to improve health conditions of a person, or maintain a good health condition. Reference is thenalso made to embodiment 1 (and corresponding figures), illustrating details of devices, methods and of respective features or terms, that are equally and optionally applicable to the present embodiment.

The present invention is not limited to the embodiment described above, but may be embodied using the components modified without departing from the scope of the invention in its implementation. An appropriate combination of the components described in the embodiment may constitute various aspects of the invention. For example, some of the components described in the embodiment may be eliminated. Further, components from different embodiments may be combined as appropriate. Also, even if certain features have been described only with reference to a device, the same feature can also be described in terms of a method (e.g. according to which the same device operated), of a program (for programming a computer so as to function like the described apparatus features), or of a medium or signal suitable or configured to carry instructions of a program. Similarly, even if a certain feature is described only with reference to a method, the same feature can also be described in terms of a unit or of a device means (or of computer program instructions) configured to perform the same described method feature, or of a program, medium or signal suitable or configured to carry instructions of a program. Still further, in the above and other (see also below) methods herein described, steps are defined like obtaining, estimating, determining, etc. It is however noted that such steps (or any combination of them) may also be caused or induced by a remote device, like for instance by a client computer or a portable terminal, on another device (like for instance a server, localized or distributed) that correspondingly performs the actual step. Thus, the mentioned steps are to be understood also as causing to obtain, causing to estimate, causing to determine, etc., such that any of their combination can be caused or induced by a device remote to the device actually performing the respective step.

REFERENCE SIGNS LIST

CS production line
B1, B2, B3 product
C1, C2, C3 cell
WR leader
WK1, WK2, WK3 worker
MO1, MO2, MO3 monitor
TM portable information terminal
DC part feeder controller
DS part feeder

RB cooperative robot
CM work monitoring camera
NW network
SS1, SS2, SS3 input and measurement device
AC1, AC2, AC3 stimulus device
1 production management apparatus
2 emotion input device
3 measurement device
4 eye movement monitoring camera
11 control unit
111 sensing data obtaining controller
112 feature quantity extraction unit
113 productivity estimation unit
114 intervention controller
115 learning data generation unit
12 storage unit
121 sensing data storage
122 learning data storage
123 intervention history storage
13 interface unit

**Claims**

1. A production management apparatus (1) for managing a production line involving an operation performed by a worker, the apparatus comprising:

    an activity obtaining unit (111) configured to obtain information indicating an activity of the worker during the operation, the information indicating an activity of the worker being information relating to at least one physiological parameter obtained by means of at least one activity sensor (3, 4);
    a first estimation unit (112) configured to estimate emotion and cognition of the worker during the operation based on the information indicating the activity, obtained by the activity obtaining unit (111), used as a primary indicator, and first learning data indicating a relationship between the activity and the emotion of the worker and a relationship between the activity and the cognition of the worker, wherein the first learning data comprises data generated on the basis of information indicating emotion of at least one worker, information indicating cognition of the at least one worker, and information indicating activity of the at least one worker, wherein said information indicating emotion relate to at least one physiological parameter obtained by means of at least one first sensor, said information indicating cognition relate to at least one parameter indicative of cognition and obtained by means of at least one second sensor, and said information indicating activity relate to at least one physiological parameter obtained by means of at least one third sensor, wherein the at least one activity sensor (3, 4) is a sensor that requires smaller information amount, and/or less processing load, and/or less time resolution, and/or constructionally simpler and/or less complex than at least one of the first sensor and the second sensor;
    a second estimation unit (113) configured to estimate productivity of the worker based on the estimated emotion and cognition each used as a secondary indicator, and second learning data indicating a relationship between the productivity, and the emotion and the cognition of the worker; and
    an intervention determination unit (114) configured to determine an intervention to be provided for the worker based on the productivity estimated by the second estimation unit (113) and a predetermined condition for providing an intervention.

2. The production management apparatus (1) according to claim 1, wherein at least two amongst the at least one first sensor, the at least one second sensor and the at least one third sensor are different from each other.

3. The production management apparatus (1) according to claim 1 or 2, wherein, when at least two amongst the at least one first sensor, the at least one second sensor and the at least one third sensor are substantially the same, then said at least two sensors being substantially the same are set according to different respective configurations.

4. The production management apparatus (1) according to any of claims 1 to 3, wherein the activity sensor (3, 4) and the at least one third sensor are substantially the same.

5. The production management apparatus (1) according to any of claims 1 to 4, wherein the second learning data comprises data generated on the basis of information indicating performance, said information indicating emotion of at least one worker, and said information indicating cognition of the at least one worker, wherein information indicating performance indicate performance in correspondence of said information indicating emotion and said information indicating cognition.

6. The production management apparatus (1) according to any of claims 1 to 5, wherein the intervention determination unit (114) is further configured to determine at least one of timing and characteristic of the intervention based at least on the productivity estimated.

7. The production management apparatus (1) according to any of claims 1 to 6, wherein the intervention determination unit (114) is configured to determine a first intervention and a second intervention to be provided to the worker at a first point in time and, respectively, second point in time, wherein the first intervention and the second intervention are different from each other.

8. The production management apparatus (1) according to any of claims 1 to 7, wherein

the intervention determination unit (114) includes
a first determination unit configured to determine that a first intervention is to be provided for the worker at a time when the productivity estimated by the second estimation unit (113) is determined not to meet a first condition; and
a second determination unit configured to determine that a second intervention different from the first intervention is to be provided for the worker at a time when the productivity estimated by the second estimation unit (113) is determined not to meet a second condition after the first intervention is provided, wherein preferably
the first determination unit determines that a visual or auditory stimulus is to be provided for the worker as the first intervention, and
the second determination unit determines that a tactile stimulus is to be provided for the worker as the second intervention.

9. The production management apparatus (1) according to claim 8, wherein

the intervention determination unit (114) further includes
a third determination unit configured to determine that the worker is to be instructed to stop the operation at a time when the productivity estimated by the second estimation unit (113) is determined not to meet a third condition after the first or second intervention is provided.

10. A system comprising a production management apparatus (1) according to any of claims 1 to 9, and at least one article obtained by means of said manufacturing apparatus.

11. A production management method to be implemented by a production management apparatus (1) that manages a production line involving an operation performed by a worker, the method comprising:

obtaining (S23) information indicating an activity of the worker during the operation, the information indicating an activity of the worker being information relating to at least one physiological parameter obtained by means of at least one activity sensor (3, 4),
estimating emotion (S26) and cognition (S27) of the worker during the operation based on the obtained information indicating the activity used as a primary indicator, and first learning data indicating a relationship between the activity, and the emotion of the worker, and a relationship between the activity and the cognition of the worker, wherein the first learning data comprises data generated on the basis of information indicating emotion of at least one worker, information indicating cognition of the at least one worker, and information indicating activity of the at least one worker, wherein said information indicating emotion relate to at least one physiological parameter obtained by means of at least one first sensor, said information indicating cognition relate to at least one parameter indicative of cognition and obtained by means of at least one second sensor, and said information indicating activity relate to at least one physiological parameter obtained by means of at least one third sensor, wherein the at least one activity sensor (3, 4) is a sensor that requires smaller information amount, and/or less processing load, and/or less time resolution, and/or constructionally simpler and/or less complex than at least one of the first sensor and the second sensor;
estimating productivity (S28) of the worker based on the estimated emotion and cognition each used as a secondary indicator, and second learning data indicating a relationship between the productivity, and the emotion and the cognition of the worker; and

determining timing (S29) to intervene for the worker and a detail of the intervention based on the productivity estimated by the second estimation unit (113) and a predetermined condition for providing an intervention.

**12.** A drive assisting apparatus (1) for providing driving assistance, the apparatus comprising:

an activity obtaining unit (111) configured to obtain information indicating an activity of a subject during driving a vehicle, the information indicating an activity of the subject being information relating to at least one physiological parameter obtained by means of at least one activity sensor (3, 4);

a first estimation unit configured to estimate emotion and cognition of the subject during driving based on the information indicating the activity, obtained by the activity obtaining unit, used as a primary indicator, and first learning data indicating a relationship between the activity and the emotion of the subject and a relationship between the activity and the cognition of the subject;

a second estimation unit (113) configured to estimate performance of the subject based on the estimated emotion and cognition each used as a secondary indicator, and second learning data indicating a relationship between performance, and the emotion and the cognition of the subject when driving, wherein the first learning data comprises data generated on the basis of information indicating emotion of at least one subject, information indicating cognition of the at least one subject, and information indicating activity of the at least one subject, wherein said information indicating emotion relate to at least one physiological parameter obtained by means of at least one first sensor, said information indicating cognition relate to at least one parameter indicative of cognition and obtained by means of at least one second sensor, and said information indicating activity relate to at least one physiological parameter obtained by means of at least one third sensor, wherein the at least one activity sensor (3, 4) is a sensor that requires smaller information amount, and/or less processing load, and/or less time resolution, and/or constructionally simpler and/or less complex than at least one of the first sensor and the second sensor; and

an intervention determination unit (114) configured to determine an intervention to be provided for the subject based on the performance estimated by the second estimation unit (113) and a predetermined condition for providing an intervention.

**13.** A drive assisting method for providing driving assistance, the method comprising steps of:

obtaining information indicating an activity of a subject during driving a vehicle, the information indicating an activity of the subject being information relating to at least one physiological parameter obtained by means of at least one activity sensor (3, 4);

estimating emotion and cognition of the subject during driving based on the obtained information indicating the activity used as a primary indicator, and first learning data indicating a relationship between the activity and the emotion of the subject and a relationship between the activity and the cognition of the subject, wherein the first learning data comprises data generated on the basis of information indicating emotion of at least one subject, information indicating cognition of the at least one subject, and information indicating activity of the at least one subject, wherein said information indicating emotion relate to at least one physiological parameter obtained by means of at least one first sensor, said information indicating cognition relate to at least one parameter indicative of cognition and obtained by means of at least one second sensor, and said information indicating activity relate to at least one physiological parameter obtained by means of at least one third sensor, wherein the at least one activity sensor (3, 4) is a sensor that requires smaller information amount, and/or less processing load, and/or less time resolution, and/or constructionally simpler and/or less complex than at least one of the first sensor and the second sensor;

estimating performance of the subject based on the estimated emotion and cognition each used as a secondary indicator, and second learning data indicating a relationship between performance, and the emotion and the cognition of the subject when driving; and

determining an intervention to be provided for the subject based on the performance estimated by the second estimation unit (113) and a predetermined condition for providing an intervention.

**14.** An apparatus for healthcare support (1) of a subject, the apparatus comprising:

an activity obtaining unit (111) configured to obtain information indicating an activity of a subject when executing an operation, the information indicating an activity of the subject being information relating to at least one physiological parameter obtained by means of at least one activity sensor (3, 4);

a first estimation unit configured to estimate emotion and cognition of the subject during executing the operation based on the information indicating the activity, obtained by the activity obtaining unit, used as a primary indicator, and first learning data indicating a relationship between the activity and the emotion of the subject and a relationship between the activity and the cognition of the subject;

a second estimation unit (113) configured to estimate performance of the subject based on the estimated emotion and cognition each used as a secondary indicator, and second learning data indicating a relationship between performance, and the emotion and the cognition of the subject when driving, wherein the first learning data comprises data generated on the basis of information indicating emotion of at least one subject, information indicating cognition of the at least one subject, and information indicating activity of the at least one subject, wherein said information indicating emotion relate to at least one physiological parameter obtained by means of at least one first sensor, said information indicating cognition relate to at least one parameter indicative of cognition and obtained by means of at least one second sensor, and said information indicating activity relate to at least one physiological parameter obtained by means of at least one third sensor, wherein the at least one activity sensor (3, 4) is a sensor that requires smaller information amount, and/or less processing load, and/or less time resolution, and/or constructionally simpler and/or less complex than at least one of the first sensor and the second sensor; and

an intervention determination unit (114) configured to determine an intervention to be provided for the subject based on the performance estimated by the second estimation unit (113) and a predetermined condition for providing an intervention, wherein executing an operation preferably includes at least one amongst executing an interacting operation with a machine and performing a physical exercise.

15. A method for healthcare support of a subject, the method comprising steps of:

obtaining information indicating an activity of a subject when executing an operation, the information indicating an activity of the subject being information relating to at least one physiological parameter obtained by means of at least one activity sensor (3, 4);

estimating emotion and cognition of the subject during executing the operation based on the obtained information indicating the activity used as a primary indicator, and first learning data indicating a relationship between the activity and the emotion of the subject and a relationship between the activity and the cognition of the subject, wherein the first learning data comprises data generated on the basis of information indicating emotion of at least one subject, information indicating cognition of the at least one subject, and information indicating activity of the at least one subject, wherein said information indicating emotion relate to at least one physiological parameter obtained by means of at least one first sensor, said information indicating cognition relate to at least one parameter indicative of cognition and obtained by means of at least one second sensor, and said information indicating activity relate to at least one physiological parameter obtained by means of at least one third sensor, wherein the at least one activity sensor (3, 4) is a sensor that requires smaller information amount, and/or less processing load, and/or less time resolution, and/or constructionally simpler and/or less complex than at least one of the first sensor and the second sensor;

estimating performance of the subject based on the estimated emotion and cognition each used as a secondary indicator, and second learning data indicating a relationship between performance, and the emotion and the cognition of the subject when driving; and

determining an intervention to be provided for the subject based on the performance estimated by the second estimation unit (113) and a predetermined condition for providing an intervention.

## Patentansprüche

1. Produktionsverwaltungsvorrichtung (1) zur Verwaltung einer Produktionslinie, die einen Vorgang beinhaltet, der durch einen Arbeiter durchgeführt wird, wobei die Vorrichtung umfasst:

eine Aktivitätserlangungseinheit (111), die konfiguriert ist, um Informationen zu erlangen, die eine Aktivität des Arbeiters während des Vorgangs angeben, wobei die Informationen, die eine Aktivität des Arbeiters angeben, Informationen sind, die sich auf mindestens einen physiologischen Parameter beziehen, der mittels mindestens eines Aktivitätssensors (3, 4) erlangt wird;

eine erste Schätzeinheit (112), die konfiguriert ist, um basierend auf den durch die Aktivitätserlangungseinheit (111) erlangten Informationen, die die Aktivität angeben, die als ein primärer Indikator verwendet wird, Emotion und Kognition des Arbeiters während des Vorgangs und erste Lerndaten, die eine Beziehung zwischen der Aktivität und der Emotion des Arbeiters und eine Beziehung zwischen der Aktivität und der Kognition des Arbeiters angeben, zu schätzen, wobei die ersten Lerndaten Daten umfassen, die basierend auf Informationen, die Emotion von mindestens einem Arbeiter angeben, Informationen, die Kognition des mindestens einen Arbeiters angeben, und Informationen, die Aktivität des mindestens einen Arbeiters angeben, erzeugt werden, wobei sich die Informationen, die Emotion angeben, auf mindestens einen physiologischen Parameter beziehen, der mittels mindestens eines ersten Sensors erlangt wird, wobei sich die Informationen, die Kognition angeben, auf mindestens einen Parameter beziehen, der Kognition angibt und mittels mindestens eines zweiten Sensors erlangt wird, und wobei sich die Informationen, die Aktivität angeben, auf mindestens einen physiologischen Parameter beziehen, der mittels mindestens eines dritten Sensors erlangt wird, wobei der mindestens eine Aktivitätssensor (3, 4) ein Sensor ist, der eine kleinere Informationsmenge und/oder eine geringere Verarbeitungslast und/oder eine geringere Zeitauflösung erfordert und/oder konstruktionsmäßig einfacher und/oder weniger komplex ist als mindestens einer von dem ersten Sensor und dem zweiten Sensor;
eine zweite Schätzeinheit (113), die konfiguriert ist, um basierend auf der geschätzten Emotion und Kognition, die jeweils als ein sekundärer Indikator verwendet werden, die Produktivität des Arbeiters und zweite Lerndaten, die eine Beziehung zwischen der Produktivität und der Emotion und der Kognition des Arbeiters angeben, zu schätzen; und
eine Interventionsbestimmungseinheit (114), die konfiguriert ist, um basierend auf der durch die zweite Schätzeinheit (113) geschätzten Produktivität und einer vorbestimmten Bedingung zur Bereitstellung einer Intervention eine für den Arbeiter bereitzustellende Intervention zu bestimmen.

2. Produktionsverwaltungsvorrichtung (1) nach Anspruch 1, wobei sich mindestens zwei unter dem mindestens einen ersten Sensor, dem mindestens einen zweiten Sensor und dem mindestens einen dritten Sensor voneinander unterscheiden.

3. Produktionsverwaltungsvorrichtung (1) nach Anspruch 1 oder 2, wobei dann, wenn mindestens zwei unter dem mindestens einen ersten Sensor, dem mindestens einen zweiten Sensor und dem mindestens einen dritten Sensor im Wesentlichen gleich sind, die mindestens zwei im Wesentlichen gleichen Sensoren entsprechend unterschiedlichen jeweiligen Konfigurationen eingestellt werden.

4. Produktionsverwaltungsvorrichtung (1) nach einem der Ansprüche 1 bis 3, wobei der Aktivitätssensor (3, 4) und der mindestens eine dritte Sensor im Wesentlichen gleich sind.

5. Produktionsverwaltungsvorrichtung (1) nach einem der Ansprüche 1 bis 4, wobei die zweiten Lerndaten Daten umfassen, die basierend auf Informationen erzeugt werden, die Leistung angeben, wobei die Informationen Emotion mindestens eines Arbeiters angeben, und wobei die Informationen Kognition des mindestens einen Arbeiters angeben, wobei die Informationen, die Leistung angeben, die Leistung in Übereinstimmung mit den Informationen, die Emotionen angeben, und den Informationen, die Kognition angeben, angeben.

6. Produktionsverwaltungsvorrichtung (1) nach einem der Ansprüche 1 bis 5, wobei die Interventionsbestimmungseinheit (114) weiter konfiguriert ist, um mindestens basierend auf der geschätzten Produktivität mindestens eines von einer Zeitsteuerung und einer Eigenschaft der Intervention zu bestimmen.

7. Produktionsverwaltungsvorrichtung (1) nach einem der Ansprüche 1 bis 6, wobei die Interventionsbestimmungseinheit (114) konfiguriert ist, um eine erste Intervention und eine zweite Intervention zu bestimmen, die dem Arbeiter zu einem ersten bzw. zweiten Zeitpunkt bereitgestellt werden sollen, wobei sich die erste Intervention und die zweite Intervention voneinander unterscheiden.

8. Produktionsverwaltungsvorrichtung (1) nach einem der Ansprüche 1 bis 7, wobei

die Interventionsbestimmungseinheit (114)
eine erste Bestimmungseinheit, die konfiguriert ist, um zu bestimmen, dass eine erste Intervention für den Arbeiter zu einem Zeitpunkt bereitgestellt werden soll, wenn bestimmt wird, dass die durch die zweite Schätzeinheit (113) geschätzte Produktivität eine erste Bedingung nicht erfüllt; und
eine zweite Bestimmungseinheit, die konfiguriert ist, um zu bestimmen, dass eine zweite Intervention, die sich von der ersten Intervention unterscheidet, für den Arbeiter zu einem Zeitpunkt bereitgestellt werden soll, wenn bestimmt wird, dass die durch die zweite Schätzeinheit (113) geschätzte Produktivität eine zweite Bedingung nicht erfüllt, nachdem die erste Intervention bereitgestellt ist, einschließt, wobei vorzugsweise
die erste Bestimmungseinheit bestimmt, dass ein visueller oder akustischer Reiz für den Arbeiter als die erste Intervention bereitgestellt werden soll, und
die zweite Bestimmungseinheit bestimmt, dass ein taktiler Reiz für den Arbeiter als die zweite Intervention bereitgestellt werden soll.

9. Produktionsverwaltungsvorrichtung (1) nach Anspruch 8, wobei

die Interventionsbestimmungseinheit (114) weiter Folgendes einschließt
eine dritte Bestimmungseinheit, die konfiguriert ist, um zu bestimmen, dass der Arbeiter angewiesen werden soll, den Vorgang zu einem Zeitpunkt zu stoppen, wenn bestimmt wird, dass die durch die zweite Schätzeinheit (113) geschätzte Produktivität eine dritte Bedingung nicht erfüllt, nachdem die erste oder die zweite Intervention bereitgestellt ist.

10. System, umfassend eine Produktionsverwaltungsvorrichtung (1) nach einem der Ansprüche 1 bis 9, und mindestens einen Artikel, der mittels der Herstellungsvorrichtung erlangt wird.

11. Produktionsverwaltungsverfahren, das durch eine Produktionsverwaltungsvorrichtung (1) implementiert werden soll, die eine Produktionslinie verwaltet, die einen Vorgang beinhaltet, der durch einen Arbeiter durchgeführt werden soll, wobei das Verfahren umfasst:

Erlangen (S23) von Informationen, die eine Aktivität des Arbeiters während des Vorgangs angeben, wobei die Informationen, die eine Aktivität des Arbeiters angeben, Informationen sind, die sich auf mindestens einen physiologischen Parameter beziehen, der mittels mindestens eines Aktivitätssensors (3, 4) erlangt wird,
Schätzen von Emotion (S26) und Kognition (S27) des Arbeiters während des Vorgangs basierend auf den erlangten Informationen, die die Aktivität angeben, die als ein primärer Indikator verwendet wird, und von ersten Lerndaten, die eine Beziehung zwischen der Aktivität und der Emotion des Arbeiters und eine Beziehung zwischen der Aktivität und der Kognition des Arbeiters angeben, wobei die ersten Lerndaten Daten umfassen, die basierend auf Informationen, die Emotion von mindestens einem Arbeiter angeben, Informationen, die Kognition des mindestens einen Arbeiters angeben, und Informationen, die Aktivität des mindestens einen Arbeiters angeben, erzeugt werden, wobei sich die Informationen, die Emotion angeben, auf mindestens einen physiologischen Parameter beziehen, der mittels mindestens eines ersten Sensors erlangt wird, wobei sich die Informationen, die Kognition angeben, auf mindestens einen Parameter beziehen, der Kognition angibt und mittels mindestens eines zweiten Sensors erlangt wird, und wobei sich die Informationen, die Aktivität angeben, auf mindestens einen physiologischen Parameter beziehen, der mittels mindestens eines dritten Sensors erlangt wird, wobei der mindestens eine Aktivitätssensor (3, 4) ein Sensor ist, der eine kleinere Informationsmenge und/oder eine geringere Verarbeitungslast und/oder eine geringere Zeitauflösung erfordert und/oder konstruktionsmäßig einfacher und/oder weniger komplex ist als mindestens einer von dem ersten Sensor und dem zweiten Sensor;
Schätzen von Produktivität (S28) des Arbeiters basierend auf der geschätzten Emotion und Kognition, die jeweils als ein sekundärer Indikator verwendet werden, und von zweiten Lerndaten, die eine Beziehung zwischen der Produktivität und der Emotion und der Kognition des Arbeiters angeben; und
Bestimmen einer Zeitsteuerung (S29) zur Intervention für den Arbeiter und eines Details der Intervention basierend auf der durch die zweite Schätzeinheit (113) geschätzten Produktivität und einer vorbestimmten Bedingung zur Bereitstellung einer Intervention.

12. Fahrassistenzvorrichtung (1) zur Bereitstellung von Fahrassistenz, wobei die Vorrichtung umfasst:

eine Aktivitätserlangungseinheit (111), die konfiguriert ist, um Informationen zu erlangen, die eine Aktivität eines Subjekts während des Fahrens eines Fahrzeugs angeben, wobei die Informationen, die eine Aktivität des Subjekts angeben, Informationen sind, die sich auf mindestens einen physiologischen Parameter beziehen, der mittels mindestens eines Aktivitätssensors (3, 4) erlangt wird;
eine erste Schätzeinheit, die konfiguriert ist, um basierend auf den durch die Aktivitätserlangungseinheit erlangten Informationen, die die Aktivität angeben, die als ein primärer Indikator verwendet wird, Emotion und Kognition des Subjekts während des Fahrens und erste Lerndaten, die eine Beziehung zwischen der Aktivität und der Emotion des Subjekts und eine Beziehung zwischen der Aktivität und der Kognition des Subjekts angeben, zu schätzen,
eine zweite Schätzeinheit (113), die konfiguriert ist, um basierend auf der geschätzten Emotion und Kognition, die jeweils als ein sekundärer Indikator verwendet werden, die Leistung des Subjekts und zweite Lerndaten, die eine Beziehung zwischen der Leistung und der Emotion und der Kognition des Subjekts beim Fahren angeben, zu schätzen; wobei die ersten Lerndaten Daten umfassen, die basierend auf Informationen, die Emotion von mindestens einem Subjekt angeben, Informationen, die Kognition des mindestens einen Subjekts angeben, und Informationen, die Aktivität des mindestens einen Subjekts angeben, erzeugt werden, wobei sich die Informationen, die Emotion angeben, auf mindestens einen physiologischen Parameter beziehen, der mittels mindestens eines ersten Sensors erlangt wird, wobei sich die Informationen, die Kognition angeben, auf mindestens einen Parameter beziehen, der Kognition angibt und mittels mindestens eines zweiten Sensors erlangt wird, und wobei sich die Informationen, die Aktivität angeben, auf mindestens einen physiologischen Parameter beziehen, der mittels mindestens eines dritten Sensors erlangt wird, wobei der mindestens eine Aktivitätssensor (3, 4) ein Sensor ist, der eine kleinere Informationsmenge und/oder eine geringere Verarbeitungslast und/oder eine geringere Zeitauflösung erfordert und/oder konstruktionsmäßig einfacher und/oder weniger komplex ist als mindestens einer von dem ersten Sensor und dem zweiten Sensor ; und

eine Interventionsbestimmungseinheit (114), die konfiguriert ist, um basierend auf der durch die zweite Schätzeinheit (113) geschätzten Leistung und einer vorbestimmten Bedingung zur Bereitstellung einer Intervention eine für das Subjekt bereitzustellende Intervention zu bestimmen.

**13.** Fahrassistenzverfahren zur Bereitstellung von Fahrassistenz, das Verfahren umfassend die Schritte von:

Erlangen von Informationen, die eine Aktivität eines Subjekts während des Fahrens eines Fahrzeugs angeben, wobei die Informationen, die eine Aktivität des Subjekts angeben, Informationen sind, die sich auf mindestens einen physiologischen Parameter beziehen, der mittels mindestens eines Aktivitätssensors (3, 4) erlangt wird;
Schätzen von Emotion und Kognition des Subjekts während des Fahrens basierend auf den erlangten Informationen, die die Aktivität angeben, die als ein primärer Indikator verwendet wird, und von ersten Lerndaten, die eine Beziehung zwischen der Aktivität und der Emotion des Subjekts und eine Beziehung zwischen der Aktivität und der Kognition des Subjekts angeben, wobei die ersten Lerndaten Daten umfassen, die basierend auf Informationen, die Emotion von mindestens einem Subjekt angeben, Informationen, die Kognition des mindestens einen Subjekts angeben, und Informationen, die Aktivität des mindestens einen Subjekts angeben, erzeugt werden, wobei sich die Informationen, die Emotion angeben, auf mindestens einen physiologischen Parameter beziehen, der mittels mindestens eines ersten Sensors erlangt wird, wobei sich die Informationen, die Kognition angeben, auf mindestens einen Parameter beziehen, der Kognition angibt und mittels mindestens eines zweiten Sensors erlangt wird, und wobei sich die Informationen, die Aktivität angeben, auf mindestens einen physiologischen Parameter beziehen, der mittels mindestens eines dritten Sensors erlangt wird, wobei der mindestens eine Aktivitätssensor (3, 4) ein Sensor ist, der eine kleinere Informationsmenge und/oder eine geringere Verarbeitungslast und/oder eine geringere Zeitauflösung erfordert und/oder konstruktionsmäßig einfacher und/oder weniger komplex ist als mindestens einer von dem ersten Sensor und dem zweiten Sensor;
Schätzen von Leistung des Subjekts basierend auf der geschätzten Emotion und Kognition, die jeweils als ein sekundärer Indikator verwendet werden, und von zweiten Lerndaten, die eine Beziehung zwischen Leistung und der Emotion und der Kognition des Subjekts beim Fahren angeben; und
Bestimmen einer Intervention, die für das Subjekt bereitgestellt werden soll, basierend auf der durch die zweite Schätzeinheit (113) geschätzten Leistung und einer vorbestimmten Bedingung zur Bereitstellung einer Intervention.

**14.** Vorrichtung zur Unterstützung der medizinischen Versorgung (1) eines Subjekts, wobei die Vorrichtung umfasst:

eine Aktivitätserlangungseinheit (111), die konfiguriert ist, um Informationen zu erlangen, die eine Aktivität eines Subjekts bei der Ausführung eines Vorgangs angeben, wobei die Informationen, die eine Aktivität des Subjekts angeben, Informationen sind, die sich auf mindestens einen physiologischen Parameter beziehen, der mittels mindestens eines Aktivitätssensors (3, 4) erlangt wird;
eine erste Schätzeinheit, die konfiguriert ist, um basierend auf den durch die Aktivitätserlangungseinheit erlangten Informationen, die die Aktivität angeben, die als ein primärer Indikator verwendet wird, Emotion und Kognition des Subjekts bei der Ausführung eines Vorgangs und erste Lerndaten, die eine Beziehung zwischen der Aktivität und der Emotion des Subjekts und eine Beziehung zwischen der Aktivität und der Kognition des Subjekts angeben, zu schätzen;
eine zweite Schätzeinheit (113), die konfiguriert ist, um basierend auf der geschätzten Emotion und Kognition, die jeweils als ein sekundärer Indikator verwendet werden, die Leistung des Subjekts und zweite Lerndaten, die eine Beziehung zwischen der Leistung und der Emotion und der Kognition des Subjekts beim Fahren angeben, zu schätzen; wobei die ersten Lerndaten Daten umfassen, die basierend auf Informationen, die Emotion von mindestens einem Subjekt angeben, Informationen, die Kognition des mindestens einen Subjekts angeben, und Informationen, die Aktivität des mindestens einen Subjekts angeben, erzeugt werden, wobei sich die Informationen, die Emotion angeben, auf mindestens einen physiologischen Parameter beziehen, der mittels mindestens eines ersten Sensors erlangt wird, wobei sich die Informationen, die Kognition angeben, auf mindestens einen Parameter beziehen, der Kognition angibt und mittels mindestens eines zweiten Sensors erlangt wird, und wobei sich die Informationen, die Aktivität angeben, auf mindestens einen physiologischen Parameter beziehen, der mittels mindestens eines dritten Sensors erlangt wird, wobei der mindestens eine Aktivitätssensor (3, 4) ein Sensor ist, der eine kleinere Informationsmenge und/oder eine geringere Verarbeitungslast und/oder eine geringere Zeitauflösung erfordert und/oder konstruktionsmäßig einfacher und/oder weniger komplex ist als mindestens einer von dem ersten Sensor und dem zweiten Sensor; und

eine Interventionsbestimmungseinheit (114), die konfiguriert ist, um basierend auf der durch die zweite Schätzeinheit (113) geschätzten Leistung und einer vorbestimmten Bedingung zur Bereitstellung einer Intervention eine für das Subjekt bereitzustellende Intervention zu bestimmen, wobei das Ausführen eines Vorgangs vorzugsweise mindestens eines unter Ausführen eines Wechselwirkungsvorgangs mit einer Maschine und Durchführen einer körperlichen Betätigung einschließt.

**15.** Verfahren zur Unterstützung der medizinischen Versorgung eines Subjekts, das Verfahren umfassend die Schritte von:

Erlangen von Informationen, die eine Aktivität eines Subjekts bei der Ausführung eines Vorgangs angeben, wobei die Informationen, die eine Aktivität des Subjekts angeben, Informationen sind, die sich auf mindestens einen physiologischen Parameter beziehen, der mittels mindestens eines Aktivitätssensors (3, 4) erlangt wird;

Schätzen von Emotion und Kognition des Subjekts bei der Ausführung eines Vorgangs basierend auf den erlangten Informationen, die die Aktivität angeben, die als ein primärer Indikator verwendet wird, und von ersten Lerndaten, die eine Beziehung zwischen der Aktivität und der Emotion des Subjekts und eine Beziehung zwischen der Aktivität und der Kognition des Subjekts angeben, wobei die ersten Lerndaten Daten umfassen, die basierend auf Informationen, die Emotion von mindestens einem Subjekt angeben, Informationen, die Kognition des mindestens einen Subjekts angeben, und Informationen, die Aktivität des mindestens einen Subjekts angeben, erzeugt werden, wobei sich die Informationen, die Emotion angeben, auf mindestens einen physiologischen Parameter beziehen, der mittels mindestens eines ersten Sensors erlangt wird, wobei sich die Informationen, die Kognition angeben, auf mindestens einen Parameter beziehen, der Kognition angibt und mittels mindestens eines zweiten Sensors erlangt wird, und wobei sich die Informationen, die Aktivität angeben, auf mindestens einen physiologischen Parameter beziehen, der mittels mindestens eines dritten Sensors erlangt wird, wobei der mindestens eine Aktivitätssensor (3, 4) ein Sensor ist, der eine kleinere Informationsmenge und/oder eine geringere Verarbeitungslast und/oder eine geringere Zeitauflösung erfordert und/oder konstruktionsmäßig einfacher und/oder weniger komplex ist als mindestens einer von dem ersten Sensor und dem zweiten Sensor;

Schätzen von Leistung des Subjekts basierend auf der geschätzten Emotion und Kognition, die jeweils als ein sekundärer Indikator verwendet werden, und von zweiten Lerndaten, die eine Beziehung zwischen Leistung und der Emotion und der Kognition des Subjekts beim Fahren angeben; und

Bestimmen einer Intervention, die für das Subjekt bereitgestellt werden soll, basierend auf der durch die zweite Schätzeinheit (113) geschätzten Leistung und einer vorbestimmten Bedingung zur Bereitstellung einer Intervention.

## Revendications

**1.** Appareil (1) de gestion de production pour gérer une ligne de production impliquant une opération mise en œuvre par un travailleur, l'appareil comprenant :

une unité (111) d'obtention d'activité configurée pour obtenir des informations indiquant une activité du travailleur pendant l'opération, les informations indiquant une activité du travailleur étant des informations se rapportant à au moins un paramètre physiologique obtenu au moyen d'au moins un capteur (3, 4) d'activité ;

une première unité (112) d'estimation configurée pour estimer l'émotion et la cognition du travailleur durant l'opération sur la base des informations indiquant l'activité, obtenue par l'unité (111) d'obtention d'activité, utilisées comme indicateur primaire, et de premières données d'apprentissage indiquant une relation entre l'activité et l'émotion du travailleur et une relation entre l'activité et la cognition du travailleur, dans lequel les premières données d'apprentissage comprennent des données générées sur la base d'informations indiquant l'émotion d'au moins un travailleur, d'informations indiquant la cognition du au moins un travailleur, et d'informations indiquant l'activité du au moins un travailleur, dans lequel lesdites informations indiquant l'émotion se rapportent à au moins un paramètre physiologique obtenu au moyen d'au moins un premier capteur, lesdites informations indiquant la cognition se rapportent à au moins un paramètre indicatif de la cognition et obtenu au moyen d'au moins un deuxième capteur, et lesdites informations indiquant l'activité se rapportent à au moins un paramètre physiologique obtenu au moyen d'au moins un troisième capteur, dans lequel le au moins un capteur (3, 4) d'activité est un capteur qui nécessite une plus petite quantité d'informations, et/ou une moindre charge de traitement, et/ou une moindre résolution temporelle, et/ou est de construction plus simple et/ou moins complexe qu'au moins un du premier capteur et du deuxième capteur ;

une deuxième unité (113) d'estimation configurée pour estimer la productivité du travailleur sur la base de l'émotion et de la cognition estimées utilisées chacune comme indicateur secondaire, et de deuxièmes données d'apprentissage indiquant une relation entre la productivité, et l'émotion et la cognition du travailleur ; et

une unité (114) de détermination d'intervention configurée pour déterminer une intervention à fournir au travailleur sur la base de la productivité estimée par la deuxième unité (113) d'estimation et d'une condition prédéterminée pour fournir une intervention.

2. Appareil (1) de gestion de production selon la revendication 1, dans lequel au moins deux parmi le au moins un premier capteur, le au moins un deuxième capteur et le au moins un troisième capteur sont différents l'un de l'autre.

3. Appareil (1) de gestion de production selon la revendication 1 ou 2, dans lequel, lorsqu'au moins deux parmi le au moins un premier capteur, le au moins un deuxième capteur et le au moins un troisième capteur sont sensiblement les mêmes, alors lesdits au moins deux capteurs qui sont sensiblement les mêmes sont réglés selon des configurations respectives différentes.

4. Appareil (1) de gestion de production selon l'une quelconque des revendications 1 à 3, dans lequel le capteur (3, 4) d'activité et le au moins un troisième capteur sont sensiblement les mêmes.

5. Appareil (1) de gestion de production selon l'une quelconque des revendications 1 à 4, dans lequel les deuxièmes données d'apprentissage comprennent des données générées sur la base d'informations indiquant la performance, desdites informations indiquant l'émotion d'au moins un travailleur, et desdites informations indiquant la cognition du au moins un travailleur, dans lequel les informations indiquant la performance indiquent la performance en correspondance desdites informations indiquant l'émotion et desdites informations indiquant la cognition.

6. Appareil (1) de gestion de production selon l'une quelconque des revendications 1 à 5, dans lequel l'unité (114) de détermination d'intervention est en outre configurée pour déterminer au moins parmi un moment et une caractéristique de l'intervention sur la base d'au moins la productivité estimée.

7. Appareil (1) de gestion de production selon l'une quelconque des revendications 1 à 6, dans lequel l'unité (114) de détermination d'intervention est configurée pour déterminer une première intervention et une deuxième intervention à fournir au travailleur à un premier point dans le temps et, respectivement, un deuxième point dans le temps, dans lequel la première intervention et la deuxième intervention sont différentes l'une de l'autre.

8. Appareil (1) de gestion de production selon l'une quelconque des revendications 1 à 7, dans lequel

l'unité (114) de détermination d'intervention inclut
une première unité de détermination configurée pour déterminer qu'une première intervention doit être fournie pour le travailleur à un moment où la productivité estimée par la deuxième unité (113) d'estimation est déterminée comme ne répondant pas à une première condition ; et
une deuxième unité de détermination configurée pour déterminer qu'une deuxième intervention différente de la première intervention doit être fournie pour le travailleur à un moment où la productivité estimée par la deuxième unité (113) d'estimation est déterminée comme ne répondant pas à une deuxième condition après la fourniture de la première intervention, dans lequel préférablement la première unité de détermination détermine qu'un stimulus visuel ou auditif doit être fourni pour le travailleur en tant que première intervention, et
la deuxième unité de détermination détermine qu'un stimulus tactile doit être fourni pour le travailleur en tant que deuxième intervention.

9. Appareil (1) de gestion de production selon la revendication 8, dans lequel

l'unité (114) de détermination d'intervention inclut en outre
une troisième unité de détermination configurée pour déterminer que le travailleur doit recevoir l'ordre d'arrêter l'opération à un moment où la productivité estimée par la deuxième unité (113) d'estimation est déterminée comme ne répondant pas à une troisième condition après la fourniture de la première ou de la deuxième

**EP 3 589 206 B1**

intervention.

10. Système comprenant un appareil (1) de gestion de production selon l'une quelconque des revendications 1 à 9, et au moins un article obtenu au moyen dudit appareil de fabrication.

11. Procédé de gestion de production à mettre en œuvre par un appareil (1) de gestion de production qui gère une ligne de production impliquant une opération mise en œuvre par un travailleur, le procédé comprenant :

l'obtention (S23) d'informations indiquant une activité du travailleur pendant l'opération, les informations indiquant une activité du travailleur étant des informations se rapportant à au moins un paramètre physiologique obtenu au moyen d'au moins un capteur (3, 4) d'activité,
l'estimation de l'émotion (S26) et de la cognition (S27) du travailleur durant l'opération sur la base des informations obtenues indiquant l'activité utilisées comme indicateur primaire, et de premières données d'apprentissage indiquant une relation entre l'activité, et l'émotion du travailleur, et une relation entre l'activité et la cognition du travailleur, dans lequel les premières données d'apprentissage comprennent des données générées sur la base d'informations indiquant l'émotion d'au moins un travailleur, d'informations indiquant la cognition du au moins un travailleur, et d'informations indiquant l'activité du au moins un travailleur, dans lequel lesdites informations indiquant l'émotion se rapportent à au moins un paramètre physiologique obtenu au moyen d'au moins un premier capteur, lesdites informations indiquant la cognition se rapportent à au moins un paramètre indicatif de la cognition et obtenu au moyen d'au moins un deuxième capteur, et lesdites informations indiquant l'activité se rapportent à au moins un paramètre physiologique obtenu au moyen d'au moins un troisième capteur, dans lequel le au moins un capteur (3, 4) d'activité est un capteur qui nécessite une plus petite quantité d'informations, et/ou une moindre charge de traitement, et/ou une moindre résolution temporelle, et/ou est de construction plus simple et/ou moins complexe qu'au moins un du premier capteur et du deuxième capteur ;
l'estimation de la productivité (S28) du travailleur sur la base de l'émotion et de la cognition estimées utilisées chacune comme indicateur secondaire, et de deuxièmes données d'apprentissage indiquant une relation entre la productivité, et l'émotion et la cognition du travailleur ; et
la détermination d'un moment (S29) d'intervention pour le travailleur et d'un détail de l'intervention sur la base de la productivité estimée par la deuxième unité (113) d'estimation et d'une condition prédéterminée pour fournir une intervention.

12. Appareil (1) d'aide à la conduite pour fournir une aide à la conduite, l'appareil comprenant :

une unité (111) d'obtention d'activité configurée pour obtenir des informations indiquant une activité d'un sujet pendant la conduite d'un véhicule, les informations indiquant une activité du sujet étant des informations se rapportant à au moins un paramètre physiologique obtenu au moyen d'au moins un capteur (3, 4) d'activité ;
une première unité d'estimation configurée pour estimer l'émotion et la cognition du sujet pendant la conduite sur la base des informations indiquant l'activité, obtenue par l'unité d'obtention d'activité, utilisées comme indicateur primaire, et de premières données d'apprentissage indiquant une relation entre l'activité et l'émotion du sujet et une relation entre l'activité et la cognition du sujet ;
une deuxième unité (113) d'estimation configurée pour estimer la performance du sujet sur la base de l'émotion et de la cognition estimées utilisées chacune comme indicateur secondaire, et de deuxièmes données d'apprentissage indiquant une relation entre la performance, et l'émotion et la cognition du sujet lors de la conduite, dans lequel les premières données d'apprentissage comprennent des données générées sur la base d'informations indiquant l'émotion d'au moins un sujet, d'informations indiquant la cognition du au moins un sujet, et d'informations indiquant l'activité du au moins un sujet, dans lequel lesdites informations indiquant l'émotion se rapportent à au moins un paramètre physiologique obtenu au moyen d'au moins un premier capteur, lesdites informations indiquant la cognition se rapportent à au moins un paramètre indicatif de la cognition et obtenu au moyen d'au moins un deuxième capteur, et lesdites informations indiquant l'activité se rapportent à au moins un paramètre physiologique obtenu au moyen d'au moins un troisième capteur, dans lequel le au moins un capteur (3, 4) d'activité est un capteur qui nécessite une plus petite quantité d'informations, et/ou une moindre charge de traitement, et/ou une moindre résolution temporelle, et/ou est de construction plus simple et/ou moins complexe qu'au moins un du premier capteur et du deuxième capteur ; et
une unité (114) de détermination d'intervention configurée pour déterminer une intervention à fournir pour le sujet sur la base de la performance estimée par la deuxième unité (113) d'estimation et d'une condition prédéterminée pour fournir une intervention.

13. Procédé d'aide à la conduite pour fournir une aide à la conduite, le procédé comprenant les étapes consistant à :

obtenir des informations indiquant une activité d'un sujet pendant la conduite d'un véhicule, les informations indiquant une activité du sujet étant des informations se rapportant à au moins un paramètre physiologique obtenu au moyen d'au moins un capteur (3, 4) d'activité ;

estimer l'émotion et la cognition du sujet pendant la conduite sur la base des informations obtenues indiquant l'activité utilisées comme indicateur primaire, et de premières données d'apprentissage indiquant une relation entre l'activité et l'émotion du sujet et une relation entre l'activité et la cognition du sujet, dans lequel les premières données d'apprentissage comprennent des données générées sur la base d'informations indiquant l'émotion d'au moins un sujet, d'informations indiquant la cognition du au moins un sujet, et d'informations indiquant l'activité du au moins un sujet, dans lequel lesdites informations indiquant l'émotion se rapportent à au moins un paramètre physiologique obtenu au moyen d'au moins un premier capteur, lesdites informations indiquant la cognition se rapportent à au moins un paramètre indicatif de la cognition et obtenu au moyen d'au moins un deuxième capteur, et lesdites informations indiquant l'activité se rapportent à au moins un paramètre physiologique obtenu au moyen d'au moins un troisième capteur, dans lequel le au moins un capteur (3, 4) d'activité est un capteur qui nécessite une plus petite quantité d'informations, et/ou une moindre charge de traitement, et/ou une moindre résolution temporelle, et/ou est de construction plus simple et/ou moins complexe qu'au moins un du premier capteur et du deuxième capteur ;

estimer la performance du sujet sur la base de l'émotion et de la cognition estimées utilisées chacune comme indicateur secondaire, et de deuxièmes données d'apprentissage indiquant une relation entre la performance, et l'émotion et la cognition du sujet lors de la conduite ; et

déterminer une intervention à fournir pour le sujet sur la base de la performance estimée par la deuxième unité (113) d'estimation et d'une condition prédéterminée pour fournir une intervention.

**14.** Appareil (1) pour soutien sanitaire d'un sujet, l'appareil comprenant :

une unité (111) d'obtention d'activité configurée pour obtenir des informations indiquant une activité d'un sujet pendant la mise en œuvre d'une opération, les informations indiquant une activité du sujet étant des informations se rapportant à au moins un paramètre physiologique obtenu au moyen d'au moins un capteur (3, 4) d'activité ;

une première unité d'estimation configurée pour estimer l'émotion et la cognition du sujet pendant la mise en œuvre de l'opération sur la base des informations indiquant l'activité, obtenue par l'unité d'obtention d'activité, utilisées comme indicateur primaire, et de premières données d'apprentissage indiquant une relation entre l'activité et l'émotion du sujet et une relation entre l'activité et la cognition du sujet ;

une deuxième unité (113) d'estimation configurée pour estimer la performance du sujet sur la base de l'émotion et de la cognition estimées utilisées chacune comme indicateur secondaire, et de deuxièmes données d'apprentissage indiquant une relation entre la performance, et l'émotion et la cognition du sujet lors de la conduite, dans lequel les premières données d'apprentissage comprennent des données générées sur la base d'informations indiquant l'émotion d'au moins un sujet, d'informations indiquant la cognition du au moins un sujet, et d'informations indiquant l'activité du au moins un sujet, dans lequel lesdites informations indiquant l'émotion se rapportent à au moins un paramètre physiologique obtenu au moyen d'au moins un premier capteur, lesdites informations indiquant la cognition se rapportent à au moins un paramètre indicatif de la cognition et obtenu au moyen d'au moins un deuxième capteur, et lesdites informations indiquant l'activité se rapportent à au moins un paramètre physiologique obtenu au moyen d'au moins un troisième capteur, dans lequel le au moins un capteur (3, 4) d'activité est un capteur qui nécessite une plus petite quantité d'informations, et/ou une moindre charge de traitement, et/ou une moindre résolution temporelle, et/ou est de construction plus simple et/ou moins complexe qu'au moins un du premier capteur et du deuxième capteur ; et

une unité (114) de détermination d'intervention configurée pour déterminer une intervention à fournir pour le sujet sur la base de la performance estimée par la deuxième unité (113) d'estimation et d'une condition prédéterminée pour fournir une intervention, dans lequel la mise en œuvre d'une opération inclut préférablement au moins une parmi la mise en œuvre d'une opération d'interaction avec une machine et la mise en œuvre d'un exercice physique.

**15.** Procédé pour soutien sanitaire d'un sujet, le procédé comprenant les étapes consistant à :

l'obtention d'informations indiquant une activité d'un sujet pendant la mise en œuvre d'une opération, les informations indiquant une activité du sujet étant des informations se rapportant à au moins un paramètre physiologique obtenu au moyen d'au moins un capteur (3, 4) d'activité ;

l'estimation de l'émotion et de la cognition du sujet pendant la mise en œuvre de l'opération sur la base des informations obtenues indiquant l'activité utilisées comme indicateur primaire, et de premières données d'apprentissage indiquant une relation entre l'activité et l'émotion du sujet et une relation entre l'activité et la cognition du sujet, dans lequel les premières données d'apprentissage comprennent des données générées sur la base d'informations indiquant l'émotion d'au moins un sujet, d'informations indiquant la cognition du au moins un sujet, et d'informations indiquant l'activité du au moins un sujet, dans lequel lesdites informations indiquant l'émotion se rapportent à au moins un paramètre physiologique obtenu au moyen d'au moins un premier capteur, lesdites informations indiquant la cognition se rapportent à au moins un paramètre indicatif de la cognition et obtenu au moyen d'au moins un deuxième capteur, et lesdites informations indiquant l'activité se rapportent à au moins un paramètre physiologique obtenu au moyen d'au moins un troisième capteur, dans lequel le au moins un capteur (3, 4) d'activité est un capteur qui nécessite une plus petite quantité d'informations, et/ou une moindre charge de traitement, et/ou une moindre résolution temporelle, et/ou est de construction plus simple et/ou moins complexe qu'au moins un du premier capteur et du deuxième capteur ;

l'estimation de la performance du sujet sur la base de l'émotion et de la cognition estimées utilisées chacune comme indicateur secondaire, et de deuxièmes données d'apprentissage indiquant une relation entre la performance, et l'émotion et la cognition du sujet lors de la conduite ; et

la détermination d'une intervention à fournir pour le sujet sur la base de la performance estimée par la deuxième unité (113) d'estimation et d'une condition prédéterminée pour fournir une intervention.

FIG. 1

FIG. 2

FIG. 3

# FIG. 4

Production management apparatus 1

Storage unit 12
- Sensing data storage 121
- Learning data storage 122
- Intervention history storage 123

Control unit 11
- Sensing data obtaining controller 111
- Feature quantity extraction unit 112
- Learning data generation unit 115
- Productivity estimation unit 113
- Intervention controller 114

Interface unit 13

SS1 to SS3
CM
TM
MO1 to MO3
AC1 to AC3

**FIG. 5**

Learning Mode (Emotion)

```
        ┌──────────────┐
        │    Start     │
        └──────────────┘
               │
               ▼
  ┌─────────────────────────────────┐
  │   Obtain and store scale data   │ ～ S11
  └─────────────────────────────────┘
               │
               ▼
  ┌─────────────────────────────────┐
  │ Obtain and store measurement data │ ～ S12
  └─────────────────────────────────┘
               │
               ▼
  ┌─────────────────────────────────┐
  │ Generate and store emotion learning data │ ～ S13
  └─────────────────────────────────┘
               │
               ▼
        ┌──────────────┐
        │     End      │
        └──────────────┘
```

**FIG. 6**

Learning Mode (Cognition)

```
        ┌──────────────┐
        │    Start     │
        └──────────────┘
               │
               ▼
  ┌─────────────────────────────────┐
  │ Obtain and store measurement data │ ～ S14
  └─────────────────────────────────┘
               │
               ▼
  ┌─────────────────────────────────┐
  │ Obtain and store cognition data │ ～ S15
  └─────────────────────────────────┘
               │
               ▼
  ┌─────────────────────────────────┐
  │ Generate and store cognition learning data │ ～ S16
  └─────────────────────────────────┘
               │
               ▼
        ┌──────────────┐
        │     End      │
        └──────────────┘
```

## FIG. 7

Generating and Storing Emotion Learning Data

```
                    ┌─────────────┐
                    │    Start    │
                    └──────┬──────┘
                           │
    ┌────────────────────────────────────────────────┐
    │      Set window unit duration at initial value  │──S131
    └────────────────────────────────────────────────┘
(C2)──────────────────────►│
    ┌────────────────────────────────────────────────┐
    │               Select window Wi                  │──S132
    └────────────────────────────────────────────────┘
                           │
    ┌────────────────────────────────────────────────┐
    │        Read scale data within window Wi         │──S133
    └────────────────────────────────────────────────┘
                           │
    ┌────────────────────────────────────────────────┐
    │   Calculate variations among feature quantities │──S134
    │   for arousal and for valence                   │
    └────────────────────────────────────────────────┘
                           │
    ┌────────────────────────────────────────────────┐
    │  Read measurement data obtained within window   │──S135
    │  Wi                                             │
    └────────────────────────────────────────────────┘
                           │
    ┌────────────────────────────────────────────────┐
    │  Calculate variations among feature quantities  │──S136
    │  of measurement data                            │
    └────────────────────────────────────────────────┘
                           │
    ┌────────────────────────────────────────────────┐
    │  Generate regression equations for arousal and  │──S137
    │  for valence                                    │
    └────────────────────────────────────────────────┘
                           │
    ┌────────────────────────────────────────────────┐
    │    Store the generated regression equations     │──S138
    └────────────────────────────────────────────────┘
                           │
                                            S139
           No      ╱◇───────────────────◇╲
    ┌─────────────◇   All windows Wi selected?  ◇
    │              ╲◇───────────────────◇╱
    │                        │ Yes
    │                     ┌─────┐
    │                     │ C1  │
    │                     └─────┘
```

FIG. 8

C1

Calculate emotion estimates $\hat{A}i$ and $\hat{V}i$ using regression equations ~S141

Calculate $\Sigma$ (A-Â) and store the results ~S142

S143

No ← Changing window unit duration and shift complete? →

Yes

S144

Change unit duration and shift of window Wi

C2

Select combination of window unit duration and shift that meets $\Sigma$ (A-Â) = min ~S145

Set selected combination of window unit duration and shift ~S146

Store regression equations corresponding to selected combination into learning data storage ~S147

Return

# FIG. 9

Generating and Storing Cognition Learning Data

```
              ┌──────────────┐
              │    Start     │
              └──────────────┘
                     │
     ┌───────────────┤
     │       ┌────────────────────────────────────┐
     │       │   Select operation time period     │──~S161
     │       └────────────────────────────────────┘
     │                      │
     │       ┌────────────────────────────────────┐
     │       │ Read image data indicating operation results │──~S162
     │       └────────────────────────────────────┘
     │                      │
     │       ┌────────────────────────────────────┐
     │       │ Extract feature quantities indicating │──~S163
     │       │   success or failure in operation   │
     │       └────────────────────────────────────┘
     │                      │
     │       ┌────────────────────────────────────┐
     │       │ Read measurement data indicating eye and │──~S164
     │       │         hand movements             │
     │       └────────────────────────────────────┘
     │                      │
     │       ┌────────────────────────────────────┐
     │       │ Calculate change in feature quantity │──~S165
     │       │   from each set of measurement data │
     │       └────────────────────────────────────┘
     │                      │
     │       ┌────────────────────────────────────┐
     │       │ Generate regression equation for estimating │──~S166
     │       │            cognition               │
     │       └────────────────────────────────────┘
     │                      │
     │                                      S167
     │  No    ◇────────────────────────────────◇
     └────────  All operation time periods selected?  ◇
             ◇────────────────────────────────◇
                          │ Yes
              ┌────────────────────────────────────┐
              │    Store the regression equations   │──~S168
              └────────────────────────────────────┘
                          │
              ┌──────────────┐
              │     End      │
              └──────────────┘
```

FIG. 10

FIG. 11

FIG. 12

Emotion Estimation

Start

Receive feature quantities ~S261

Read regression equations ~S262

Calculate emotion estimates $\hat{A}i$ and $\hat{V}i$
for arousal and for valence ~S263

Return

FIG. 13

Cognition Estimation

Start

Receive feature quantities ~S271

Read regression equation ~S272

Calculate cognition estimate using
eye movement and hand movement ~S273

Return

# FIG. 14

Productivity Estimation and Intervention Control

```
                    ( Start )
                        │
                        │         S281
                        ▼
           ┌──────────────────────────┐
           │ Calculate difference between │
           │ operation times          │
           └──────────────────────────┘
                        │         S282
                        ▼
           ┌──────────────────────────┐
           │ Calculate time deviations │
           └──────────────────────────┘
                        │
                        │              S291
                        ▼
                  ◇ ΔPi > thi? ◇ ─────No──────────────────────┐
                        │                                       │
                       Yes                                      │
                        │              S292                     │
                        ▼                                       │
              ◇ First intervention ◇ ──────Yes────┐            │
              ◇    provided?       ◇               │            │
                        │                          │            │
                       No      S293                │            │
                        ▼                          │            │
           ┌──────────────────┐                   │            │
           │ Provide first    │                   ▼   S294     │
           │ intervention     │         ◇ Second intervention ◇ ──Yes──┐
           │ (Display message)│         ◇     provided?       ◇        │
           └──────────────────┘                   │                     │
                        │                         No    S295            │
                        │                          ▼                     │
                        │              ┌────────────────────┐           │
                        │              │ Provide second     │           │
                        │              │ intervention       │     S296  │
                        │              │ (Display message   │    ▼      │
                        │              │ and drive vibrator)│  ┌──────────────┐
                        │              └────────────────────┘  │ Instruct to stop │
                        │                          │           │ operation (rest) │
                        │                          │           └──────────────┘
                        │                          │                     │
                        ◄──────────────────────────┴─────────────────────┘
                        │
                        ▼
                   ( Return )
```

FIG. 15

First intervention
(Display message)

Second intervention
(Provide vibration
stimulus)

Third intervention
(Instruct to stop
operation)

FIG. 16

First intervention
(Display message)

FIG. 17

High arousal (exciting, agitating)

"slaughter"

"miracle"

Low valence
(negative)  "death"  "shadow"  "comfort"  High valence
(positive)

"fatigued"

"relaxed"

Low arousal (soothing, calming)

FIG. 18

**FIG. 19**

Classification (grouping, stratification)

Identifying the emotional quadrant

High arousal

1                    2

Low valence ←————————————————→ High valence

3                    4

Low arousal

**FIG. 20**

Variations (evaluated in scales)

Calculating emotional changes for arousal and for valence

High arousal

100

-100                          0              100
Low valence ←——————————————————————→ High valence

K2

K1 ●

+20

+50

-100

Low arousal

# FIG. 21

FIG. 22

| | | Visual system | | Autonomic nervous system | | | Musculoskeletal system | | |
|---|---|---|---|---|---|---|---|---|---|
| | | LoS | Ocular potential | EEG Single channel | ECG, pulse wave | Blood pressure | Skin potential | Movement and motion | Muscle potential |
| Cognition | Evaluation | O | ▲ | O | X | X | X | O | ▲ |
| | Signal | ▸ Position of LoS<br>▸ Track of LoS<br>▸ LoS speed<br>▸ Speed of following objects<br>▸ Congestion angle<br>▸ Angle of field of vision | ▸ Difference in muscular potential between eyes and forehead<br><br>Problem: Noise | ▸ Increase and decrease in wavelength $\alpha \cdot \beta$<br>▸ Wavelength ratio $\alpha/\beta$ | ▸ RRI<br>▸ LF/HF<br>▸ No. of heart beats | No suitable signals available | ▸ GSR<br>▸ SSR<br>▸ EDR | ▸ Actions<br>▸ Track of actions<br>▸ Action speed<br>▸ Action patterns | Relative changes in active muscular potential against sensory stimulations |
| Emotion | Evaluation | O | ▲ | O | O | O | ▲ | X | ▲ |
| | Signal | ▸ Size of pupils<br>▸ No. of blinks | ▸ No. of blinks<br><br>Problem: Facial mimetic muscle noise | ▸ Increase and decrease in wavelength $\alpha \cdot \beta \cdot \Theta$ | ▸ CVRR<br>▸ RRI<br>▸ LF/HF | Blood pressure fluctuations | ▸ GSR/SC | No suitable signals available | Relative changes in active muscular potential against sensory stimulations |

# FIG. 23

Measurements related to Cognitive and
Emotional systems

AUTONOMIC NERVOUS
SYSTEM

VISUAL SYSTEM
Ocular
movement: Line
of Sight (LoS),
ocular potential

EEG (a single-channel
measurement device)

ECG, pulse wave

Blood pressure

Records of emotions by
measuring physiological
parameters

MUSCULO-SKELETAL
SYSTEM

Muscle potential

Skin potential (resistence)

Exercise functions:
amount of activity, number
of steps, motions

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 5530019 B
- WO 2009052633 A1, MOTT CHRISTOPHER [CA]; MOLLICONE DANIEL [US]; VAN DONGEN HANS
- WO 2006000166 A1
- US 2014240132 A1, BYCHKOV DAVID
- US 2014336473 A1, GRECO DEVON
- JP 2011019921 A
- US 2009066521 A1, ATLAS DAN
- JP 2016252368 A
- WO 2017055272 A
- WO 2017058414 A

### Non-patent literature cited in the description

- **STEVEN J. LUCK**. An Introduction to the Event-Related Potential Technique
- **J. POSNER et al.** The Neurophysiological Bases of Emotion: An fMRI Study of the Affective Circumplex Using Emotion-Denoting Words. *Hum Brain Mapp.*, March 2009, vol. 30 (3), 883-895